# EUROPEAN PATENT APPLICATION

(11) **EP 4 851 189 A1**
(43) Date of publication of application: **09.12.2026**
(21) Application number: 25747826.3
(22) Date of filing: 24.01.2025
(51) Int. Cl.: C07K 16/18, C07K 16/28, C12N 15/13, C12N 15/85, A61K 47/68, A61K 39/395, A61P 35/00

(54) **ANTI-DLL3 ANTIBODY AND USE**

(30) Priority: 30.01.2024 CN 202410125989
(71) Applicant: CSPC Megalith Biopharmaceutical Co., Ltd., Shijiazhuang, Hebei 050035 (CN)
(72) Inventor: HUI, Xiwu, Shijiazhuang, Hebei 050035 (CN); DING, Huandi, Shijiazhuang, Hebei 050035 (CN); FENG, Jing, Shijiazhuang, Hebei 050035 (CN); WANG, Ruihuan, Shijiazhuang, Hebei 050035 (CN); ZHANG, Yaoqing, Shijiazhuang, Hebei 050035 (CN); WANG, Yancui, Shijiazhuang, Hebei 050035 (CN); DAN, Mo, Shijiazhuang, Hebei 050035 (CN); YAO, Bing, Shijiazhuang, Hebei 050035 (CN); WANG, Mingxiao, Shijiazhuang, Hebei 050035 (CN)
(74) Representative: Gille Hrabal Partnerschaftsgesellschaft mbB Patentanwälte
(86) International application number: PCT/CN2025/074698
(87) International publication number: WO 2025/162258

(57) **Abstract**

Provided are an anti-DLL3 antibody, an antibody-drug conjugate comprising the antibody, and a diagnosis and treatment method for a disease related to expression of DLL3, such as cancer.

## Description

The present disclosure claims priority to Patent Application No. 202410125989.2, filed with the China National Intellectual Property Administration on January 30, 2024 and entitled "ANTI-DLL3 ANTIBODY AND USE", which is incorporated herein by reference in its entirety.

### TECHNICAL FIELD

The present disclosure pertains to the field of biopharmaceuticals and particularly relates to an anti-DLL3 antibody and use thereof.

### BACKGROUND

Small cell lung cancer (SCLC) is a highly aggressive neuroendocrine malignant tumor, accounting for about 15% of all lung cancer cases. Many patients are already in the extensive stage at diagnosis. Although small cell lung cancer is sensitive to radiotherapy and chemotherapy, it is highly prone to relapse. Etoposide has been used in combination with carboplatin or cisplatin as the standard first-line treatment regimen for patients with extensive-stage SCLC for a long time. The response rate of the first-line treatment is 70%-90% in limited-stage SCLC, and the response rate is 50%-60% in extensive-stage SCLC. Within one year of receiving the first-line treatment for SCLC, about 80% of limited-stage patients and nearly all extensive-stage patients experience relapse or progression; there is a significant unmet clinical need. Studies have shown that DLL3 is highly expressed in more than 80% of patients with small cell lung cancer (SCLC), and the high expression of DLL3 in SCLC is negatively correlated with the patient's survival-the higher the DLL3 expression, the shorter the patient's survival.

DLL3 (delta-like ligand 3) is a single-pass transmembrane protein consisting of 619 amino acids. Its complete structure contains one DSL domain, one intracellular domain, and six epidermal growth factor-like domains. This target is hardly expressed in normal tissues but is specifically and highly expressed in neuroendocrine tumors. Therefore, DLL3 is promising as a potential target for lung cancer treatment. Several DLL3-targeting drugs are currently under development, primarily including bispecific antibodies, cell therapies, and ADC drugs. For example, Amgen's bispecific antibody AMG 757 (Patent No. US2017037130A1) is now in Phase 3 clinical trials and has shown some advantages in improving median DOR, median OS, and safety. Rovalpituzumab tesirine (Rova-T, Patent No. US9770518B1) is the first DLL3-targeting ADC drug developed by AbbVie, and it is also the first targeted therapy drug to enter clinical studies for SCLC. However, due to the unacceptable toxic side effects of the PBD cytotoxic drug, the drug could not be tolerated by patients in the Phase 3 clinical study, leading to insufficient effectiveness; the study has been terminated.

In order to offer patients a wider range of treatment regimens and meet their more diverse therapeutic needs, the present disclosure provides DLL3 antibodies with higher affinities. Moreover, the antibody-drug conjugates of the present disclosure have the ability to kill tumor cells *in vivo* and *in vitro* to a greater extent than Rova-T.

### SUMMARY

In a first aspect, the present disclosure provides an anti-DLL3 antibody or an antigen-binding fragment thereof, comprising a heavy chain variable region, wherein the heavy chain variable region comprises an HCDR1, an HCDR2, and an HCDR3, wherein:
(1) the sequence of the HCDR1 is the sequence set forth in SEQ ID NO.25 or a sequence having at least 80%, 85%, 90%, 95%, 98%, or 99% identity to the sequence set forth in SEQ ID NO.25, the sequence of the HCDR2 is the sequence set forth in SEQ ID NO.26 or a sequence having at least 80%, 85%, 90%, 95%, 98%, or 99% identity to the sequence set forth in SEQ ID NO.26, and the sequence of the HCDR3 is the sequence set forth in SEQ ID NO.27 or a sequence having at least 80%, 85%, 90%, 95%, 98%, or 99% identity to the sequence set forth in SEQ ID NO.27;
(2) the sequence of the HCDR1 is the sequence set forth in SEQ ID NO.1 or a sequence having at least 80%, 85%, 90%, 95%, 98%, or 99% identity to the sequence set forth in SEQ ID NO.1, the sequence of the HCDR2 is the sequence set forth in SEQ ID NO.2 or a sequence having at least 80%, 85%, 90%, 95%, 98%, or 99% identity to the sequence set forth in SEQ ID NO.2, and the sequence of the HCDR3 is the sequence set forth in SEQ ID NO.3 or a sequence having at least 80%, 85%, 90%, 95%, 98%, or 99% identity to the sequence set forth in SEQ ID NO.3;
(3) the sequence of the HCDR1 is the sequence set forth in SEQ ID NO.9 or a sequence having at least 80%, 85%, 90%, 95%, 98%, or 99% identity to the sequence set forth in SEQ ID NO.9, the sequence of the HCDR2 is the sequence set forth in SEQ ID NO.10 or a sequence having at least 80%, 85%, 90%, 95%, 98%, or 99% identity to the sequence set forth in SEQ ID NO.10, and the sequence of the HCDR3 is the sequence set forth in SEQ ID NO.11 or a sequence having at least 80%, 85%, 90%, 95%, 98%, or 99% identity to the sequence set forth in SEQ ID NO.11;
(4) the sequence of the HCDR1 is the sequence set forth in SEQ ID NO.17 or a sequence having at least 80%, 85%, 90%, 95%, 98%, or 99% identity to the sequence set forth in SEQ ID NO.17, the sequence of the HCDR2 is the sequence set forth in SEQ ID NO.18 or a sequence having at least 80%, 85%, 90%, 95%, 98%, or 99% identity to the sequence set forth in SEQ ID NO.18, and the sequence of the HCDR3 is the sequence set forth in SEQ ID NO.19 or a sequence having at least 80%, 85%, 90%, 95%, 98%, or 99% identity to the sequence set forth in SEQ ID NO.19; or
(5) the sequence of the HCDR1 is the sequence set forth in SEQ ID NO.33 or a sequence having at least 80%, 85%, 90%, 95%, 98%, or 99% identity to the sequence set forth in SEQ ID NO.33, the sequence of the HCDR2 is the sequence set forth in SEQ ID NO.34 or a sequence having at least 80%, 85%, 90%, 95%, 98%, or 99% identity to the sequence set forth in SEQ ID NO.34, and the sequence of the HCDR3 is the sequence set forth in SEQ ID NO.35 or a sequence having at least 80%, 85%, 90%, 95%, 98%, or 99% identity to the sequence set forth in SEQ ID NO.35.

In a second aspect, the present disclosure provides an anti-DLL3 antibody or an antigen-binding fragment thereof, comprising a light chain variable region, wherein the light chain variable region comprises an LCDR1, an LCDR2, and an LCDR3, wherein:
(1) the sequence of the LCDR1 is the sequence set forth in SEQ ID NO.28 or a sequence having at least 80%, 85%, 90%, 95%, 98%, or 99% identity to the sequence set forth in SEQ ID NO.28, the sequence of the LCDR2 is the sequence set forth in SEQ ID NO.29 or a sequence having at least 80%, 85%, 90%, 95%, 98%, or 99% identity to the sequence set forth in SEQ ID NO.29, and the sequence of the LCDR3 is the sequence set forth in SEQ ID NO.30 or a sequence having at least 80%, 85%, 90%, 95%, 98%, or 99% identity to the sequence set forth in SEQ ID NO.30;
(2) the sequence of the LCDR1 is the sequence set forth in SEQ ID NO.4 or a sequence having at least 80%, 85%, 90%, 95%, 98%, or 99% identity to the sequence set forth in SEQ ID NO.4, the sequence of the LCDR2 is the sequence set forth in SEQ ID NO.5 or a sequence having at least 80%, 85%, 90%, 95%, 98%, or 99% identity to the sequence set forth in SEQ ID NO.5, and the sequence of the LCDR3 is the sequence set forth in SEQ ID NO.6 or a sequence having at least 80%, 85%, 90%, 95%, 98%, or 99% identity to the sequence set forth in SEQ ID NO.6;
(3) the sequence of the LCDR1 is the sequence set forth in SEQ ID NO.12 or a sequence having at least 80%, 85%, 90%, 95%, 98%, or 99% identity to the sequence set forth in SEQ ID NO.12, the sequence of the LCDR2 is the sequence set forth in SEQ ID NO.13 or a sequence having at least 80%, 85%, 90%, 95%, 98%, or 99% identity to the sequence set forth in SEQ ID NO.13, and the sequence of the LCDR3 is the sequence set forth in SEQ ID NO.14 or a sequence having at least 80%, 85%, 90%, 95%, 98%, or 99% identity to the sequence set forth in SEQ ID NO.14;
(4) the sequence of the LCDR1 is the sequence set forth in SEQ ID NO.20 or a sequence having at least 80%, 85%, 90%, 95%, 98%, or 99% identity to the sequence set forth in SEQ ID NO.20, the sequence of the LCDR2 is the sequence set forth in SEQ ID NO.21 or a sequence having at least 80%, 85%, 90%, 95%, 98%, or 99% identity to the sequence set forth in SEQ ID NO.21, and the sequence of the LCDR3 is the sequence set forth in SEQ ID NO.22 or a sequence having at least 80%, 85%, 90%, 95%, 98%, or 99% identity to the sequence set forth in SEQ ID NO.22; or
(5) the sequence of the LCDR1 is the sequence set forth in SEQ ID NO.36 or a sequence having at least 80%, 85%, 90%, 95%, 98%, or 99% identity to the sequence set forth in SEQ ID NO.36, the sequence of the LCDR2 is the sequence set forth in SEQ ID NO.37 or a sequence having at least 80%, 85%, 90%, 95%, 98%, or 99% identity to the sequence set forth in SEQ ID NO.37, and the sequence of the LCDR3 is the sequence set forth in SEQ ID NO.38 or a sequence having at least 80%, 85%, 90%, 95%, 98%, or 99% identity to the sequence set forth in SEQ ID NO.38.

In a third aspect, the present disclosure provides an anti-DLL3 antibody or an antigen-binding fragment thereof, comprising a heavy chain variable region and a light chain variable region, wherein the heavy chain variable region comprises an HCDR1, an HCDR2, and an HCDR3, and the light chain variable region comprises an LCDR1, an LCDR2, and an LCDR3, wherein:
(1) the sequence of the HCDR1 is the sequence set forth in SEQ ID NO.25 or a sequence having at least 80%, 85%, 90%, 95%, 98%, or 99% identity to the sequence set forth in SEQ ID NO.25, the sequence of the HCDR2 is the sequence set forth in SEQ ID NO.26 or a sequence having at least 80%, 85%, 90%, 95%, 98%, or 99% identity to the sequence set forth in SEQ ID NO.26, the sequence of the HCDR3 is the sequence set forth in SEQ ID NO.27 or a sequence having at least 80%, 85%, 90%, 95%, 98%, or 99% identity to the sequence set forth in SEQ ID NO.27, the sequence of the LCDR1 is the sequence set forth in SEQ ID NO.28 or a sequence having at least 80%, 85%, 90%, 95%, 98%, or 99% identity to the sequence set forth in SEQ ID NO.28, the sequence of the LCDR2 is the sequence set forth in SEQ ID NO.29 or a sequence having at least 80%, 85%, 90%, 95%, 98%, or 99% identity to the sequence set forth in SEQ ID NO.29, and the sequence of the LCDR3 is the sequence set forth in SEQ ID NO.30 or a sequence having at least 80%, 85%, 90%, 95%, 98%, or 99% identity to the sequence set forth in SEQ ID NO.30;
(2) the sequence of the HCDR1 is the sequence set forth in SEQ ID NO.1 or a sequence having at least 80%, 85%, 90%, 95%, 98%, or 99% identity to the sequence set forth in SEQ ID NO.1, the sequence of the HCDR2 is the sequence set forth in SEQ ID NO.2 or a sequence having at least 80%, 85%, 90%, 95%, 98%, or 99% identity to the sequence set forth in SEQ ID NO.2, the sequence of the HCDR3 is the sequence set forth in SEQ ID NO.3 or a sequence having at least 80%, 85%, 90%, 95%, 98%, or 99% identity to the sequence set forth in SEQ ID NO.3, the sequence of the LCDR1 is the sequence set forth in SEQ ID NO.4 or a sequence having at least 80%, 85%, 90%, 95%, 98%, or 99% identity to the sequence set forth in SEQ ID NO.4, the sequence of the LCDR2 is the sequence set forth in SEQ ID NO.5 or a sequence having at least 80%, 85%, 90%, 95%, 98%, or 99% identity to the sequence set forth in SEQ ID NO.5, and the sequence of the LCDR3 is the sequence set forth in SEQ ID NO.6 or a sequence having at least 80%, 85%, 90%, 95%, 98%, or 99% identity to the sequence set forth in SEQ ID NO.6;
(3) the sequence of the HCDR1 is the sequence set forth in SEQ ID NO.9 or a sequence having at least 80%, 85%, 90%, 95%, 98%, or 99% identity to the sequence set forth in SEQ ID NO.9, the sequence of the HCDR2 is the sequence set forth in SEQ ID NO.10 or a sequence having at least 80%, 85%, 90%, 95%, 98%, or 99% identity to the sequence set forth in SEQ ID NO.10, the sequence of the HCDR3 is the sequence set forth in SEQ ID NO.11 or a sequence having at least 80%, 85%, 90%, 95%, 98%, or 99% identity to the sequence set forth in SEQ ID NO.11, the sequence of the LCDR1 is the sequence set forth in SEQ ID NO.12 or a sequence having at least 80%, 85%, 90%, 95%, 98%, or 99% identity to the sequence set forth in SEQ ID NO.12, the sequence of the LCDR2 is the sequence set forth in SEQ ID NO.13 or a sequence having at least 80%, 85%, 90%, 95%, 98%, or 99% identity to the sequence set forth in SEQ ID NO.13, and the sequence of the LCDR3 is the sequence set forth in SEQ ID NO.14 or a sequence having at least 80%, 85%, 90%, 95%, 98%, or 99% identity to the sequence set forth in SEQ ID NO.14;
(4) the sequence of the HCDR1 is the sequence set forth in SEQ ID NO.17 or a sequence having at least 80%, 85%, 90%, 95%, 98%, or 99% identity to the sequence set forth in SEQ ID NO.17, the sequence of the HCDR2 is the sequence set forth in SEQ ID NO.18 or a sequence having at least 80%, 85%, 90%, 95%, 98%, or 99% identity to the sequence set forth in SEQ ID NO.18, the sequence of the HCDR3 is the sequence set forth in SEQ ID NO.19 or a sequence having at least 80%, 85%, 90%, 95%, 98%, or 99% identity to the sequence set forth in SEQ ID NO.19, the sequence of the LCDR1 is the sequence set forth in SEQ ID NO.20 or a sequence having at least 80%, 85%, 90%, 95%, 98%, or 99% identity to the sequence set forth in SEQ ID NO.20, the sequence of the LCDR2 is the sequence set forth in SEQ ID NO.21 or a sequence having at least 80%, 85%, 90%, 95%, 98%, or 99% identity to the sequence set forth in SEQ ID NO.21, and the sequence of the LCDR3 is the sequence set forth in SEQ ID NO.22 or a sequence having at least 80%, 85%, 90%, 95%, 98%, or 99% identity to the sequence set forth in SEQ ID NO.22; or
(5) the sequence of the HCDR1 is the sequence set forth in SEQ ID NO.33 or a sequence having at least 80%, 85%, 90%, 95%, 98%, or 99% identity to the sequence set forth in SEQ ID NO.33, the sequence of the HCDR2 is the sequence set forth in SEQ ID NO.34 or a sequence having at least 80%, 85%, 90%, 95%, 98%, or 99% identity to the sequence set forth in SEQ ID NO.34, the sequence of the HCDR3 is the sequence set forth in SEQ ID NO.35 or a sequence having at least 80%, 85%, 90%, 95%, 98%, or 99% identity to the sequence set forth in SEQ ID NO.35, the sequence of the LCDR1 is the sequence set forth in SEQ ID NO.36 or a sequence having at least 80%, 85%, 90%, 95%, 98%, or 99% identity to the sequence set forth in SEQ ID NO.36, the sequence of the LCDR2 is the sequence set forth in SEQ ID NO.37 or a sequence having at least 80%, 85%, 90%, 95%, 98%, or 99% identity to the sequence set forth in SEQ ID NO.37, and the sequence of the LCDR3 is the sequence set forth in SEQ ID NO.38 or a sequence having at least 80%, 85%, 90%, 95%, 98%, or 99% identity to the sequence set forth in SEQ ID NO.38.

In the present disclosure, the CDR sequences are defined according to Kabat.

The anti-DLL3 antibody or the antigen-binding fragment thereof described in the present disclosure is a murine, chimeric, humanized, or fully human antibody or antigen-binding fragment; preferably, the anti-DLL3 antibody or the antigen-binding fragment thereof described in the present disclosure is a humanized antibody or antigen-binding fragment.

In a fourth aspect, the present disclosure provides an anti-DLL3 antibody or an antigen-binding fragment thereof, wherein the anti-DLL3 antibody or the antigen-binding fragment thereof comprises a heavy chain variable region, wherein:
(1) the sequence of the heavy chain variable region is the sequence set forth in SEQ ID NO.59 or a sequence having at least 80%, 85%, 90%, 95%, 98%, or 99% identity to the sequence set forth in SEQ ID NO.59;
(2) the sequence of the heavy chain variable region is the sequence set forth in SEQ ID NO.7 or a sequence having at least 80%, 85%, 90%, 95%, 98%, or 99% identity to the sequence set forth in SEQ ID NO.7;
(3) the sequence of the heavy chain variable region is the sequence set forth in SEQ ID NO.15 or a sequence having at least 80%, 85%, 90%, 95%, 98%, or 99% identity to the sequence set forth in SEQ ID NO.15;
(4) the sequence of the heavy chain variable region is the sequence set forth in SEQ ID NO.23 or a sequence having at least 80%, 85%, 90%, 95%, 98%, or 99% identity to the sequence set forth in SEQ ID NO.23;
(5) the sequence of the heavy chain variable region is the sequence set forth in SEQ ID NO.31 or a sequence having at least 80%, 85%, 90%, 95%, 98%, or 99% identity to the sequence set forth in SEQ ID NO.31;
(6) the sequence of the heavy chain variable region is the sequence set forth in SEQ ID NO.39 or a sequence having at least 80%, 85%, 90%, 95%, 98%, or 99% identity to the sequence set forth in SEQ ID NO.39;
(7) the sequence of the heavy chain variable region is the sequence set forth in SEQ ID NO.54 or a sequence having at least 80%, 85%, 90%, 95%, 98%, or 99% identity to the sequence set forth in SEQ ID NO.54; or
(8) the sequence of the heavy chain variable region is the sequence set forth in SEQ ID NO.56 or a sequence having at least 80%, 85%, 90%, 95%, 98%, or 99% identity to the sequence set forth in SEQ ID NO.56.

In a fifth aspect, the present disclosure provides an anti-DLL3 antibody or an antigen-binding fragment thereof, wherein the anti-DLL3 antibody or the antigen-binding fragment thereof comprises a light chain variable region, wherein:
(1) the sequence of the light chain variable region is the sequence set forth in SEQ ID NO.59 or a sequence having at least 80%, 85%, 90%, 95%, 98%, or 99% identity to the sequence set forth in SEQ ID NO.59;
(2) the sequence of the light chain variable region is the sequence set forth in SEQ ID NO.8 or a sequence having at least 80%, 85%, 90%, 95%, 98%, or 99% identity to the sequence set forth in SEQ ID NO.8;
(3) the sequence of the light chain variable region is the sequence set forth in SEQ ID NO.16 or a sequence having at least 80%, 85%, 90%, 95%, 98%, or 99% identity to the sequence set forth in SEQ ID NO.16;
(4) the sequence of the light chain variable region is the sequence set forth in SEQ ID NO.24 or a sequence having at least 80%, 85%, 90%, 95%, 98%, or 99% identity to the sequence set forth in SEQ ID NO.24;
(5) the sequence of the light chain variable region is the sequence set forth in SEQ ID NO.32 or a sequence having at least 80%, 85%, 90%, 95%, 98%, or 99% identity to the sequence set forth in SEQ ID NO.32;
(6) the sequence of the light chain variable region is the sequence set forth in SEQ ID NO.40 or a sequence having at least 80%, 85%, 90%, 95%, 98%, or 99% identity to the sequence set forth in SEQ ID NO.40;
(7) the sequence of the light chain variable region is the sequence set forth in SEQ ID NO.55 or a sequence having at least 80%, 85%, 90%, 95%, 98%, or 99% identity to the sequence set forth in SEQ ID NO.55; or
(8) the sequence of the light chain variable region is the sequence set forth in SEQ ID NO.57 or a sequence having at least 80%, 85%, 90%, 95%, 98%, or 99% identity to the sequence set forth in SEQ ID NO.57.

In a sixth aspect, the present disclosure provides an anti-DLL3 antibody or an antigen-binding fragment thereof, wherein the anti-DLL3 antibody or the antigen-binding fragment thereof comprises a heavy chain variable region and a light chain variable region, wherein:
(1) the sequence of the heavy chain variable region is the sequence set forth in SEQ ID NO.58 or a sequence having at least 80%, 85%, 90%, 95%, 98%, or 99% identity to the sequence set forth in SEQ ID NO.58, and the sequence of the light chain variable region is the sequence set forth in SEQ ID NO.59 or a sequence having at least 80%, 85%, 90%, 95%, 98%, or 99% identity to the sequence set forth in SEQ ID NO.59;
(2) the sequence of the heavy chain variable region is the sequence set forth in SEQ ID NO.7 or a sequence having at least 80%, 85%, 90%, 95%, 98%, or 99% identity to the sequence set forth in SEQ ID NO.7, and the sequence of the light chain variable region is the sequence set forth in SEQ ID NO.8 or a sequence having at least 80%, 85%, 90%, 95%, 98%, or 99% identity to the sequence set forth in SEQ ID NO.8;
(3) the sequence of the heavy chain variable region is the sequence set forth in SEQ ID NO.15 or a sequence having at least 80%, 85%, 90%, 95%, 98%, or 99% identity to the sequence set forth in SEQ ID NO.15, and the sequence of the light chain variable region is the sequence set forth in SEQ ID NO.16 or a sequence having at least 80%, 85%, 90%, 95%, 98%, or 99% identity to the sequence set forth in SEQ ID NO.16;
(4) the sequence of the heavy chain variable region is the sequence set forth in SEQ ID NO.23 or a sequence having at least 80%, 85%, 90%, 95%, 98%, or 99% identity to the sequence set forth in SEQ ID NO.23, and the sequence of the light chain variable region is the sequence set forth in SEQ ID NO.24 or a sequence having at least 80%, 85%, 90%, 95%, 98%, or 99% identity to the sequence set forth in SEQ ID NO.24;
(5) the sequence of the heavy chain variable region is the sequence set forth in SEQ ID NO.31 or a sequence having at least 80%, 85%, 90%, 95%, 98%, or 99% identity to the sequence set forth in SEQ ID NO.31, and the sequence of the light chain variable region is the sequence set forth in SEQ ID NO.32 or a sequence having at least 80%, 85%, 90%, 95%, 98%, or 99% identity to the sequence set forth in SEQ ID NO.32;
(6) the sequence of the heavy chain variable region is the sequence set forth in SEQ ID NO.39 or a sequence having at least 80%, 85%, 90%, 95%, 98%, or 99% identity to the sequence set forth in SEQ ID NO.39, and the sequence of the light chain variable region is the sequence set forth in SEQ ID NO.40 or a sequence having at least 80%, 85%, 90%, 95%, 98%, or 99% identity to the sequence set forth in SEQ ID NO.40;
(7) the sequence of the heavy chain variable region is the sequence set forth in SEQ ID NO.54 or a sequence having at least 80%, 85%, 90%, 95%, 98%, or 99% identity to the sequence set forth in SEQ ID NO.54, and the sequence of the light chain variable region is the sequence set forth in SEQ ID NO.55 or a sequence having at least 80%, 85%, 90%, 95%, 98%, or 99% identity to the sequence set forth in SEQ ID NO.55; or
(8) the sequence of the heavy chain variable region is the sequence set forth in SEQ ID NO.56 or a sequence having at least 80%, 85%, 90%, 95%, 98%, or 99% identity to the sequence set forth in SEQ ID NO.56, and the sequence of the light chain variable region is the sequence set forth in SEQ ID NO.57 or a sequence having at least 80%, 85%, 90%, 95%, 98%, or 99% identity to the sequence set forth in SEQ ID NO.57.

In some embodiments of the first to sixth aspects, the anti-DLL3 antibody or the antigen-binding fragment thereof specifically binds to human DLL3. In some specific embodiments, the anti-DLL3 antibody or the antigen-binding fragment thereof binds to a DLL3 protein epitope that is conserved between DLL3 proteins from different species (e.g., between human DLL3 and cynomolgus monkey DLL3).

In the present disclosure, the antigen-binding fragment is an Fv, VHH, scFv, Fab, Fab', or (Fab')₂.

In some embodiments of the first to sixth aspects, the anti-DLL3 antibody or the antigen-binding fragment thereof has ADCC activity.

In some embodiments of the first to sixth aspects, the anti-DLL3 antibody or the antigen-binding fragment thereof has CDC activity.

In some embodiments of the first to sixth aspects, the anti-DLL3 antibody or the antigen-binding fragment thereof has ADCP activity.

In some embodiments of the first to sixth aspects, the anti-DLL3 antibody is a full-length antibody.

In some embodiments of the first to sixth aspects, the anti-DLL3 antibody is a monoclonal antibody.

In some embodiments of the first to sixth aspects, the anti-DLL3 antibody is an IgM, IgD, IgG, IgA or IgE type antibody. In some specific embodiments, the anti-DLL3 antibody is an IgG antibody. In some more specific embodiments, the anti-DLL3 antibody is an IgG1 antibody.

In some embodiments of the first to sixth aspects, the anti-DLL3 antibody is of the IgG1, IgG2, or IgG4 isotype.

In some embodiments of the first to sixth aspects, the anti-DLL3 antibody comprises a light chain constant region of the κ subtype or the λ subtype.

In some embodiments of the first to sixth aspects, the anti-DLL3 antibody comprises a human IgG1 heavy chain constant region and a human κ light chain constant region.

In some embodiments of the first to sixth aspects, the anti-DLL3 antibody comprises a wild-type Fc region.

In some embodiments of the first to sixth aspects, the anti-DLL3 antibody or the antigen-binding fragment thereof is engineered to have enhanced ADCC, CDC, and/or ADCP activity. In some specific embodiments, the anti-DLL3 antibody comprises an engineered Fc region that makes the antibody have enhanced ADCC, CDC, and/or ADCP effects.

In some embodiments of the first to sixth aspects, the anti-DLL3 antibody or the antigen-binding fragment thereof is engineered to have reduced ADCC, CDC, and/or ADCP activity. In some specific embodiments, the anti-DLL3 antibody comprises an engineered Fc region that makes the antibody have reduced ADCC, CDC, and/or ADCP effects. For the target DLL3 against which the present disclosure is directed and for *in vivo* use of the antibody, the antibody having reduced or even eliminated ADCC, CDC, and/or ADCP activity may be beneficial because overly strong Fc effects may affect or even diminish the efficacy of the antibody. Methods of reducing or eliminating Fc effects are known in the art, e.g., 1) amino acid residue mutation engineering (primarily to reduce binding to related receptors), 2) glycosylation engineering, and 3) IgG4 type antibody engineering.

In the present disclosure, as a non-limiting example, the Fc region of the anti-DLL3 antibody may comprise an amino acid mutation that reduces ADCC, CDC, and/or ADCP effects.

In a seventh aspect, the present disclosure provides an isolated nucleic acid molecule encoding the anti-DLL3 antibody or the antigen-binding fragment thereof according to the first to sixth aspects.

In some embodiments, the present disclosure provides a combination of isolated polynucleotides, comprising a polynucleotide encoding a light chain of the antibody or the antigen-binding fragment thereof of the present disclosure and a polynucleotide encoding a heavy chain of the antibody or the antigen-binding fragment thereof of the present disclosure. In some embodiments, the polynucleotides are operably linked to regulatory sequences that can be recognized by a host cell transformed with the vector.

In an eighth aspect, the present disclosure provides a vector comprising the nucleic acid molecule according to the seventh aspect.

In some embodiments, the expression vector of the present disclosure comprises the nucleic acid molecule described in the present disclosure or the combination of polynucleotides described in the present disclosure, and the polynucleotides are operably linked to regulatory sequences that allow the polypeptides encoded by the polynucleotides to be expressed in a host cell or a cell-free expression system. The selection of the expression vector depends on the selection of the host cell and can be made so as to have the desired expression and regulatory characteristics in the selected host cell.

The nucleic acid in the vector may be operably linked to one or more expression control sequences. As used herein, "operably linked" means incorporation into a genetic construct such that the expression control sequence effectively controls the expression of the target coding sequence. Examples of expression control sequences include promoters, enhancers, and transcription termination regions. A promoter is an expression control sequence consisting of a region of a DNA molecule typically located within 100 nucleotides upstream of the transcription start site (usually near the start site of RNA polymerase II). In order to place the coding sequence under the control of the promoter, the translation start site of the polypeptide translation reading frame must be located between 1 to about 50 nucleotides downstream of the promoter. Enhancers provide expression specificity in terms of time, location, and level. Unlike promoters, enhancers can function when located at different distances from the transcription site. Enhancers may also be located downstream of the transcription start site. When an RNA polymerase is capable of transcribing a coding sequence into an mRNA, and the mRNA can then be translated into a protein encoded by the coding sequence, the coding sequence is "operably linked" to and "under the control" of an expression control sequence in a cell.

Suitable expression vectors include, but are not limited to, plasmids and viral vectors derived from, for example, phages, baculoviruses, tobacco mosaic virus, herpesviruses, cytomegaloviruses, retroviruses, vaccinia virus, adenoviruses, and adeno-associated viruses. Many vectors and expression systems are commercially available from companies such as Novagen (Madison, WI), Clontech (Palo Alto, CA), Stratagene (La Jolla, CA), and Invitrogen Life Technologies (Carlsbad, CA).

An expression vector may comprise a tag sequence. The tag sequence is typically expressed as a fusion with the encoded polypeptide. Such a tag may be inserted at any position within the polypeptide, including the carboxyl-terminus or the amino-terminus. Examples of useful tags include, but are not limited to, Fc fragments, polyhistidine, green fluorescent protein (GFP), glutathione S-transferase (GST), c-myc, hemagglutinin, FlagTM tag (Kodak, New Haven, CT), maltose E binding protein, and protein A. In some embodiments, the nucleic acid molecule encoding the DLL3 fusion polypeptide is present in a vector comprising a nucleic acid encoding one or more domains of an Ig heavy chain constant region, the domains corresponding, for example, to the amino acid sequences of a hinge region, a CH2 region, and a CH3 region of a human immunoglobulin Cγ1 chain (Fc fragments).

In a ninth aspect, the present disclosure provides a host cell comprising the nucleic acid molecule according to the seventh aspect or the vector according to the eighth aspect.

In some embodiments, the host cell may be a prokaryotic host cell, a eukaryotic host cell, or a phage. The prokaryotic host cell may be *Escherichia coli*, *Bacillus subtilis*, *Streptomyces*, *Proteus mirabilis*, or the like. The eukaryotic host cell may be a fungus such as *Pichia pastoris*, *Saccharomyces cerevisiae*, *Schizosaccharomyces*, or *Trichoderma*, an insect cell such as *Spodoptera frugiperda*, a plant cell such as tobacco, or a mammalian cell such as a BHK cell, a CHO cell, a COS cell, or a myeloma cell. In some embodiments, the host cell is preferably a mammalian cell, more preferably a BHK cell, a CHO cell, an NSO cell, or a COS cell.

In a tenth aspect, the present disclosure provides a bispecific antibody comprising a first antigen-binding site and a second antigen-binding site, wherein one of the antigen-binding sites comprises the anti-DLL3 antibody or the antigen-binding fragment thereof according to the first to sixth aspects; preferably, the first antigen-binding site of the bispecific antibody comprises the anti-DLL3 antibody or the antigen-binding fragment thereof according to the first to sixth aspects, and the second antigen-binding site comprises another anti-DLL3 antibody or an antigen-binding fragment thereof according to the first to sixth aspects; more preferably, the first antigen-binding site and the second antigen-binding site of the bispecific antibody bind to different epitopes of the same antigen.

Preferably, the bispecific antibody of the present disclosure is an IgG-like bispecific antibody, wherein a left arm is the first antigen-binding site, and a right arm is the second antigen-binding site.

Preferably, the first antigen-binding site of the bispecific antibody of the present disclosure comprises a first heavy chain and a first light chain, and the second antigen-binding site comprises a second heavy chain and a second light chain.

In an eleventh aspect, the present disclosure provides a bispecific antibody, wherein:
(1) the first antigen-binding site comprises a heavy chain variable region and a light chain variable region, wherein the heavy chain variable region comprises an HCDR1 whose sequence is the sequence set forth in SEQ ID NO.25 or a sequence having 85%, 90%, 95%, 98%, or 99% identity to the sequence set forth in SEQ ID NO.25, an HCDR2 whose sequence is the sequence set forth in SEQ ID NO.26 or a sequence having 85%, 90%, 95%, 98%, or 99% identity to the sequence set forth in SEQ ID NO.26, and an HCDR3 whose sequence is the sequence set forth in SEQ ID NO.27 or a sequence having 85%, 90%, 95%, 98%, or 99% identity to the sequence set forth in SEQ ID NO.27, and the light chain variable region comprises an LCDR1 whose sequence is the sequence set forth in SEQ ID NO.28 or a sequence having 85%, 90%, 95%, 98%, or 99% identity to the sequence set forth in SEQ ID NO.28, an LCDR2 whose sequence is the sequence set forth in SEQ ID NO.29 or a sequence having 85%, 90%, 95%, 98%, or 99% identity to the sequence set forth in SEQ ID NO.29, and an LCDR3 whose sequence is the sequence set forth in SEQ ID NO.30 or a sequence having 85%, 90%, 95%, 98%, or 99% identity to the sequence set forth in SEQ ID NO.30; the second antigen-binding site comprises a heavy chain variable region and a light chain variable region, wherein the heavy chain variable region comprises an HCDR1 whose sequence is the sequence set forth in SEQ ID NO.17 or a sequence having 85%, 90%, 95%, 98%, or 99% identity to the sequence set forth in SEQ ID NO.17, an HCDR2 whose sequence is the sequence set forth in SEQ ID NO.18 or a sequence having 85%, 90%, 95%, 98%, or 99% identity to the sequence set forth in SEQ ID NO.18, and an HCDR3 whose sequence is the sequence set forth in SEQ ID NO.19 or a sequence having 85%, 90%, 95%, 98%, or 99% identity to the sequence set forth in SEQ ID NO.19, and the light chain variable region comprises an LCDR1 whose sequence is the sequence set forth in SEQ ID NO.20 or a sequence having 85%, 90%, 95%, 98%, or 99% identity to the sequence set forth in SEQ ID NO.20, an LCDR2 whose sequence is the sequence set forth in SEQ ID NO.21 or a sequence having 85%, 90%, 95%, 98%, or 99% identity to the sequence set forth in SEQ ID NO.21, and an LCDR3 whose sequence is the sequence set forth in SEQ ID NO.22 or a sequence having 85%, 90%, 95%, 98%, or 99% identity to the sequence set forth in SEQ ID NO.22;
(2) the first antigen-binding site comprises a heavy chain variable region and a light chain variable region, wherein the heavy chain variable region comprises an HCDR1 whose sequence is the sequence set forth in SEQ ID NO.25 or a sequence having 85%, 90%, 95%, 98%, or 99% identity to the sequence set forth in SEQ ID NO.25, an HCDR2 whose sequence is the sequence set forth in SEQ ID NO.26 or a sequence having 85%, 90%, 95%, 98%, or 99% identity to the sequence set forth in SEQ ID NO.26, and an HCDR3 whose sequence is the sequence set forth in SEQ ID NO.27 or a sequence having 85%, 90%, 95%, 98%, or 99% identity to the sequence set forth in SEQ ID NO.27, and the light chain variable region comprises an LCDR1 whose sequence is the sequence set forth in SEQ ID NO.28 or a sequence having 85%, 90%, 95%, 98%, or 99% identity to the sequence set forth in SEQ ID NO.28, an LCDR2 whose sequence is the sequence set forth in SEQ ID NO.29 or a sequence having 85%, 90%, 95%, 98%, or 99% identity to the sequence set forth in SEQ ID NO.29, and an LCDR3 whose sequence is the sequence set forth in SEQ ID NO.30 or a sequence having 85%, 90%, 95%, 98%, or 99% identity to the sequence set forth in SEQ ID NO.30; the second antigen-binding site comprises a heavy chain variable region and a light chain variable region, wherein the heavy chain variable region comprises an HCDR1 whose sequence is the sequence set forth in SEQ ID NO.1 or a sequence having 85%, 90%, 95%, 98%, or 99% identity to the sequence set forth in SEQ ID NO.1, an HCDR2 whose sequence is the sequence set forth in SEQ ID NO.2 or a sequence having 85%, 90%, 95%, 98%, or 99% identity to the sequence set forth in SEQ ID NO.2, and an HCDR3 whose sequence is the sequence set forth in SEQ ID NO.3 or a sequence having 85%, 90%, 95%, 98%, or 99% identity to the sequence set forth in SEQ ID NO.3, and the light chain variable region comprises an LCDR1 whose sequence is the sequence set forth in SEQ ID NO.4 or a sequence having 85%, 90%, 95%, 98%, or 99% identity to the sequence set forth in SEQ ID NO.4, an LCDR2 whose sequence is the sequence set forth in SEQ ID NO.5 or a sequence having 85%, 90%, 95%, 98%, or 99% identity to the sequence set forth in SEQ ID NO.5, and an LCDR3 whose sequence is the sequence set forth in SEQ ID NO.6 or a sequence having 85%, 90%, 95%, 98%, or 99% identity to the sequence set forth in SEQ ID NO.6; or
(3) the first antigen-binding site comprises a heavy chain variable region and a light chain variable region, wherein the heavy chain variable region comprises an HCDR1 whose sequence is the sequence set forth in SEQ ID NO.17 or a sequence having 85%, 90%, 95%, 98%, or 99% identity to the sequence set forth in SEQ ID NO.17, an HCDR2 whose sequence is the sequence set forth in SEQ ID NO.18 or a sequence having 85%, 90%, 95%, 98%, or 99% identity to the sequence set forth in SEQ ID NO.18, and an HCDR3 whose sequence is the sequence set forth in SEQ ID NO.19 or a sequence having 85%, 90%, 95%, 98%, or 99% identity to the sequence set forth in SEQ ID NO.19, and the light chain variable region comprises an LCDR1 whose sequence is the sequence set forth in SEQ ID NO.20 or a sequence having 85%, 90%, 95%, 98%, or 99% identity to the sequence set forth in SEQ ID NO.20, an LCDR2 whose sequence is the sequence set forth in SEQ ID NO.21 or a sequence having 85%, 90%, 95%, 98%, or 99% identity to the sequence set forth in SEQ ID NO.21, and an LCDR3 whose sequence is the sequence set forth in SEQ ID NO.22 or a sequence having 85%, 90%, 95%, 98%, or 99% identity to the sequence set forth in SEQ ID NO.22; the second antigen-binding site comprises a heavy chain variable region and a light chain variable region, wherein the heavy chain variable region comprises an HCDR1 whose sequence is the sequence set forth in SEQ ID NO.1 or a sequence having 85%, 90%, 95%, 98%, or 99% identity to the sequence set forth in SEQ ID NO.1, an HCDR2 whose sequence is the sequence set forth in SEQ ID NO.2 or a sequence having 85%, 90%, 95%, 98%, or 99% identity to the sequence set forth in SEQ ID NO.2, and an HCDR3 whose sequence is the sequence set forth in SEQ ID NO.3 or a sequence having 85%, 90%, 95%, 98%, or 99% identity to the sequence set forth in SEQ ID NO.3, and the light chain variable region comprises an LCDR1 whose sequence is the sequence set forth in SEQ ID NO.4 or a sequence having 85%, 90%, 95%, 98%, or 99% identity to the sequence set forth in SEQ ID NO.4, an LCDR2 whose sequence is the sequence set forth in SEQ ID NO.5 or a sequence having 85%, 90%, 95%, 98%, or 99% identity to the sequence set forth in SEQ ID NO.5, and an LCDR3 whose sequence is the sequence set forth in SEQ ID NO.6 or a sequence having 85%, 90%, 95%, 98%, or 99% identity to the sequence set forth in SEQ ID NO.6.

Further, the present disclosure provides a bispecific antibody, wherein:
(1) the first antigen-binding site comprises a heavy chain variable region and a light chain variable region, wherein the sequence of the heavy chain variable region is the sequence set forth in SEQ ID NO.31 or a sequence having 85%, 90%, 95%, 98%, or 99% identity to the sequence set forth in SEQ ID NO.31, and the sequence of the light chain variable region is the sequence set forth in SEQ ID NO.32 or a sequence having 85%, 90%, 95%, 98%, or 99% identity to the sequence set forth in SEQ ID NO.32; the second antigen-binding site comprises a heavy chain variable region and a light chain variable region, wherein the sequence of the heavy chain variable region is the sequence set forth in SEQ ID NO.23 or a sequence having 85%, 90%, 95%, 98%, or 99% identity to the sequence set forth in SEQ ID NO.23, and the sequence of the light chain variable region is the sequence set forth in SEQ ID NO.24 or a sequence having 85%, 90%, 95%, 98%, or 99% identity to the sequence set forth in SEQ ID NO.24;
(2) the first antigen-binding site comprises a heavy chain variable region and a light chain variable region, wherein the sequence of the heavy chain variable region is the sequence set forth in SEQ ID NO.31 or a sequence having 85%, 90%, 95%, 98%, or 99% identity to the sequence set forth in SEQ ID NO.31, and the sequence of the light chain variable region is the sequence set forth in SEQ ID NO.32 or a sequence having 85%, 90%, 95%, 98%, or 99% identity to the sequence set forth in SEQ ID NO.32; the second antigen-binding site comprises a heavy chain variable region and a light chain variable region, wherein the sequence of the heavy chain variable region is the sequence set forth in SEQ ID NO.7 or a sequence having 85%, 90%, 95%, 98%, or 99% identity to the sequence set forth in SEQ ID NO.7, and the sequence of the light chain variable region is the sequence set forth in SEQ ID NO.8 or a sequence having 85%, 90%, 95%, 98%, or 99% identity to the sequence set forth in SEQ ID NO.8;
(3) the first antigen-binding site comprises a heavy chain variable region and a light chain variable region, wherein the sequence of the heavy chain variable region is the sequence set forth in SEQ ID NO.23 or a sequence having 85%, 90%, 95%, 98%, or 99% identity to the sequence set forth in SEQ ID NO.23, and the sequence of the light chain variable region is the sequence set forth in SEQ ID NO.24 or a sequence having 85%, 90%, 95%, 98%, or 99% identity to the sequence set forth in SEQ ID NO.24; the second antigen-binding site comprises a heavy chain variable region and a light chain variable region, wherein the sequence of the heavy chain variable region is the sequence set forth in SEQ ID NO.7 or a sequence having 85%, 90%, 95%, 98%, or 99% identity to the sequence set forth in SEQ ID NO.7, and the sequence of the light chain variable region is the sequence set forth in SEQ ID NO.8 or a sequence having 85%, 90%, 95%, 98%, or 99% identity to the sequence set forth in SEQ ID NO.8.

In the present disclosure, the bispecific antibody further comprises a constant region sequence.

In the present disclosure, the bispecific antibody is an IgG1 antibody.

Preferably, the bispecific antibody described in the present disclosure carries a CH1-CL chain exchange between the first heavy chain and the first light chain or between the second heavy chain and the second light chain.

Preferably, an Fc region of the bispecific antibody described in the present disclosure comprises an amino acid mutation that reduces ADCC, CDC, and/or ADCP effects.

Further preferably, the Fc region of the bispecific antibody described in the present disclosure comprises, in a CH3 region, knob- and/or hole-forming amino acid mutations that facilitate purification of the bispecific antibody; further preferably, the hole-forming mutations are Y349C, T366S, L368A, and Y407V, and the knob-forming mutations are S354C and T366W.

Further preferably, the present disclosure provides a bispecific antibody, wherein:
(1) the bispecific antibody comprises 2 heavy chains and 2 light chains, wherein the amino acid sequence of a heavy chain 1 is set forth in SEQ ID NO.41, the amino acid sequence of a heavy chain 2 is set forth in SEQ ID NO.43, the amino acid sequence of a light chain 1 is set forth in SEQ ID NO.46, and the amino acid sequence of a light chain 2 is set forth in SEQ ID NO.48;
(2) the bispecific antibody comprises 2 heavy chains and 2 light chains, wherein the amino acid sequence of a heavy chain 1 is set forth in SEQ ID NO.41, the amino acid sequence of a heavy chain 2 is set forth in SEQ ID NO.50, the amino acid sequence of a light chain 1 is set forth in SEQ ID NO.46, and the amino acid sequence of a light chain 2 is set forth in SEQ ID NO.51;
(3) the bispecific antibody comprises 2 heavy chains and 2 light chains, wherein the amino acid sequence of a heavy chain 1 is set forth in SEQ ID NO.52, the amino acid sequence of a heavy chain 2 is set forth in SEQ ID NO.50, the amino acid sequence of a light chain 1 is set forth in SEQ ID NO.53, and the amino acid sequence of a light chain 2 is set forth in SEQ ID NO.51.

In a twelfth aspect, the present disclosure provides a nucleic acid molecule encoding the aforementioned bispecific antibody.

In some embodiments, the present disclosure provides a combination of isolated polynucleotides, comprising a polynucleotide encoding a light chain of the antibody or the antigen-binding fragment thereof of the present disclosure and a polynucleotide encoding a heavy chain of the antibody or the antigen-binding fragment thereof of the present disclosure. In some embodiments, the polynucleotides are operably linked to regulatory sequences that can be recognized by a host cell transformed with the vector.

In a thirteenth aspect, the present disclosure provides an expression vector comprising the nucleic acid molecule according to the twelfth aspect.

In some embodiments, the expression vector of the present disclosure comprises the nucleic acid molecule described in the present disclosure or the combination of polynucleotides described in the present disclosure, and the polynucleotides are operably linked to regulatory sequences that allow the polypeptides encoded by the polynucleotides to be expressed in a host cell or a cell-free expression system. The selection of the expression vector depends on the selection of the host cell and can be made so as to have the desired expression and regulatory characteristics in the selected host cell.

The nucleic acid in the vector may be operably linked to one or more expression control sequences. As used herein, "operably linked" means incorporation into a genetic construct such that the expression control sequence effectively controls the expression of the target coding sequence. Examples of expression control sequences include promoters, enhancers, and transcription termination regions. A promoter is an expression control sequence consisting of a region of a DNA molecule typically located within 100 nucleotides upstream of the transcription start site (usually near the start site of RNA polymerase II). In order to place the coding sequence under the control of the promoter, the translation start site of the polypeptide translation reading frame must be located between 1 to about 50 nucleotides downstream of the promoter. Enhancers provide expression specificity in terms of time, location, and level. Unlike promoters, enhancers can function when located at different distances from the transcription site. Enhancers may also be located downstream of the transcription start site. When an RNA polymerase is capable of transcribing a coding sequence into an mRNA, and the mRNA can then be translated into a protein encoded by the coding sequence, the coding sequence is "operably linked" to and "under the control" of an expression control sequence in a cell.

Suitable expression vectors include, but are not limited to, plasmids and viral vectors derived from, for example, phages, baculoviruses, tobacco mosaic virus, herpesviruses, cytomegaloviruses, retroviruses, vaccinia virus, adenoviruses, and adeno-associated viruses. Many vectors and expression systems are commercially available from companies such as Novagen (Madison, WI), Clontech (Palo Alto, CA), Stratagene (La Jolla, CA), and Invitrogen Life Technologies (Carlsbad, CA).

An expression vector may comprise a tag sequence. The tag sequence is typically expressed as a fusion with the encoded polypeptide. Such a tag may be inserted at any position within the polypeptide, including the carboxyl-terminus or the amino-terminus. Examples of useful tags include, but are not limited to, Fc fragments, polyhistidine, green fluorescent protein (GFP), glutathione S-transferase (GST), c-myc, hemagglutinin, FlagTM tag (Kodak, New Haven, CT), maltose E binding protein, and protein A. In some embodiments, the nucleic acid molecule encoding the DLL3 fusion polypeptide is present in a vector comprising a nucleic acid encoding one or more domains of an Ig heavy chain constant region, the domains corresponding, for example, to the amino acid sequences of a hinge region, a CH2 region, and a CH3 region of a human immunoglobulin Cγ1 chain (Fc fragments).

In a fourteenth aspect, the present disclosure provides a host cell comprising the expression vector according to the thirteenth aspect.

In some embodiments, the host cell may be a prokaryotic host cell, a eukaryotic host cell, or a phage. The prokaryotic host cell may be *Escherichia coli*, *Bacillus subtilis*, *Streptomyces*, *Proteus mirabilis*, or the like. The eukaryotic host cell may be a fungus such as *Pichia pastoris*, *Saccharomyces cerevisiae*, *Schizosaccharomyces*, or *Trichoderma*, an insect cell such as *Spodoptera frugiperda*, a plant cell such as tobacco, or a mammalian cell such as a BHK cell, a CHO cell, a COS cell, or a myeloma cell. In some embodiments, the host cell is preferably a mammalian cell, more preferably a BHK cell, a CHO cell, an NSO cell, or a COS cell.

In a fifteenth aspect, the present disclosure provides an antibody-drug conjugate comprising the aforementioned anti-DLL3 antibody or the antigen-binding fragment thereof or the aforementioned bispecific antibody.

Preferably, the antibody-drug conjugate is obtained by conjugating a therapeutic agent to the aforementioned anti-DLL3 antibody or the antigen-binding fragment thereof or the aforementioned bispecific antibody.

In the present disclosure, the therapeutic agent may be an anti-tumor drug, such as a cytotoxic drug, an immunopotentiator, or a radioisotope.

In some embodiments, the types of the cytotoxic drug include a tubulin inhibitor, a DNA topoisomerase inhibitor, a DNA-damaging agent, an antimetabolite, or an anti-tumor antibiotic. In some embodiments, the tubulin inhibitor includes, but is not limited to, auristatin derivatives (e.g., MMAE (monomethyl auristatin E) or MMAF (monomethyl auristatin F)) or mertansine alkaloid derivatives (e.g., DM1, DM4, ansamitocin, mertansine, or dolastatin and derivatives thereof).

In some embodiments, the DNA topoisomerase inhibitor is a camptothecin analog or a DNA topoisomerase I inhibitor or a derivative thereof, such as DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, or N-[2-(dimethylamino)ethyl]-4-acridinecarboxamide.

In some embodiments, the DNA-damaging agent includes, but is not limited to, calicheamicins, duocarmycins, and anthramycin-derived PBDs (pyrrolobenzodiazepines).

In some embodiments, the antimetabolite includes, but is not limited to, methotrexate, 6-mercaptopurine, or 5-fluorouracil.

In some embodiments, the anti-tumor antibiotic includes, but is not limited to, polypeptide antibiotics (e.g., actinomycin D or bleomycin) or anthraquinone drugs (e.g., doxorubicin or mitoxantrone hydrochloride).

In some embodiments, the immunopotentiator includes, but is not limited to, levamisole, pidotimod, imiquimod, isoprinosine, polyinosinic:polycytidylic acid, or polyinosinic:polyuridylic acid.

In some embodiments, the radionuclide is a diagnostic radionuclide or a therapeutic radionuclide. According to an embodiment of the present disclosure, the diagnostic radionuclide is at least one of ⁶⁸Ga, ⁶⁴Cu, ¹⁸F, ⁸⁶Y, ⁸⁹Zr, ¹¹¹In, ^{99m}Tc, ¹¹C, ¹²³I, ¹²⁵I, and ¹²⁴I. According to an embodiment of the present disclosure, the therapeutic radionuclide is at least one of ¹⁷⁷Lu, ⁹⁰Y, ¹²⁵I, ¹³¹I, ²¹¹At, ¹¹¹In, ¹⁵³Sm, ¹⁸⁶Re, ¹⁸⁸Re, ⁶⁷Cu, ²²⁵Ac, ²¹³Bi, ²¹²Bi, and ²¹²Pb.

In some embodiments, the antibody-drug conjugate described in the present disclosure has a structure represented by formula (I):

A-(L-D)_{d} (I)

wherein A is the anti-DLL3 antibody or the antigen-binding fragment thereof according to the first to sixth aspects of the present disclosure or the bispecific antibody according to the tenth to eleventh aspects of the present disclosure;
L is a linker moiety with one end linked to A and one end linked to a therapeutic agent D;
d represents a molar ratio of the therapeutic agent to A (also known as DAR, i.e., drug-to-antibody ratio), and is selected from an integer and a decimal from 1 to 12 (e.g., 1, 2, 3, 4, 5, 6, 7, 8, 9, 10, 11, or 12);
when d is a decimal, d refers to an average number of linker-therapeutic agent (L-D) compounds conjugated to each A.

In some embodiments, A is selected from antibody 22B12E5, antibody 25G10A9, antibody 31B6G8, antibody 33B10E8, antibody 44B8H2, Hab22B12E5, Hab31B6G8, Hab33B10E8, 33+31, 33+22, and 31+22.

In some embodiments, the anti-DLL3 antibody or the antigen-binding fragment thereof of the present disclosure is covalently linked to the therapeutic agent by a linker.

In some embodiments, L is a cleavable linker.

In some embodiments, L is cleavable under intracellular conditions or microenvironmental conditions.

In one embodiment, L is hydrolyzable at a pH of less than 5.5.

In some embodiments, L is cleavable by an intracellular protease.

In some embodiments, L is a cathepsin-cleavable linker.

In some embodiments, L includes a dipeptide or a tetrapeptide.

In some embodiments, the dipeptide is selected from valine (Val)-citrulline (Cit) and valine (Val)-alanine (Ala), and the tetrapeptide is selected from glycine (Gly)-glycine (Gly)-phenylalanine (Phe)-glycine (Gly).

In some embodiments, the antibody is linked to the linker by a cysteine sulfhydryl group of the antibody.

In one embodiment, the antibody is linked to the linker by an amino group of the antibody (particularly an amino group of a glutamine residue).

In some embodiments, L is a cleavable linker, including but not limited to an acid-labile linker (e.g., a hydrazone linker), a disulfide bond-containing linker, a peptidase-sensitive linker (e.g., a peptide linker comprising amino acids, such as valine and/or citrulline, e.g., citrulline-valine or phenylalanine-lysine), a photo-labile linker, a dimethyl linker, a thioether linker, a hydrophilic linker designed to avoid multidrug transporter-mediated resistance, or the like.

In some embodiments, -L- is selected from -L₁-L₂-L₃-L₄-, wherein L₁ is a covalent linking unit covalently linked to A, L₂ is an extension unit, L₃ is selected from a peptide residue consisting of 2-8 amino acids, and L₄ is a bond or a self-cleaving fragment.

In some embodiments, L₁ is selected from 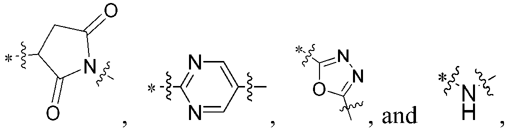 wherein * indicates linking to A.

In some embodiments, L₂ is selected from -L₂ₐ-, -L₂ₐ-C(O)-, and -L₂ₐ-NH-C(O)-L_{2b}-C(O)-, wherein L₂ₐ and L_{2b} are each independently selected from -C₁-C₆ alkylene-, -C₁-C₃ alkylene-C₃-C₆ cycloalkylene-, -ethynylene-C₁-C₆ alkylene-, -(CH₂CH₂O)ₙ-, -(CH₂CH₂O)ₙC₁₋₃ alkylene-, -C₁₋₃ alkylene(CH₂CH₂O)ₙC₁₋₃ alkylene-, -C₁₋₃ alkylene-O-C₁₋₃ alkylene, phenyl, and -phenyl-C₁₋₃ alkylene-, wherein n is selected from an integer of 1-12 (preferably 3-8, e.g., 1, 2, 3, 4, 5, 6, 7, 8, 9, 10, 11, or 12).

In some embodiments, L₂ is selected from -L₂ₐ-, -L₂ₐ-C(O)-, and -L₂ₐ-NH-C(O)-L_{2b}-C(O)-, wherein L₂ₐ and L_{2b} are each independently selected from -(CH₂)₅-, phenylene, -phenyl-CH₂-, and - CH₂-O-CH₂-.

In some embodiments, L₂ is selected from: 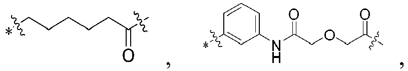 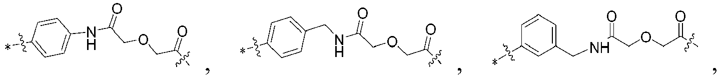 (CH₂CH₂O)ₙCH₂C(O)-, - (CH₂CH₂O)ₙCH₂CH₂-, 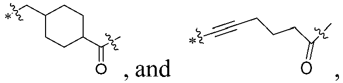 wherein the * position is linked to L₁, and n is as defined above.

In some embodiments, L₃ is selected from a peptide residue consisting of 2-8 amino acids, and the amino acids are selected from phenylalanine, valine, alanine, serine, glutamine, arginine, lysine, asparagine, glutamic acid, proline, citrulline, aspartic acid, and glycine.

In some embodiments, L₃ is selected from: 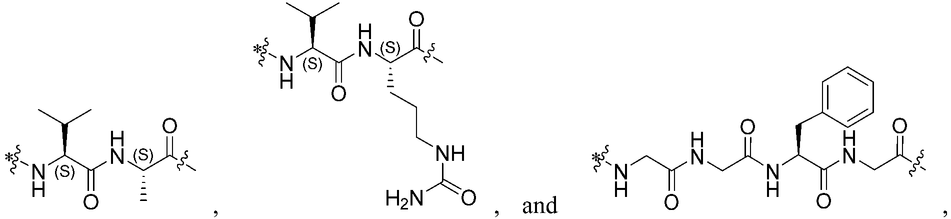 wherein the * position is linked to L₂.

In some embodiments, L₄ is selected from: a bond, -NCH₂-, and 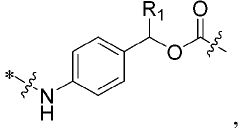 wherein the * position is linked to L₃, and R₁ is selected from hydrogen and 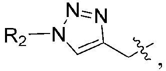 wherein R₂ is selected from: 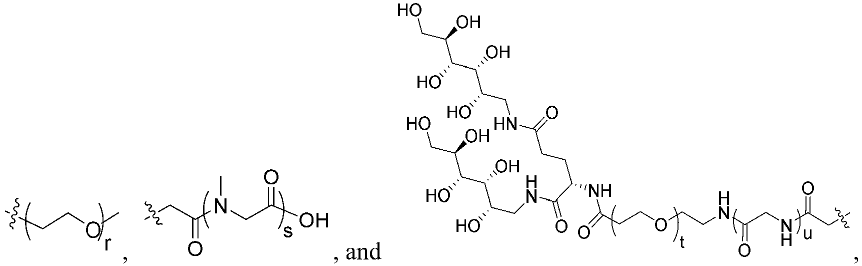 wherein r, s, t, and u are each independently selected from an integer of 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50), and r is preferably an integer of 8-24.

In some embodiments, R₂ is selected from the following structures: 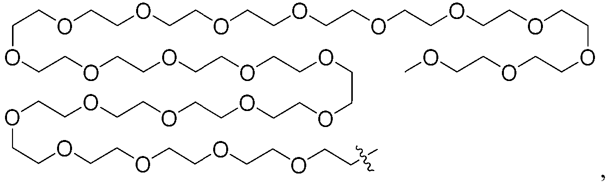 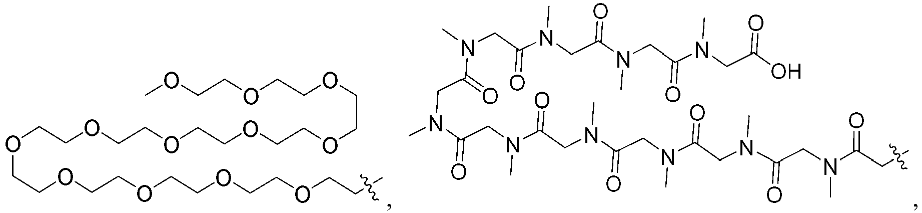 and

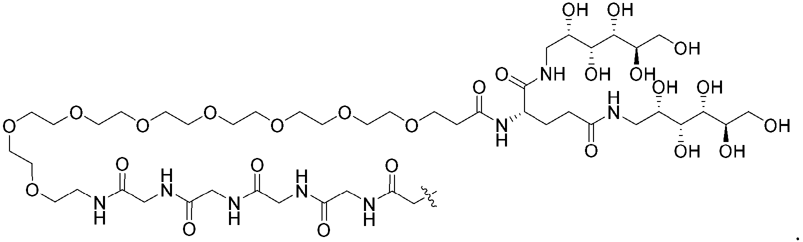

In some embodiments, L₄ is selected from -NCH₂-,

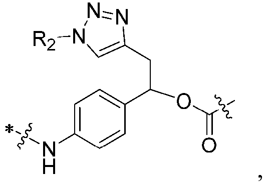

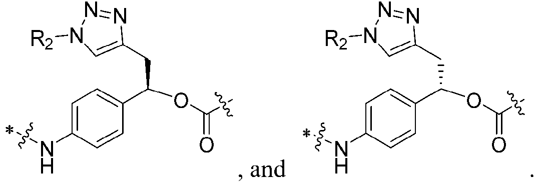

In some embodiments, L₄ is selected from: -NCH₂-,

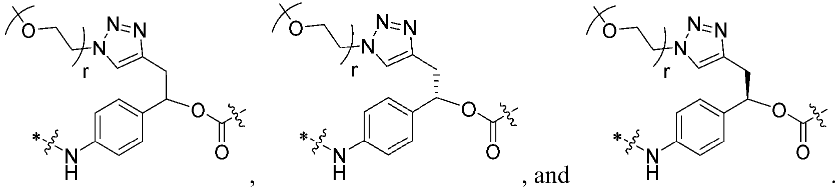

In some embodiments, L is selected from: 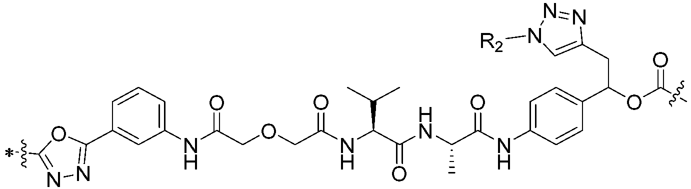 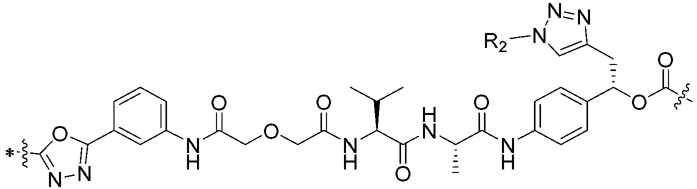 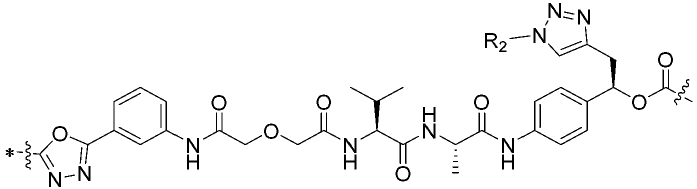 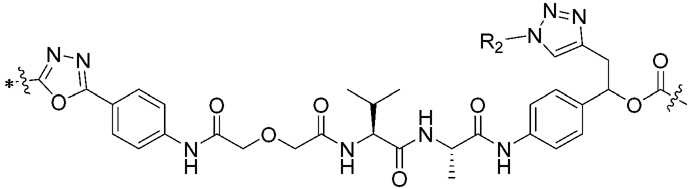 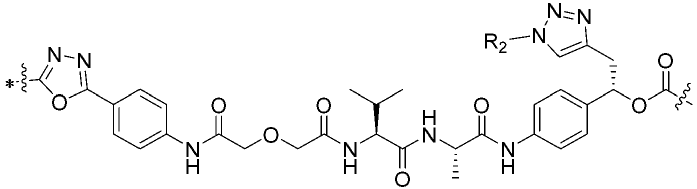 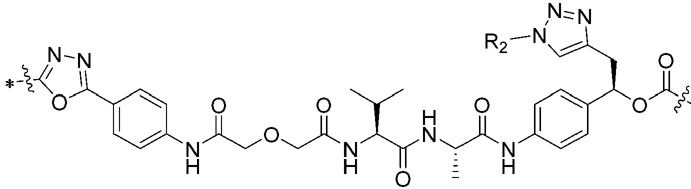 and 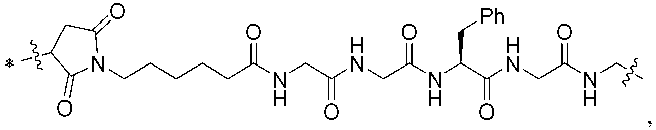 wherein the * position is linked to A.

In some embodiments, L is selected from: 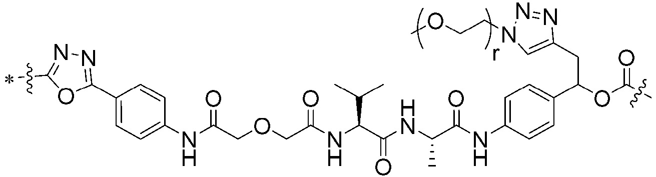 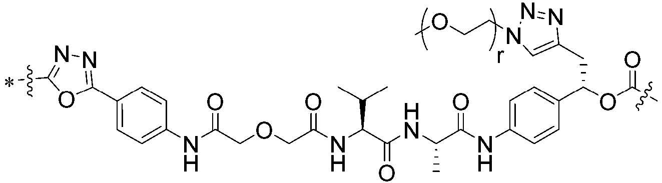 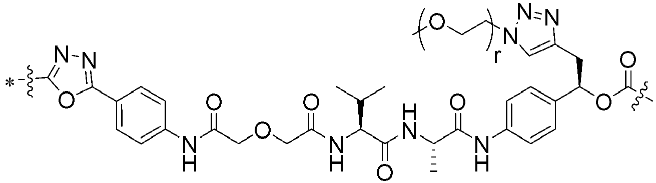 and 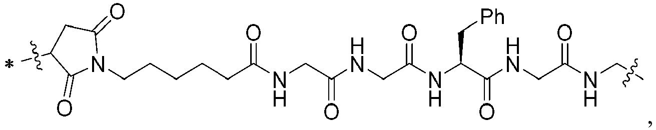 wherein the * position is linked to A.

In some embodiments, D is selected from: 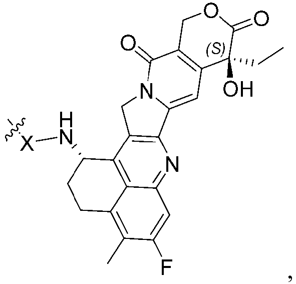 wherein X is selected from a bond and 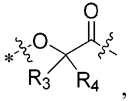 wherein R₃ and R₄ are each independently selected from H, C₁₋₃ alkyl (preferably methyl, ethyl, n-propyl, or isopropyl), and 3-6 membered cycloalkyl (preferably cyclopropyl, cyclobutyl, cyclopentyl, or cyclohexyl), or R₃ and R₄, together with the carbon atom to which they are attached, form 3-6 membered cycloalkyl (preferably cyclopropyl, cyclobutyl, cyclopentyl, or cyclohexyl), wherein the * position is linked to L₄.

In some embodiments, X is selected from a bond,

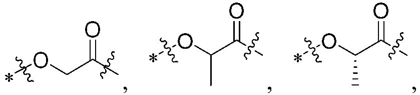

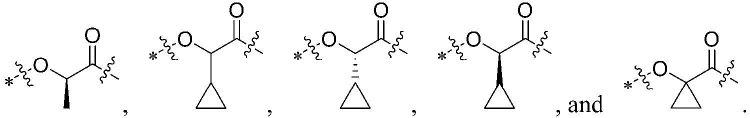

In some embodiments, D is selected from: 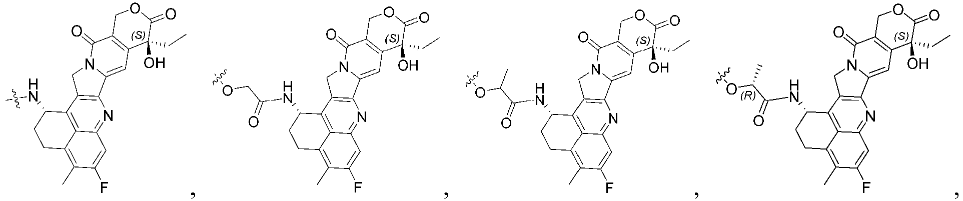 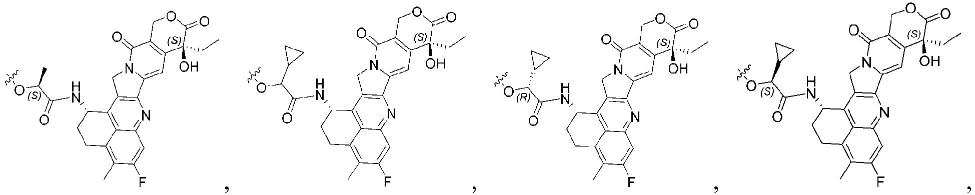 and

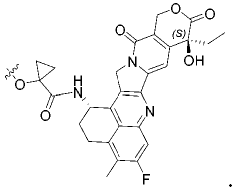

In some embodiments, the antibody-drug conjugate of formula I has the following structure: 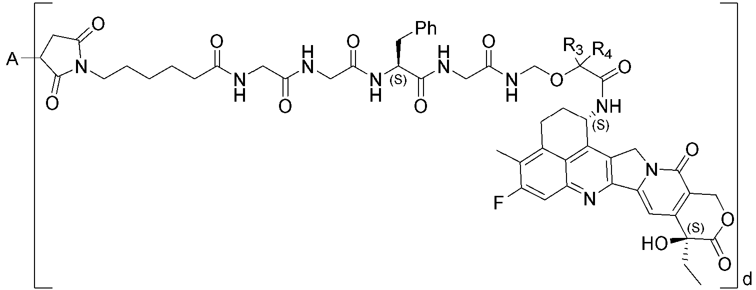 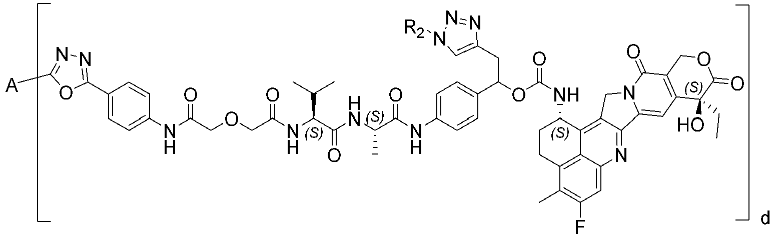 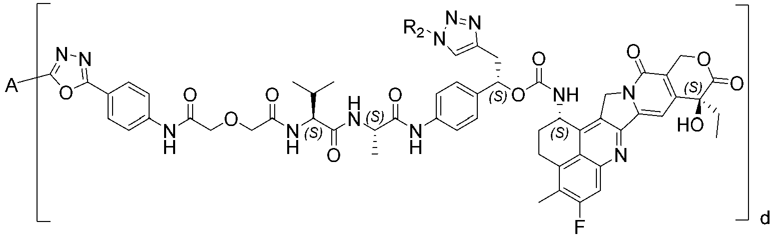 or

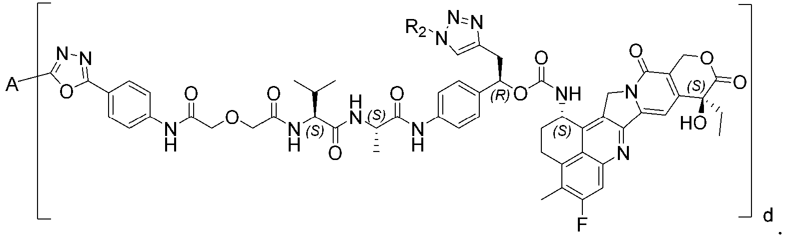

In some embodiments, the antibody-drug conjugate of formula I has the following structure: 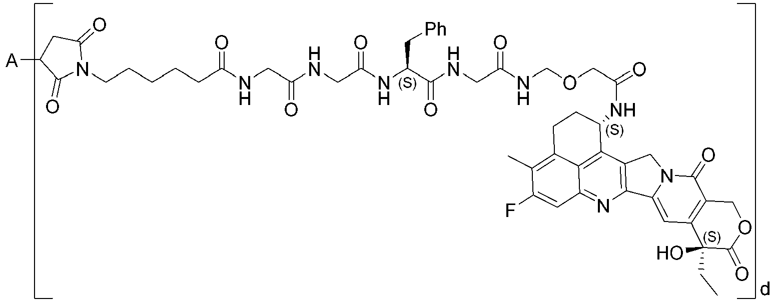 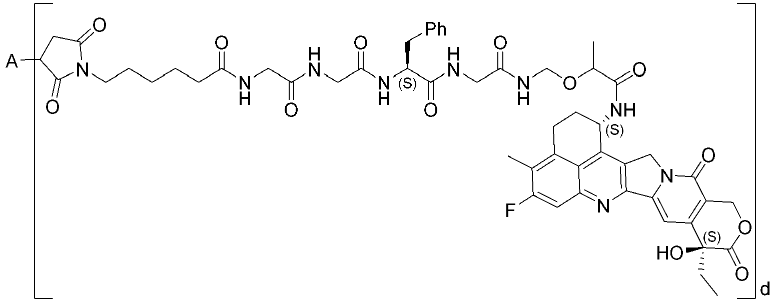 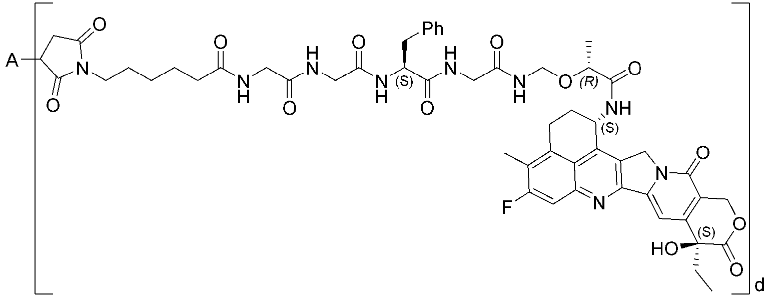 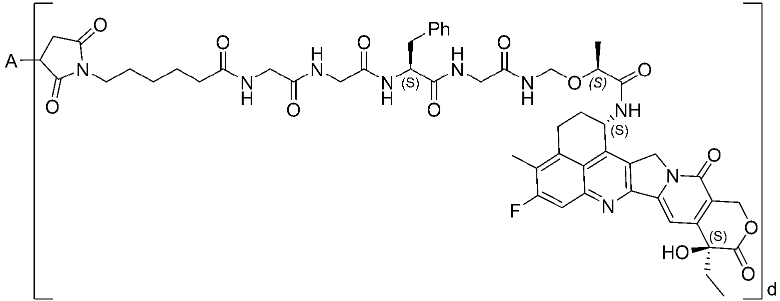 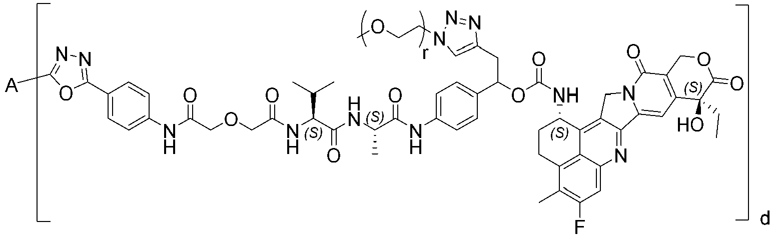 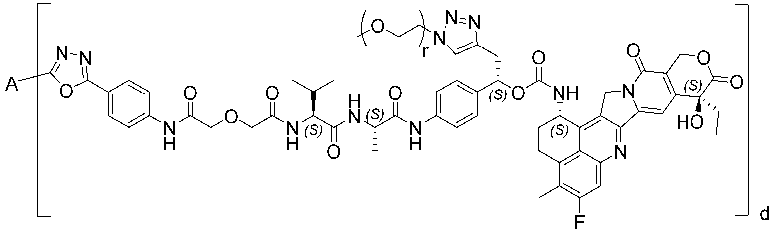 or 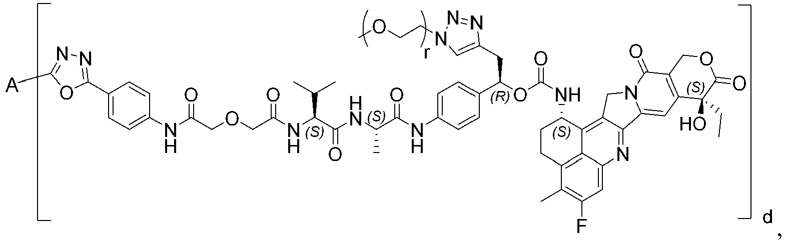 wherein r is selected from an integer of 1-50, preferably an integer of 8-24, and preferably 8, 9, 10, 11, 12, 13, 14, 15, 16, 17, 18, 19, 20, 21, 22, 23, or 24.

In some embodiments, in the antibody-drug conjugate of formula I, A is selected from Hab33B10E8.

In a sixteenth aspect, the present disclosure provides a fusion protein comprising the anti-DLL3 antibody or the antigen-binding fragment thereof according to the first to sixth aspects.

Examples of the fusion protein described in the present disclosure include Fab fusion proteins, Fc fusion proteins, single-chain antibody (scFv) fusion proteins, etc., which are named according to the site to which an effector protein (e.g., a cytokine) is fused.

In a seventeenth aspect, the present disclosure provides a pharmaceutical composition comprising the aforementioned anti-DLL3 antibody or the antigen-binding fragment thereof, the aforementioned bispecific antibody, the aforementioned antibody-drug conjugate, or the aforementioned fusion protein.

In the present disclosure, the pharmaceutical composition according to the seventeenth aspect further comprises a pharmaceutically acceptable carrier.

In an eighteenth aspect, the present disclosure provides use of the aforementioned anti-DLL3 antibody or the antigen-binding fragment thereof, the aforementioned bispecific antibody, the aforementioned antibody-drug conjugate, the aforementioned fusion protein, or the aforementioned pharmaceutical composition in the preparation of a medicament for treating a tumor.

In some embodiments, the tumor is a tumor expressing DLL3 (DLL3+) on the surface of tumor cells.

In some embodiments, the tumor is a tumor highly expressing DLL3 (DLL3+) on the surface of tumor cells. In some embodiments, the tumor highly expressing DLL3 (DLL3+) means that at least 60% of the tumor cells in a tumor cell population express DLL3. In some embodiments, the tumor highly expressing DLL3 (DLL3+) means that at least 70% of the tumor cells in a tumor cell population express DLL3. In some embodiments, the tumor highly expressing DLL3 (DLL3+) means that at least 80% of the tumor cells in a tumor cell population express DLL3. In some embodiments, the tumor highly expressing DLL3 (DLL3+) means that at least 90% of the tumor cells in a tumor cell population express DLL3. In some embodiments, the tumor highly expressing DLL3 (DLL3+) means that at least 95% of the tumor cells in a tumor cell population express DLL3. In some embodiments, the tumor highly expressing DLL3 (DLL3+) means that at least 98% of the tumor cells in a tumor cell population express DLL3. In some embodiments, the tumor highly expressing DLL3 (DLL3+) means that at least 99% of the tumor cells in a tumor cell population express DLL3.

In some embodiments, the tumor is a solid tumor.

In some embodiments, the tumor is a hematologic tumor.

In some embodiments, the tumor is a malignant tumor.

In some embodiments, the tumor is cancer.

In some embodiments, the tumor is lung cancer, more preferably small cell lung cancer.

In a nineteenth aspect, the present disclosure provides a method for treating a tumor, particularly a tumor expressing DLL3 (DLL3+) on the surface of tumor cells, in an individual, comprising administering to the individual a therapeutically effective amount of the aforementioned anti-DLL3 antibody or the antigen-binding fragment thereof, the aforementioned bispecific antibody, the aforementioned antibody-drug conjugate, the aforementioned fusion protein, or the aforementioned pharmaceutical composition.

In some embodiments, the tumor is a tumor expressing DLL3 (DLL3+) on the surface of tumor cells.

In some embodiments, the tumor is a tumor highly expressing DLL3 (DLL3+) on the surface of tumor cells. In some embodiments, the tumor highly expressing DLL3 (DLL3+) means that at least 60% of the tumor cells in a tumor cell population express DLL3. In some embodiments, the tumor highly expressing DLL3 (DLL3+) means that at least 70% of the tumor cells in a tumor cell population express DLL3. In some embodiments, the tumor highly expressing DLL3 (DLL3+) means that at least 80% of the tumor cells in a tumor cell population express DLL3. In some embodiments, the tumor highly expressing DLL3 (DLL3+) means that at least 90% of the tumor cells in a tumor cell population express DLL3. In some embodiments, the tumor highly expressing DLL3 (DLL3+) means that at least 95% of the tumor cells in a tumor cell population express DLL3. In some embodiments, the tumor highly expressing DLL3 (DLL3+) means that at least 98% of the tumor cells in a tumor cell population express DLL3. In some embodiments, the tumor highly expressing DLL3 (DLL3+) means that at least 99% of the tumor cells in a tumor cell population express DLL3.

In some embodiments, the tumor is a solid tumor.

In some embodiments, the tumor is a hematologic tumor.

In some embodiments, the tumor is a malignant tumor.

In some embodiments, the tumor is cancer.

In some embodiments, the tumor is lung cancer, more preferably small cell lung cancer.

In a twentieth aspect, the present disclosure provides a therapeutically effective amount of the aforementioned anti-DLL3 antibody or the antigen-binding fragment thereof, the aforementioned bispecific antibody, the aforementioned antibody-drug conjugate, the aforementioned fusion protein, or the aforementioned pharmaceutical composition for use in treating a tumor, particularly a tumor expressing DLL3 (DLL3+) on the surface of tumor cells, in an individual.

In some embodiments, the tumor is a tumor expressing DLL3 (DLL3+) on the surface of tumor cells.

In some embodiments, the tumor is a tumor highly expressing DLL3 (DLL3+) on the surface of tumor cells. In some embodiments, the tumor highly expressing DLL3 (DLL3+) means that at least 60% of the tumor cells in a tumor cell population express DLL3. In some embodiments, the tumor highly expressing DLL3 (DLL3+) means that at least 70% of the tumor cells in a tumor cell population express DLL3. In some embodiments, the tumor highly expressing DLL3 (DLL3+) means that at least 80% of the tumor cells in a tumor cell population express DLL3. In some embodiments, the tumor highly expressing DLL3 (DLL3+) means that at least 90% of the tumor cells in a tumor cell population express DLL3. In some embodiments, the tumor highly expressing DLL3 (DLL3+) means that at least 95% of the tumor cells in a tumor cell population express DLL3. In some embodiments, the tumor highly expressing DLL3 (DLL3+) means that at least 98% of the tumor cells in a tumor cell population express DLL3. In some embodiments, the tumor highly expressing DLL3 (DLL3+) means that at least 99% of the tumor cells in a tumor cell population express DLL3.

In some embodiments, the tumor is a solid tumor.

In some embodiments, the tumor is a hematologic tumor.

In some embodiments, the tumor is a malignant tumor.

In some embodiments, the tumor is cancer.

In some embodiments, the tumor is lung cancer, more preferably small cell lung cancer.

### BRIEF DESCRIPTION OF THE DRAWINGS

FIG. 1 shows the results of an experiment for assessing the binding of chimeric antibodies to soluble human DLL3 protein.
FIG. 2 shows the results of an experiment for assessing the binding of chimeric antibodies to soluble cynomolgus monkey DLL3 protein.
FIG. 3 shows the results of an experiment for assessing the binding of chimeric antibodies to soluble mouse DLL3 protein.
FIG. 4A shows the results of an experiment for assessing the binding of chimeric antibodies to soluble human DLL1 protein.
FIG. 4B shows the results of an experiment for assessing the binding of chimeric antibodies to soluble human DLL4 protein.
FIG. 5A shows the results of an experiment for assessing the binding of chimeric antibodies to the truncated protein human DLL3-B (Ala 176-Leu 492).
FIG. 5B shows the results of an experiment for assessing the binding of chimeric antibodies to the truncated protein human DLL3-C (Ser 312-Leu 492).
FIG. 5C shows epitope recognition results for chimeric antibodies.
FIG. 6A shows the results of an experiment for assessing the killing of SHP77 cells by chimeric antibody-drug conjugates.
FIG. 6B shows the results of an experiment for assessing the killing of NCI-H82 cells by chimeric antibody-drug conjugates.
FIG. 7 shows the inhibition of xenograft tumor growth in NCI-H82 tumor-bearing mice by chimeric antibody-drug conjugates.
FIG. 8 shows the inhibition of xenograft tumor growth in SHP77 tumor-bearing mice by chimeric antibody-drug conjugates.
FIG. 9 illustrates the molecular structure of DLL3 biepitopic antibodies.
FIG. 10A shows the results of an experiment for assessing the binding of biepitopic antibodies to soluble human DLL3 protein.
FIG. 10B shows the results of an experiment for assessing the binding of biepitopic antibodies to soluble cynomolgus monkey DLL3 protein.
FIG. 10C shows the results of an experiment for assessing the binding of biepitopic antibodies to soluble mouse DLL3 protein.
FIG. 11 shows the results of a killing experiment of biepitopic antibody DLL3 antibody-drug conjugates.
FIG. 12A shows the results of an experiment for assessing the binding of humanized antibodies to soluble human DLL3 protein.
FIG. 12B shows the results of an experiment for assessing the binding of humanized antibodies to soluble cynomolgus monkey DLL3 protein.
FIG. 12C shows the results of an experiment for assessing the binding of humanized antibodies to soluble mouse DLL3 protein.
FIG. 13 shows the results of a killing experiment of humanized DLL3 antibody-drug conjugates.
FIG. 14 shows the inhibition of xenograft tumor growth in NCI-H82 tumor-bearing mice by humanized antibody-drug conjugates.
FIG. 15 shows the inhibition of xenograft tumor growth in SHP77 tumor-bearing mice by humanized antibody-drug conjugates.
FIG. 16 shows the single-crystal diffraction pattern of compound 82.

### Detailed Description of the Invention

### Definitions

Unless defined otherwise, all technical and scientific terms used herein have the same meanings as commonly understood by those of ordinary skill in the art. For specific definitions and terminology in the art, professionals may refer to Current Protocols in Molecular Biology (Ausubel). The abbreviations for amino acid residues refer to standard three-letter and/or one-letter codes used in the art to denote one of the 20 commonly used L-amino acids.

Although the numerical ranges and parameters shown in the broad scope of the present disclosure are approximations, the numerical values shown in the specific examples are recorded as accurately as possible. However, any numerical value inherently contains a certain error resulting from the standard deviation present in its measurement. In addition, all ranges disclosed herein should be understood to encompass any and all subranges contained therein. For example, a recorded range of "1 to 10" should be considered to include any and all subranges between a minimum value of 1 and a maximum value of 10 (inclusive); that is, all subranges starting with a minimum value of 1 or greater, such as 1 to 6.1, and all subranges ending with a maximum value of 10 or less, such as 5.5 to 10. Additionally, any reference described as "incorporated herein" should be understood to be incorporated in its entirety.

The term "subject" or "individual" as used herein refers to a mammal, such as a human, but may also be another animal, such as a wild animal, a livestock animal, or a laboratory animal (e.g., an orangutan, monkey, rat, mouse, rabbit, guinea pig, marmot, or ground squirrel).

The term "antigen" as used herein is a predetermined target to which an antibody can selectively bind. Examples of antigens include, but are not limited to, polypeptides, sugars, nucleic acids, lipids, haptens, or other naturally occurring or synthetic compounds.

Broadly, "antibody" may refer to an immunoglobulin molecule capable of specifically binding to a target via at least one antigen recognition site located in a variable region of the immunoglobulin molecule, thus encompassing an intact antibody/full-length antibody, an antibody single chain, or any antigen-binding fragment (also referred to as an "antigen-binding moiety") of an antibody. When "antibody" and "antigen-binding fragment/antigen-binding moiety" are presented in the same context, the "antibody" may be understood as being the intact body relative to the "antigen-binding fragment/antigen-binding moiety", both collectively corresponding to the broad concept of an antibody.

The term "anti-DLL3 antibody" or "antibody that binds to DLL3" includes antibodies that are capable of binding to DLL3 with a sufficient affinity such that the antibodies can be used as diagnostic agents and/or therapeutic agents when targeting DLL3. In some embodiments, the anti-DLL3 antibody binds to an unrelated, non-DLL3 protein to an extent that is less than about 10% of the extent to which the antibody binds to DLL3, as determined by, for example, fluorescence-activated cell sorting (FACS) analysis or an immunoassay (e.g., radioimmunoassay (RIA)). For an antibody that "specifically binds" to DLL3 or is "specific" for DLL3, in certain embodiments, the antibody that binds to DLL3 has a dissociation constant (KD) of less than or equal to 500 nM, 100 nM, 10 nM, 9 nM, 8 nM, 7 nM, 6 nM, 5 nM, 4 nM, 0.9 nM, 0.8 nM, 0.7 nM, 0.6 nM, 0.5 nM, 0.4 nM, 0.3 nM, 0.2 nM, or 0.1 nM. In some embodiments, the anti-DLL3 antibody binds to a DLL3 protein epitope that is conserved between DLL3 proteins from different species (e.g., between human DLL3 and cynomolgus monkey DLL3).

"Full-length antibody" refers to a protein comprising at least two heavy (H) chains and two light (L) chains interconnected by disulfide bonds. Each of the heavy chains comprises a heavy chain variable region (abbreviated as VH) and a heavy chain constant region. The heavy chain constant region comprises three domains: CH1, CH2, and CH3. Each of the light chains comprises a light chain variable region (abbreviated as VL) and a light chain constant region. The light chain constant region comprises one domain: CL. The VH and VL regions can be further subdivided into multiple hypervariable regions known as complementarity determining regions (CDRs), which are interspersed with more conserved regions known as framework regions (FRs). Each of VH and VL is composed of three CDRs and four FRs, arranged from the amino-terminus to the carboxyl-terminus in the following order: FR1, CDR1, FR2, CDR2, FR3, CDR3, FR4. These variable regions of the heavy and light chains comprise binding domains that interact with antigens. The constant regions of an antibody can mediate the binding of immunoglobulins to host tissues or factors, including various cells of the immune system (e.g., effector cells) and the first component (C1q) of the classical complement system. A full-length antibody may be an antibody of any class, such as IgD, IgE, IgG, IgA, or IgM (or a subclass thereof), but the antibody does not necessarily belong to any particular class. Immunoglobulins can be classified into different classes according to the amino acid sequence of the constant domain of the antibody heavy chain. Generally, there are five main classes of immunoglobulins: IgA, IgD, IgE, IgG, and IgM, and several of these classes can be further divided into subclasses (isotypes), such as IgG1, IgG2, IgG3, IgG4, IgA1, and IgA2. The heavy chain constant domains corresponding to the different immunoglobulin classes are referred to as α, δ, ε, γ, and µ, respectively. The subunit structures and three-dimensional structures of different classes of immunoglobulins are well known. Chimeric or humanized antibodies are also encompassed by the antibodies according to the present disclosure. It is well known to those skilled in the art that the complementarity determining regions (CDRs, generally CDR1, CDR2, and CDR3) are regions in a variable region that have the greatest impact on the affinity and specificity of an antibody. There are various common definitions for the CDR amino acid sequences in a VH or VL, such as the Kabat definition, the IMGT definition, or the Chothia definition. For the variable region amino acid sequences of a given antibody, the CDR amino acid sequences in the VH and VL amino acid sequences can generally be determined according to different definitions. In an embodiment of the present disclosure, the CDR amino acid sequences are defined using Kabat. For the variable region amino acid sequences of a given antibody, the CDR amino acid sequences in the variable region amino acid sequences can be analyzed in a variety of ways.

The term "murine antibody" refers to an antibody obtained by fusing B cells from immunized mice with myeloma cells, selecting mouse hybrid fusion cells capable of both infinite proliferation and antibody secretion, and then screening, preparing, and purifying the obtained antibodies. Murine antibodies generally have immunogenicity and therefore require subsequent humanization.

The term "humanized antibody" refers to an antibody obtained by grafting CDR sequences derived from another mammalian species, such as a mouse species, onto human framework sequences. To preserve the binding affinity, some residues in the backbone (referred to as FR) segments may be modified. The humanized antibodies or fragments thereof according to the present disclosure can be prepared using techniques known to those skilled in the art.

The term "chimeric antibody" refers to an antibody in which the variable region sequences are from one species and the constant region sequences are from another species, e.g., an antibody in which the variable region sequences are from a mouse antibody and the constant region sequences are from a human antibody. The chimeric antibody or the fragment thereof according to the present disclosure can be prepared by using genetic recombination technology. For example, the chimeric antibody can be produced by cloning a recombinant DNA comprising a promoter and sequences encoding the variable regions of the non-human, especially murine, monoclonal antibody according to the present disclosure, as well as sequences encoding human antibody constant regions. The chimeric antibody of the present disclosure encoded by such recombinant genes will be, for example, a murine-human chimera whose specificity is determined by the variable regions derived from murine DNA and whose isotype is determined by the constant regions derived from human DNA.

The term "monoclonal antibody" refers to an antibody obtained from a substantially homogeneous population of antibodies; that is, the antibodies constituting the population are identical except that naturally occurring mutations may be present in a small number of individuals.

The terms "antigen-binding fragment", "antigen-binding moiety", and "antigen-binding region" as used herein are used interchangeably to refer to a portion of an antibody that comprises amino acid residues that interact with an antigen and confer specificity and affinity for the antigen to the binding agent, and particular refer to an antibody fragment such as Fv, Fab, F(ab')₂, or Fab', optionally a fragment comprising a chemical modification that increases the half-life; the chemical modification is, for example, the addition of a poly(alkylene)glycol such as polyethylene glycol ("PEGylation") (a PEGylated fragment referred to as Fv-PEG, scFv-PEG, Fab-PEG, F(ab')₂-PEG, or Fab'-PEG) ("PEG" refers to polyethylene glycol), and the fragment has DLL3-binding activity. Preferably, the antigen-binding fragment will be composed of or comprise a portion of the sequence of the heavy or light chain variable chain of the antibody from which it is derived, and the portion of the sequence is sufficient to retain binding specificity that is the same as that of the antibody from which it is derived and a sufficient affinity; such an antigen-binding fragment will comprise at least 5 amino acids, preferably 10, 15, 25, 50, and 100 contiguous amino acids of the antibody sequence from which it is derived. Examples of antigen-binding fragments include, but are not limited to: (1) a Fab fragment that may be a monovalent fragment having a VL-CL chain and a VH-CH1 chain; (2) a F(ab')₂ fragment that may be a bivalent fragment having two Fab' fragments connected by the disulfide bridges (i.e., a dimer of Fab') in the hinge region; (3) an Fv fragment having the VL and VH domains of a single arm of an antibody; and (4) a VHH fragment consisting of a VH domain.

The term "single-chain antibody (scFv)" refers to a single polypeptide chain formed by linking a VH domain and a VL domain by a peptide linker. (scFv)₂ comprises two VH domains linked by a peptide linker and two VL domains, and the two VL domains are combined with the two VH domains via disulfide bridges.

The terms "Fc fragment", "Fc region", "Fc domain", and "Fc moiety" or similar terms refer to a portion of an antibody heavy chain constant region, including the hinge region and the CH2 and CH3 fragments of the constant region. The Fc region of the anti-DLL3 antibody may be engineered or modified (including effector function-related modifications) to, for example, reduce or eliminate antibody-dependent cellular cytotoxicity (ADCC) and/or complement-dependent cytotoxicity (CDC), which can be achieved by introducing one or more amino acid substitutions/mutations into the Fc region of the antibody.

The term "specific binding" or "specifically bind" as used herein refers to a non-random binding reaction between two molecules, such as the binding of an antibody to an antigenic epitope.

The term "bispecific antibody" refers to an antibody that has the ability to bind to two antigenic epitopes simultaneously. The two antigenic epitopes may be on different antigens or on the same antigen. Bispecific antibodies may have a variety of structural configurations. For example, a bispecific antibody may consist of two Fc fragments and two antigen-binding fragments fused thereto, respectively, and is structurally similar to a natural antibody, with the difference being that the two arms bind to different antigen targets or epitopes; the bispecific antibody is also referred to as an IgG-like bispecific antibody. In contrast, a non-IgG-like bispecific antibody generally uses an scFv fragment or a Fab fragment as a basic moiety, and the scFv fragment or the Fab fragment can form a dimer, a trimer, a tetramer, a pentamer, or even a higher-order oligomer through the use of an appropriately engineered peptide chain linker.

In general, for the preparation of a monoclonal antibody or a functional fragment thereof, particularly a murine monoclonal antibody or a functional fragment thereof, reference can be made to the techniques described particularly in the manual "Antibodies" (Harlow and Lane, Antibodies: A Laboratory Manual, Cold Spring Harbor Laboratory, Cold Spring Harbor NY, pp. 726, 1988) or the techniques for preparation from hybridoma cells described by Kohler and Milstein (Nature, 256:495-497, 1975).

In describing some embodiments involving amino acid or nucleic acid sequence mutations, the present disclosure uses the notation "XaaaY" (e.g., L234A), wherein "aaa" represents the sequence position of an amino acid or base (when a specific reference sequence is present, "aaa" represents the sequential position of a residue in that reference sequence; or a position numbering method commonly used in the art, e.g., the EU numbering system, may also be used), "X" represents the original amino acid or base at "aaa", and "Y" represents the amino acid or base at "aaa" after the change. As an example, when describing the L234A mutation in a human heavy chain constant region, it means that leucine (L) at position 234 is mutated to alanine (A) according to the EU numbering system for the human heavy chain constant region.

The term "isolated" biological components (e.g., nucleic acids, proteins (including antibodies), or organelles) have been substantially isolated or purified from other biological components (i.e., other chromosomal and extra-chromosomal DNA and RNA, proteins, and organelles) in the environment in which the isolated biological components naturally occur (e.g., cells). Nucleic acids and proteins that have been "isolated" include those purified by standard purification methods. The term also includes nucleic acids and proteins prepared by recombinant expression in host cells as well as chemically synthesized nucleic acids.

"Expression vector" is a vector comprising one or more expression control sequences. "Expression control sequence" is a DNA sequence that controls and regulates the transcription and/or translation of another DNA sequence.

The term "antibody-drug conjugate" or "ADC" means that a targeting ligand such as an antibody (e.g., a monoclonal antibody) or an antibody fragment is linked to a bioactive molecule with biological activity by a linker.

The term "linker-drug compound" refers to a partial structure in an "antibody-drug conjugate" consisting of a linker and a bioactive compound.

The term "linker" refers to a chemical structure fragment with one end linked to an antibody and the other end linked to a cytotoxic drug, which is represented by L; in certain embodiments of the present disclosure, the "linker" is formed by linking a "linker-drug compound" to an antibody; in some other embodiments of the present disclosure, a portion of the "linker" is first linked to an antibody, and after the other portion is linked to a bioactive compound, the linker is formed by covalently linking the different portions of the linker. The linker in the present disclosure comprises a covalent linking unit linked to an antibody, an extension unit, a peptide residue, and optionally a self-cleaving fragment.

The linker-drug compound in the present disclosure is linked to the antibody by conventional conjugation methods in the art, including lysine conjugation, conjugation via the reducible disulfide bonds between the light and heavy chains, and site-specific conjugation. In the present disclosure, conjugation is preferably achieved via the reducible disulfide bonds between the light and heavy chains; that is, thiol groups (sulfur atoms of cysteine residues) formed by reducing one or more of the disulfide bond sites between the light and heavy chains (two sites between the heavy chains and two sites between the heavy and light chains) are reacted for linking. In the present disclosure, the linking moiety of the linker L to the antibody is represented by L₁ and is formed by reacting the L₁' group in the linker compound with the antibody, e.g., 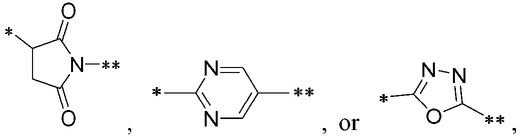 wherein * indicates linking to a sulfhydryl group of the antibody, and ** indicates linking to a spacer.

The term "extension unit" refers to a linking group between the covalent linking unit of the linker L and the polypeptide sequence, which may be any divalent organic group, such as a chemical bond, C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, or a combination of two or more thereof; the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, 5-12 membered heterocyclyl, or the combination of two or more thereof is optionally interrupted by a carbonyl group or an O, S, or N atom; the C₁₋₁₀ alkylene, C₂₋₁₀ alkenylene, C₂₋₁₀ alkynylene, C₃₋₁₀ cycloalkyl, C₆₋₁₂ aryl, 5-12 membered heteroaryl, or 5-12 membered heterocyclyl may be optionally substituted with C₁₋₆ alkyl, C₃₋₆ cycloalkyl, halogen, or C₁₋₆ haloalkyl; preferably, the extension unit comprises a hydrophilic fragment substitution or insertion; preferably, the extension unit is as defined in L₂.

The term "peptide residue" is well known in the art and is selected from a divalent peptide group comprising 2 to 8 optionally substituted protein amino acid or non-protein amino acid, or L-type or D-type amino acid residues, wherein each of the amino acid residues is identical or different and is independently selected from residues of the following amino acids: alanine (Ala), cysteine (Cys), aspartic acid (Asp), glutamic acid (Glu), phenylalanine (Phe), glycine (Gly), histidine (His), isoleucine (Ile), lysine (Lys), leucine (Leu), methionine (Met), asparagine (Asn), proline (Pro), glutamine (Gln), arginine (Arg), serine (Ser), threonine (Thr), valine (Val), tryptophan (Trp), tyrosine (Tyr), citrulline (Cit), norvaline (Nva), norleucine (Nle), and analogs of the amino acids described above. The peptide residues in the present disclosure may comprise substituted or derived amino acid residues, e.g., the amino acid residues with specifically substituted side-chain amino groups defined in CN115279417A, such as:

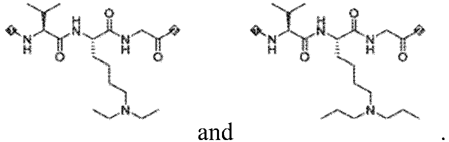

The term "self-cleaving fragment" is well known in the art, such as the p-aminobenzyl alcohol carbonate fragment conventionally used in the art; in a specific embodiment of the present disclosure, the "self-cleaving fragment" comprises a modification with a "hydrophilic fragment".

The term "hydrophilic fragment" is well known in the art, and the hydrophilic fragment may comprise a polyethylene glycol group, such as a polyethylene glycol group terminated with a methoxy group, or may be further linked to other hydrophilic fragments by the polyethylene glycol group. The hydrophilic fragment may also include a polyamino acid fragment, such as polyglycine or polysarcosine; the polyamino acid fragment may be combined with a polyethylene glycol group. The hydrophilic fragment may also be a monosaccharide, a disaccharide, or an oligosaccharide; the monosaccharide, the disaccharide, or the oligosaccharide may be a saccharide of a chain-shaped molecule or a cyclic molecule, and may comprise a glycosylamine, a sugar acid, or a phosphorylated sugar. Preferably, the saccharide group is combined with a polyethylene glycol fragment or a polyamino acid fragment; also preferably, at least two saccharide groups are introduced by polyvalent linking groups, such as linking groups based on aspartic acid, glutamic acid, or lysine. The hydrophilic fragment may also comprise a polycarboxylic acid group, a polysulfonic acid group, a chelating group, or the like; the structure of the polycarboxylic acid group is, for example: 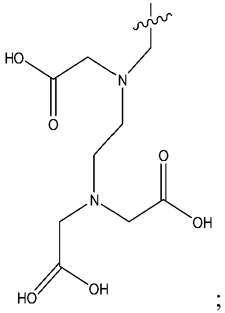 the structure of the polysulfonic acid group is, for example: 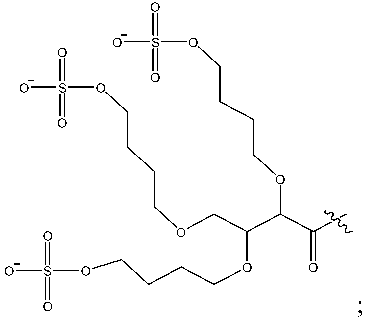 the chelating group may be, for example, a DOTA group or a NOTA group.

The term "pharmaceutical composition" as used herein means a combination of at least one drug and optionally a pharmaceutically acceptable carrier or auxiliary material that are combined to achieve a certain purpose. In certain embodiments, the pharmaceutical composition includes temporally and/or spatially separated combinations, so long as they can act together to achieve the purposes of the present disclosure. For example, the components contained in the pharmaceutical composition (e.g., the antibody, the nucleic acid molecule, the combination of nucleic acid molecules, and/or the conjugate according to the present disclosure) may be administered to an individual as a whole or separately. When separately administered to an individual, the components contained in the pharmaceutical composition may be administered to the individual simultaneously or sequentially. Preferably, the pharmaceutically acceptable carrier is water, an aqueous buffer solution, an isotonic salt solution such as PBS (phosphate-buffered saline), glucose, mannitol, dextrose, lactose, starch, magnesium stearate, cellulose, magnesium carbonate, 0.3% glycerol, hyaluronic acid, ethanol, or a polyalkylene glycol such as polypropylene glycol or a triglyceride. The type of the pharmaceutically acceptable carrier used depends particularly upon whether the composition according to the present disclosure is formulated for oral, nasal, intradermal, subcutaneous, intramuscular, or intravenous administration. The composition according to the present disclosure may comprise a wetting agent, an emulsifying agent, or a buffer substance as an additive. The pharmaceutical composition or pharmaceutical formulation according to the present disclosure may be administered by any suitable route, for example, orally, nasally, intradermally, subcutaneously, intramuscularly, or intravenously.

The term "therapeutically effective amount" or "effective amount" as used herein refers to a dose sufficient to exhibit a benefit for the individual to whom it is administered. The actual amount administered, as well as the rate and time course of administration, will depend on the condition and disease severity of the subject to be treated. The therapeutic prescription (e.g., a decision on the dose) is ultimately the responsibility of the general practitioner and other physicians and depends on their decisions; generally, the disease being treated, the condition of the individual patient, the site of delivery, the method of administration, and other factors known to the physician are taken into account.

The EC₅₀ value primarily refers to the concentration of a drug, an antibody, a toxin, or the like that achieves 50% of the maximum biological effect after a particular exposure time. In pharmacology, in addition to being used to characterize the activation ability of an agonist in an *in vitro* experiment, it can also be used to indicate the plasma concentration required to achieve half the maximum biological effect *in vivo*. In some literature, EC50 is also used to characterize the potency (including agonistic and antagonistic effects) of a compound at the cellular level, and the EC50 value can be determined by methods such as ELISA.

In the general context, the term "fusion protein" as used herein refers to a protein consisting of at least two domains, wherein the domains are not associated with each other in a natural state and are encoded by separate genes, and the genes are linked together and transcribed and translated as a whole to produce a single protein. In the technical context of the present disclosure, a "fusion protein" comprising an antibody or an antigen-binding fragment refers to a product obtained by fusing the antibody or the antigen-binding fragment with another bioactive protein using genetic engineering techniques. Such an antibody fusion protein combines the antigen-binding ability of the antibody and the unique biological properties of the bioactive protein fused with the antibody; for example, fusing with an albumin-binding fragment can extend the *in vivo* half-life of the antibody.

The term "chimeric antigen receptor" or "CAR" as used herein refers to an artificially constructed fusion polypeptide generally comprising an antigen-binding region (e.g., an antibody or an antigen-binding moiety thereof), a transmembrane domain, a costimulatory domain, and an intracellular signaling domain. CARs can utilize the antigen-binding properties of monoclonal antibodies to redirect the specificity and reactivity of T cells and other immune cells to selected targets in a non-MHC-restricted manner. In the present disclosure, the ScFv form of the antibody or bispecific antibody described in the present disclosure can be used for the antigen-binding region.

The terms "identity/homology/similarity" regarding amino acid or nucleic acid sequences are defined as the percentage of identical residues in an amino acid or nucleotide sequence variant that, after sequence alignment and introduction of gaps, achieves the maximum percent identity, if desired. Methods and computer programs used for alignment are well known in the art.

The term "tumor" as used herein refers to a neoplasm or solid lesion formed by abnormal cell growth. The tumor may be benign, premalignant, or malignant.

The term "malignant tumor" as used herein refers to or describes a physiological condition in a mammal, which is typically characterized by unregulated cell growth. Exemplary malignant tumors include: carcinomas, solid tumors, melanomas, sarcomas, hematologic tumors, germ cell tumors, and blastomas. More specific examples of malignant tumors include: multiple myeloma, renal cancer, lung cancer including small cell lung cancer, non-small cell lung cancer, lung adenocarcinoma and lung squamous cell carcinoma, bladder cancer, breast cancer, cervical cancer, colon cancer, gastric cancer including gastrointestinal cancer, prostate cancer, pancreatic cancer, peritoneal cancer, hepatocellular carcinoma, glioblastoma, ovarian cancer, liver cancer, urinary tract cancer, hepatoma, rectal cancer, colorectal cancer, endometrial or uterine cancer, salivary gland cancer, squamous cell carcinoma (e.g., squamous epithelial cell carcinoma), vulval cancer, thyroid cancer, anal cancer, penile cancer, melanoma and B-cell lymphoma, brain cancer, and head and neck cancer, as well as associated metastases.

The term "hematologic tumor" as used herein refers to a tumor caused by uncontrolled growth and proliferation of abnormal cells; in most cases, these abnormal cells originate from the bone marrow, which is also the site where blood cells are produced. Exemplary hematologic tumors include various types of leukemia, multiple myeloma, and malignant lymphoma. More specific examples of hematologic tumors include: acute lymphoblastic leukemia (ALL), chronic lymphocytic leukemia (CLL), acute myeloid leukemia (AML), chronic myeloid leukemia (CML), hairy cell leukemia (HCL), T-cell prolymphocytic leukemia, large granular lymphocytic leukemia, juvenile myelomonocytic leukemia, B-cell prolymphocytic leukemia, Burkitt leukemia and adult T-cell leukemia, non-Hodgkin's lymphoma, B-cell lymphoma, small lymphocytic lymphoma, lymphoplasmacytic lymphoma, primary macroglobulinemia (Waldenström macroglobulinemia), splenic marginal zone lymphoma, plasmacytoma, extranodal marginal zone B-cell lymphoma, MALT lymphoma, nodal marginal zone B-cell lymphoma (NMZL), follicular lymphoma, mantle cell lymphoma, diffuse large B-cell lymphoma, mediastinal (thymic) large B-cell lymphoma, intravascular large B-cell lymphoma, primary effusion lymphoma, Burkitt lymphoma, B-cell chronic lymphocytic lymphoma, classical Hodgkin's lymphoma, nodular lymphocyte-predominant Hodgkin's lymphoma, adult T-cell lymphoma, extranodal NK/T-cell lymphoma, nasal type, enteropathy-associated T-cell lymphoma, hepatosplenic T-cell lymphoma, blastic NK-cell lymphoma, mycosis fungoides, Sezary syndrome, primary cutaneous CD30-positive T-cell lymphoproliferative disorder, primary cutaneous anaplastic large cell lymphoma, lymphomatoid papulosis, angioimmunoblastic T-cell lymphoma, peripheral T-cell lymphoma, not otherwise specified, and anaplastic large cell lymphoma.

The term "solid tumor" as used herein refers to a tangible mass that can be detected through a clinical examination such as X-radiography, CT scan, B-mode ultrasound, or palpation. Clinically diagnosed and treated solid tumors are classified into two categories: malignant and benign. Malignant solid tumors include: pediatric Hodgkin's lymphoma: lymphocyte-predominant type, nodular sclerosis type, mixed cellularity type, lymphocyte depletion type; pediatric non-Hodgkin's lymphoma: precursor lymphoblastic lymphoma, small non-cleaved cell lymphoma (Burkitt/non-Burkitt lymphoma), diffuse large B-cell lymphoma, anaplastic large cell lymphoma, etc.; pediatric renal tumors: nephroblastoma (Wilms tumor), renal clear cell carcinoma, rhabdoid tumor of kidney, renal clear cell sarcoma, renal primitive neuroectodermal tumor, etc.; pediatric neuroblastoma: neuroblastoma, ganglioneuroblastoma, ganglioneuroma; pediatric extragonadal germ cell tumors: mature teratoma, immature teratoma, endodermal sinus tumor (yolk sac tumor), seminoma, dysgerminoma, chorioepithelioma, embryonal carcinoma, etc.; osteosarcoma and chondrosarcoma; pediatric rhabdomyosarcoma: embryonal type, alveolar type, pleomorphic type, etc.; pediatric soft tissue sarcomas: fibrosarcoma, malignant fibrous histiocytoma, liposarcoma, leiomyosarcoma, angiosarcoma, lymphangiosarcoma, malignant schwannoma, alveolar soft tissue sarcoma, epithelioid sarcoma, clear cell sarcoma, malignant melanoma, synovial sarcoma, desmoplastic small round cell tumor, etc.; Ewing's sarcoma family of tumors: Ewing's sarcoma, primitive neuroectodermal tumor; pediatric liver tumors: hepatoblastoma (embryonal type, fetal type, undifferentiated type), hepatocellular carcinoma; retinoblastoma; other tumors: posterior fossa medulloblastoma, nasopharyngeal carcinoma, papillary thyroid carcinoma, thymoma, pulmonary blastoma, pancreatoblastoma, islet cell tumor, ileocecal carcinoid, mesothelioma, etc. Benign solid tumors include: lymphangioma, hemangioma, thyroglossal duct cyst, etc.

### DETAILED DESCRIPTION

### Example 1: Acquisition of Anti-Human DLL3 Monoclonal Antibodies

Healthy BALB/c mice were immunized with recombinant human DLL3 protein (purchased from Acro, Cat. No. DL3-H52H4). After the first immunization, booster immunizations were performed at intervals of 14 days; a total of 4 immunizations were performed. The serum titers of the mice were determined using a flow cytometer, and mice with high serum antibody titers were selected for cell fusion. Three days before fusion, an intensive immunization was performed by tail vein injection of recombinant human DLL3 protein. On the day of fusion, the mice were euthanized, and in a sterile environment, their spleens were collected and used to prepare a single-cell suspension. SP2/0 cells and spleen cells were mixed in a 1:1 ratio and fused by electrofusion. The fused cells were plated in a 96-well cell culture plate, cultured in a 5% CO₂ incubator at 37 °C, and screened using HAT culture medium.

According to the growth of the cells, the hybridoma supernatants were assayed by ELISA at about 10 days. Primary screening was performed using recombinant human DLL3 protein. The positive clones were rescreened with human DLL3 (purchased from Acro, Cat. No. DL3-H525x), cynomolgus monkey DLL3 (purchased from Acro, Cat. No. DL3-C52H3), or mouse DLL3 (purchased from Acro, Cat. No. DL3-M52H9) or human DLL3-B (purchased from Acro, Cat. No. DL3-H52Hy) or human DLL3-C (purchased from Acro, Cat. No. DL3-H52Hz) protein. Five strains of hybridoma cells with high affinities (22B12E5, 25G10A9, 31B6G8, 33B10E8, and 44B8H2) were selected for sequencing. The RNAs of the 5 strains of cells were extracted and reverse-transcribed. After PCR amplification, the amino acid sequences of murine antibody heavy and light chain variable regions were finally obtained. The results are shown below:

| 22B12E5 | |
|---|---|
| Heavy chain CDR1: DYNMY | SEQ ID NO.1 |
| Heavy chain CDR2: YIDPYNGVTGFNQKFKG | SEQ ID NO.2 |
| Heavy chain CDR3: GQGGYYIDYAMDY | SEQ ID NO.3 |
| Heavy chain variable region sequence | SEQ ID NO.7 |
| EIQLQQSGPGLVNPGTSVKVSCKASGYSFTDYNMYWVKQSHGRSLEWIGYIDPYNGV TGFNQKFKGKATLTVDKSSSTAFMHLNSLTSEDSAVYYCSRGQGGYYIDYAMDYWGQGT SVTVSS | |
| Light chain CDR1: RASQDIGDTLA | SEQ ID NO.4 |
| Light chain CDR2: ATSNLDS | SEQ ID NO.5 |
| Light chain CDR3: LQYAISPYT | SEQ ID NO.6 |
| Light chain variable region sequence | SEQ ID NO.8 |
| DIQMTQSPSSLSASLGERVSLTCRASQDIGDTLAWLQQEPDGTIKRLIYATSNLDSGVP KRFSGSRSGSDFSLTISSLESEDFVHYYCLQYAISPYTFGGGTKLEIK | |

| 25G10A9 | |
|---|---|
| Heavy chain CDR1: TYGMS | SEQ ID NO.9 |
| Heavy chain CDR2: TIHSDGGDTYYPDSVKG | SEQ ID NO.10 |
| Heavy chain CDR3: QTGPWLLTY | SEQ ID NO.11 |
| Heavy chain variable region sequence | SEQ ID NO.15 |
| EVQLVESGGGLVQPGGSLKLSCAASGFTFRTYGMSWVRQTPDKRLELVATIHSDGGD TYYPDSVKGRFTISRDSAKNTLYLQMSSLKSEDTAVYYCARQTGPWLLTYWGQGTLVTVS A | |
| Light chain CDR1: KASQAVNTAVA | SEQ ID NO.12 |
| Light chain CDR2: WASTRHT | SEQ ID NO.13 |
| Light chain CDR3: QQHYSTPYT | SEQ ID NO.14 |
| Light chain variable region sequence | SEQ ID NO.116 |
| DIVMTQSHRFMSTSVGDRVSITCKASQAVNTAVAWYQQKPGQSPKLLIYWASTRHTG VPDRFTGSGAGTDYSLTISSVQAEDLALYYCQQHYSTPYTFGGGTKLEIK | |

| 31B6G8 | |
|---|---|
| Heavy chain CDR1: NYGMK | SEQ ID NO.17 |
| Heavy chain CDR2: WINTYTGEPTYADDFKG | SEQ ID NO.18 |
| Heavy chain CDR3: FSDYGGFDY | SEQ ID NO.19 |
| Heavy chain variable region sequence | SEQ ID NO.23 |
| QIQLVQSGPELKKPGETVKISCKASGYTFTNYGMKWVKQAPGKGLKWMGWINTYTG EPTYADDFKGRFAFSLETSASTAYLQINNLKNEDTATYFCARFSDYGGFDYWGQGTTLTVS S | |
| Light chain CDR1: KASQSVSNDVA | SEQ ID NO.20 |
| Light chain CDR2: YASNRYT | SEQ ID NO.21 |
| Light chain CDR3: QQDYSSPFT | SEQ ID NO.22 |
| Light chain variable region sequence | SEQ ID NO.24 |
| NIVMTQTPKFLLVSAGDRVTITCKASQSVSNDVAWYQQKPGQSPKLLIYYASNRYTG VPDRFTGSGYGTDFTFTISTVQAEDLAVYFCQQDYSSPFTFGSGTKLEIK | |

| 33B10E8 | |
|---|---|
| Heavy chain CDR1: DYYMK | SEQ ID NO.25 |
| Heavy chain CDR2: AFNLNNGDTFYNQKFKG | SEQ ID NO.26 |
| Heavy chain CDR3: DVYGYGDY | SEQ ID NO.27 |
| Heavy chain variable region sequence | SEQ ID NO.31 |
| EVQLQQSGPELVKPGASVKMSCKASGYTFTDYYMKWVKQSHGKSLEWIGAFNLNNG DTFYNQKFKGKATLTVDKSSSTAYMQLNSLTSEDSAVYYCARDVYGYGDYWGQGTSVT VSS | |
| Light chain CDR1: RASKSVSTSGYSYMH | SEQ ID NO.28 |
| Light chain CDR2: LASNLDS | SEQ ID NO.29 |
| Light chain CDR3: QHSRELPYT | SEQ ID NO.30 |
| Light chain variable region sequence | SEQ ID NO.32 |
| DIVLTQSPASLAVSLGQRATISCRASKSVSTSGYSYMHWYQQKPGQPPKLLIYLASNL DSGVPARFSGSGSGTDFTLNIHPVEEEDAATYYCQHSRELPYTFGGGTKLEIK | |

| 44B8H2 | |
|---|---|
| Heavy chain CDR1: NYWMH | SEQ ID NO.33 |
| Heavy chain CDR2: AIYPGNSNTAYNQKFKG | SEQ ID NO.34 |
| Heavy chain CDR3: DGYYGFAY | SEQ ID NO.35 |
| Heavy chain variable region sequence | SEQ ID NO.39 |
| EVQLQQSGTVLARPGASVKMSCKASGYSFTNYWMHWMKQRPGQGLEWIGAIYPGN | |
| SNTAYNQKFKGKAKLTAITSASSAYMELSSLTNEDSAVYYCTRDGYYGFAYWGQGTLVT VSA | |
| Light chain CDR1: KASQNVGTNVA | SEQ ID NO.36 |
| Light chain CDR2: SASYRYS | SEQ ID NO.37 |
| Light chain CDR3: QQYKNYPYT | SEQ ID NO.38 |
| Light chain variable region sequence | SEQ ID NO.40 |
| DIVMTQSQKFMSTSVGDRVSVTCKASQNVGTNVAWYQQKSGQFPKALIYSASYRYS GVPDRFTGSGSGTDFTLTISNVQSEDLAEYFCQQYKNYPYTFGGGTKLEIK | |

| | |
|---|---|
| Note: In the antibody sequences described above, the underlined parts are CDR sequences (**determined and annotated according to the Kabat numbering system**). | |

### Example 2: Determination of Affinities of Chimeric Antibodies for Recombinant Human DLL3 Protein by Fortebio

The amino acid sequences of murine antibody heavy and light chain variable regions obtained in Example 1 were used to construct human-mouse chimeric antibodies, with hIgG1/Kappa used as the constant region, and amino acid mutations L234A/L235A/P329A/P331S (numbered according to the EU index of Kabat) introduced into the Fc sequences.

A DLL3 chimeric antibody was immobilized onto a ProA biosensor to a binding response threshold of 0.3 nm. After a 120-second baseline step, the sensor was immersed in a solution of recombinant human DLL3 protein in 0.02% PBST buffer (the protein was serially diluted 3-fold with the starting concentration being 100 nM, and 7 concentrations were set). The association and dissociation times were 120 s and 300 s, respectively. According to the association curves, the affinities of the antibodies for the antigen were calculated by regression analysis using analysis software. All antibodies exhibited affinities comparable to that of rovalpituzumab (purchased from Biointron, B717301). The results are shown in Table 1.

**Table 1. The determination of the affinities of chimeric antibodies for recombinant human DLL3 protein by Fortebio**

| Sample | ka (1/Ms) | kd (1/s) | KD (M) |
|---|---|---|---|
| 22B12E5 | 4.32E+05 | 8.01E-04 | 1.85E-09 |
| 25G10A9 | 1.75E+05 | 3.34E-04 | 1.91E-09 |
| 31B6G8 | 4.95E+05 | 4.10E-04 | 8.29E-10 |
| 33B10E8 | 4.03E+05 | 2.08E-04 | 5.16E-10 |
| 44B8H2 | 1.65E+05 | 6.79E-04 | 4.11E-09 |
| Rovalpituzumab | 4.12E+05 | 3.34E-04 | 8.10E-10 |

### Example 3: Cross-Reactivity of Antibodies in Binding to Human, Monkey, and Mouse DLL3 Proteins

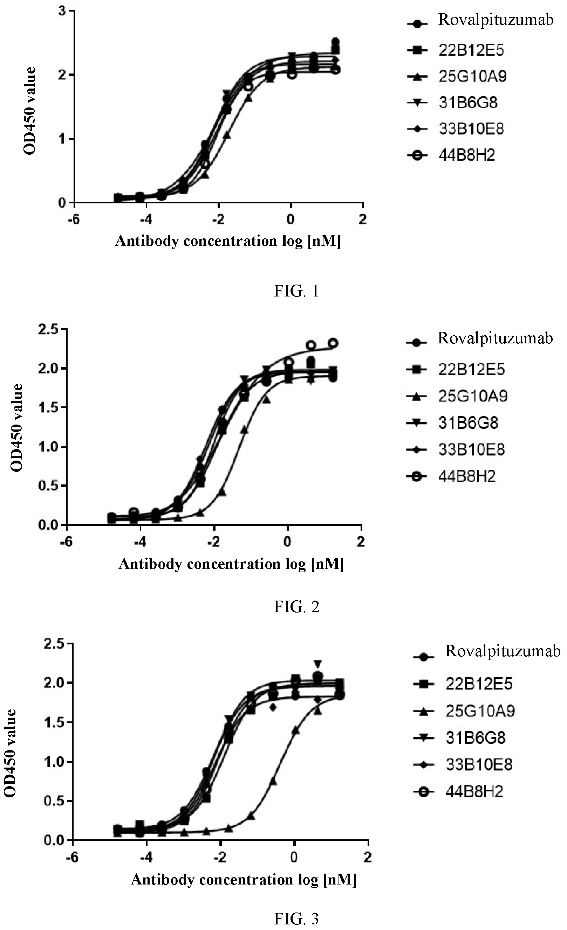

To determine the species cross-reactivity of antibodies, the affinities of the antibodies for recombinant human DLL3 protein, cynomolgus monkey DLL3, and mouse DLL3 protein were determined by ELISA. Recombinant human, cynomolgus monkey, and mouse DLL3 proteins were diluted to a final concentration of 0.5 µg/mL and added to 96-well microplates, and the plates were incubated overnight at 4 °C. The next day, the supernatants were discarded. After blocking, the antibodies (serially diluted 4-fold, 0-17.1 nM) were added. After incubation with a secondary antibody, color development was performed with a TMB chromogenic solution for 10 min. After 2 M HCl was added to stop the reactions, the absorbance was measured at 450 nm with a microplate reader. The affinities obtained after curves were fit to the results using GraphPad are shown in FIGs. 1, 2, and 3 and Table 2. The results show that the affinities of the 5 chimeric antibodies for human and cynomolgus monkey DLL3 proteins were comparable to the binding ability of rovalpituzumab. 25G10A9 bound weakly to mouse DLL3 protein, and the abilities of the remaining chimeric antibodies to bind to mouse DLL3 protein were comparable to that of rovalpituzumab.

**Table 2. The abilities of chimeric antibodies to bind to human, monkey, and mouse DLL3 proteins**

| Antibody name | Human EC50 value (nM) | Cynomolgus monkey EC50 value (nM) | Mouse EC50 value (nM) |
|---|---|---|---|
| Rovalpituzumab | 0.007758 | 0.007513 | 0.006276 |
| 22B12E5 | 0.009154 | 0.01233 | 0.01269 |
| 25G10A9 | 0.01907 | 0.04857 | 0.4199 |
| 31B6G8 | 0.007426 | 0.009731 | 0.006999 |
| 33B10E8 | 0.007555 | 0.00597 | 0.007059 |
| 44B8H2 | 0.009004 | 0.01602 | 0.008797 |

### Example 4: Cross-Reactivity of Chimeric Antibodies with Human DLL1 Protein and Human DLL4 Protein

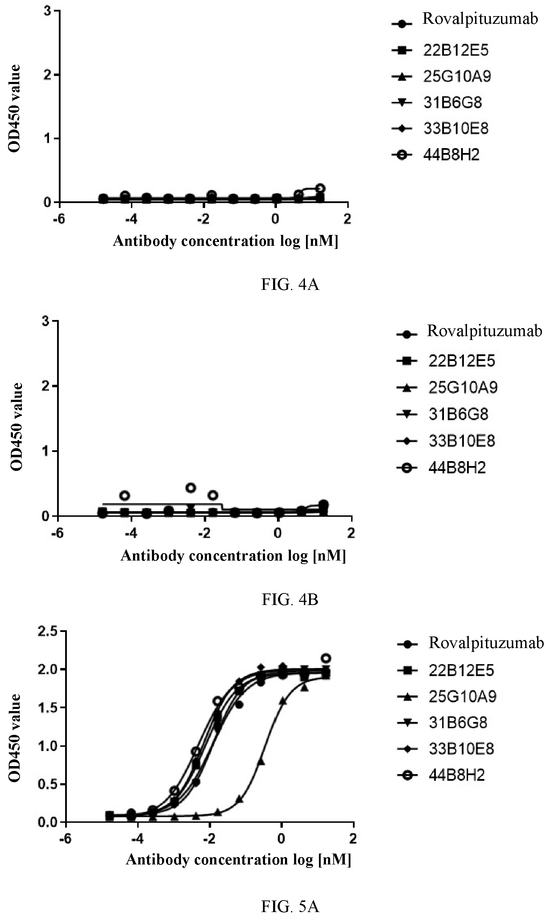

The abilities of the obtained chimeric antibodies to bind to human DLL1 protein (purchased from Acro, Cat. No. DL1-H52H8) and human DLL4 protein (purchased from Acro, Cat. No. DL4-H5227) were assessed by ELISA. Human DLL1 protein and human DLL4 protein were diluted to a final concentration of 0.5 µg/mL and added to 96-well microplates. After blocking, the antibodies (serially diluted 4-fold, 0-17.1 nM) were added, and the plates were incubated at room temperature for 1 h. The supernatants were discarded, and the plates were washed 5 times with PBST (1‰ Tween 20). An HRP-labeled goat anti-human secondary antibody was added, and the plates were incubated at room temperature for 1 h. After the plates were washed, color development was performed with a TMB chromogenic solution for 10 min. After 2 M HCl was added to stop the reactions, the absorbance was measured at 450 nm with a microplate reader. The results are shown in FIGs. 4A-4B. None of the 5 chimeric antibodies significantly cross-reacted with human DLL1 protein or human DLL4 protein.

### Example 5: Identification of Epitopes Recognized by Chimeric Antibodies

### 5.1. Identification of epitopes recognized by chimeric antibodies by ELISA

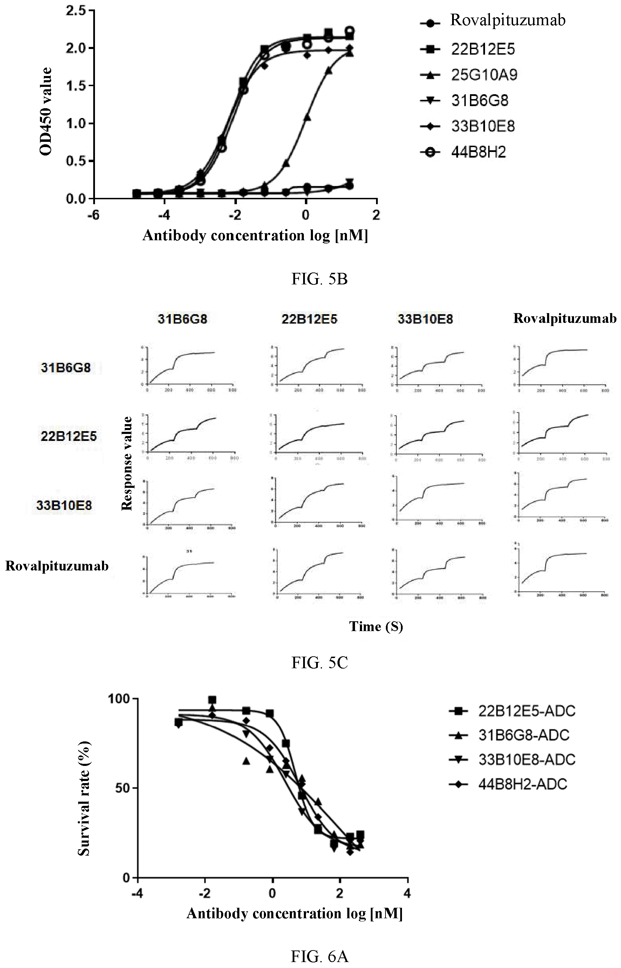

The abilities of chimeric antibodies to bind to different truncated DLL3 proteins were assessed by ELISA. Human DLL3-B protein and human DLL3-C protein were diluted to 0.5 µg/mL and used to coat microplates. After blocking, the antibodies (serially diluted 4-fold, 0-17.1 nM) were added. After 1 h of incubation with a secondary antibody, the plates were washed, color development was performed, and readings were taken. The affinities obtained after curves were fit to the results using GraphPad are shown in FIGs. 5A-5B and Table 3. The results show that chimeric antibody 31B6G8 and rovalpituzumab could bind to DLL3-B but did not bind to DLL3-C protein, indicating that 31B6G8 and rovalpituzumab may recognize the same epitope. Chimeric antibodies 22B12E5, 33B10E8, 44B8H2, and 25G10A9 bound to both DLL3-B and DLL3-C proteins, indicating that the epitopes recognized by these antibodies are different from that recognized by rovalpituzumab.

**Table 3. EC₅₀ values for the affinities of chimeric antibodies for DLL3-B and DLL3-C proteins as determined by ELISA**

| Antibody name | Human DLL3-B protein binding EC₅₀ value (nM) | Human DLL3-C protein binding EC₅₀ value (nM) |
|---|---|---|
| Rovalpituzumab | 0.01221 | ND |
| 22B12E5 | 0.007697 | 0.00745 |
| 25G10A9 | 0.3483 | 0.9799 |
| 31B6G8 | 0.01267 | ND |
| 33B10E8 | 0.0069 | 0.006246 |
| 44B8H2 | 0.004884 | 0.009031 |

### 5.2. Identification of epitopes recognized by antibodies by Octet epitope pairing

To further classify the epitopes recognized by the antibodies, we classified the antigenic epitopes recognized by chimeric antibodies 22B12E5, 33B10E8, and 31B6G8 and rovalpituzumab by Octet epitope pairing. Human DLL3 protein was loaded onto a HIS1K sensor at 5 µg/mL and then exposed to a first antibody and a second antibody. Whether the two antibodies recognize the same epitope was determined based on the binding signal of the second antibody. Data were processed using ForteBio's data analysis software 7.0. The results are shown in FIG. 5C. The epitopes recognized by 22B12E5 and 33B10E8 are completely different from that recognized by rovalpituzumab, and 31B6G8 and rovalpituzumab recognize the same epitope. The epitopes recognized by 22B12E5, 33B10E8, and 31B6G8 are different from each other.

### Example 6: Binding of Chimeric Antibodies to NCI-H82 Cells

The abilities of chimeric antibodies to bind to cell surface DLL3 were assessed using the human small cell lung cancer cell strain NCI-H82. Cells (density: 2 × 10⁶ cells/mL) were seeded in a 96-well plate at 100 µL/well. The antibodies (137 nM) were added, and the plate was incubated at 4 °C for 1 h. A goat anti-human secondary antibody was added, and labeling was performed at 4 °C for 45 min in a dark place. The cells were collected and washed, and then the fluorescence intensity of the cells was measured with a flow cytometer. The experimental results are shown in Table 4. The results show that: the chimeric antibodies could bind to the DLL3 protein expressed on NCI-H82 cells, and their readings were all higher than that of rovalpituzumab.

**Table 4. The binding of chimeric antibodies to NCI-H82 cell surface DLL3**

| Antibody name | Reading (137 nM) |
|---|---|
| Rovalpituzumab | 27,548 |
| 22B12E5 | 45,543 |
| 25G10A9 | 40,812 |
| 31B6G8 | 33,179 |
| 33B10E8 | 55,086 |
| 44B8H2 | 50,692 |

### Example 7: Assessment of Endocytic Activity of Anti-DLL3 Antibodies

The endocytosis rates of DLL3 chimeric antibodies were determined by FACS, and the abilities of the chimeric antibodies to induce the endocytosis of cell surface DLL3 were assessed using the cell strain SHP77. Cells (density: 2 × 10⁶ cells/mL) were seeded in a 96-well plate at 100 µL/well. The antibodies (final concentration: 34.2 nM) were added, and the plate was incubated at 4 °C for 1 h. The cells were washed with FACS buffer and then incubated at 4 °C and 37 °C for 2 h. A goat anti-human secondary antibody was added, and labeling was performed at 4 °C for 45 min in a dark place. The cells were collected and washed, and then the fluorescence intensity of the cells was measured with a flow cytometer. Internalization ratios were calculated: [(reading at 4 °C - negative control reading) - (reading at 37 °C - negative control reading)] / (reading at 4 °C - negative control reading) × 100%.

The calculated 2-h endocytosis ratios of the antibodies are shown in Table 5. The results show that the chimeric antibodies exhibited relatively good endocytic activity.

**Table 5. The 2-h endocytosis ratios of chimeric antibodies**

| Antibody name | Endocytosis ratio |
|---|---|
| Rovalpituzumab | 54.20% |
| 22B12E5 | 52.36% |
| 25G10A9 | 33.13% |
| 31B6G8 | 60.63% |
| 33B10E8 | 71.74% |
| 44B8H2 | 63.80% |

### Example 8: Binding of DLL3 Antibody-Drug Conjugates to Human DLL3-Expressing Cells

JSSW-001 (Shanghai Haoyuan Chemexpress Co., Ltd.) was conjugated to chimeric antibodies 22B12E5, 31B6G8, 33B10E8, and 44B8H2 and rovalpituzumab by cysteine conjugation to obtain 22B12E5-ADC, 31B6G8-ADC, 33B10E8-ADC (also referred to as Cab33-ADC in the examples below), 44B8H2-ADC, and Rova-ADC (positive control), respectively. The method steps included: TCEP (J&K Scientific) was added to the DLL3 chimeric antibodies and rovalpituzumab in an antibody-to-TCEP molar ratio of 1:10, and the mixtures were incubated at 37 °C for 2 h. JSSW-001 (Shanghai Haoyuan Chemexpress Co., Ltd.) was added to the above solutions in an antibody-to-toxin molar ratio of 1:12, and the mixtures were left to react at 25 °C for 30 min. Then, N-ethylmaleimide (J&K Scientific) was added in a molar ratio of 1:14. The mixtures were left to react at room temperature for 20 min, and the conjugation reactions were stopped. The above antibody-drug conjugates were purified by affinity chromatography using an AKTA purifier, and the obtained antibody-drug conjugate (ADC) molecules were buffer-exchanged into PBS. Analyses including SEC\LC-MS showed that Rova-ADC, 22B12E5-ADC, 31B6G8-ADC, 33B10E8-ADC, and 44B8H2-ADC all contained normally conjugated JSSW-001 molecules, and that the obtained ADCs had a free small molecule content of less than 0.0015%, an SEC purity of greater than 95%, and a DAR of about 8. The obtained ADCs met the requirements for DAR, free small molecule content, etc.

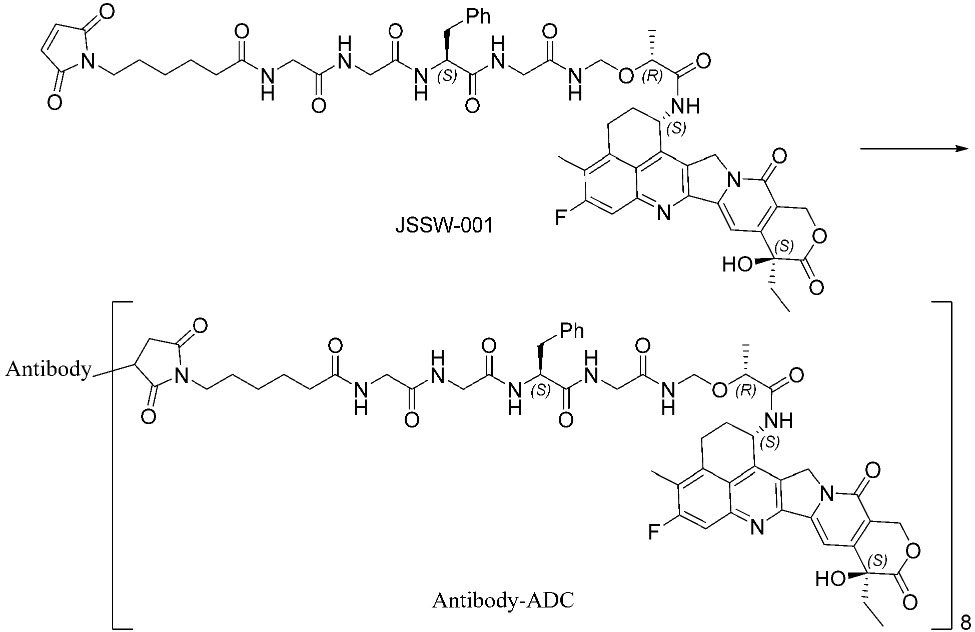

The abilities of the antibody-drug conjugates to bind to cell surface DLL3 were assessed using the cancer cell strain NCI-H82. Cells (density: 2 × 10⁶ cells/mL) were seeded in a 96-well plate at 100 µL/well. The antibodies (137 nM) were added, and the plate was incubated at 4 °C for 1 h. A goat anti-human secondary antibody was added, and labeling was performed at 4 °C for 45 min in a dark place. The cells were collected and washed, and then the fluorescence intensity of the cells was measured with a flow cytometer. The results are shown in Table 6. The abilities of the DLL3 antibody-drug conjugates to bind to NCI-H82 cell surface DLL3 were substantially consistent with the abilities of the corresponding chimeric antibodies to bind to NCI-H82 cell surface DLL3, and all, except 31B6G8-ADC, were superior to Rova-ADC.

**Table 6. The binding of DLL3 antibody-drug conjugates to NCI-H82 cells**

| DLL3 antibody-drug conjugate name | Reading (137 nM) |
|---|---|
| Rova-ADC | 40,535 |
| 22B12E5-ADC | 51,565 |
| 31B6G8-ADC | 28,797 |
| 33B10E8-ADC | 57,123 |
| 44B8H2-ADC | 43,563 |

### Example 9: Evaluation of In Vitro Cytotoxicity of ADC Samples

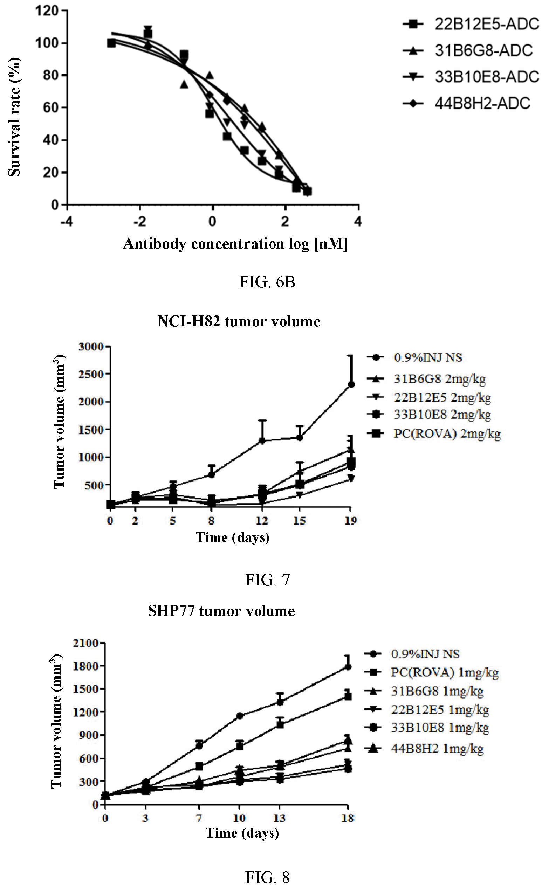

Small cell lung cancer SHP77 and NCI-H82 cells, which highly express DLL3, were selected as the cell strains for assessing *in vitro* activity, and the dose-response relationships of different antibody-drug conjugates in cell killing were observed. Cells (density: 2 × 10⁴ cells/mL) were seeded in 96-well plates at 50 µL/well. The antibodies (serially diluted 3-fold, final concentrations: 0-400 nM) were added. After six days of co-incubation, Cell Titer Glo^{®} Luminescent Cell Viability Assay Reagent was added, and the plates were shaken at room temperature for 10 min for thorough mixing. Data were read with a microplate reader and then processed with the survival rate of the drug-free whole cell group being 100% (survival rate = (test group/blank control group) × 100%), and IC₅₀ was calculated. The results are shown in FIGs. 6A-6B and Table 7. The results show that the ADCs disclosed in the present disclosure all exhibited significant *in vitro* cell killing effects on cells highly expressing DLL3, and that 33B10E8 and 22B12E5 exhibited relatively the strongest *in vitro* cytotoxic activity.

**Table 7. A killing experiment of DLL3 antibody-drug conjugates**

| Antibody-drug conjugate name | IC₅₀ value for SHP77 cells (nM) | IC₅₀ value for NCI-H82 cells (nM) |
|---|---|---|
| 22B12E5-ADC | 4.788 | 1.053 |
| 31B6G8-ADC | 105.7 | 74.43 |
| 33B10E8-ADC | 2.34 | 2.903 |
| 44B8H2-ADC | 6.578 | 39.04 |

### Example 10: Biological Activity of DLL3 Antibody-Drug Conjugates in Animals

### 10.1. In vivo anti-tumor activity of ADC drugs in NCI-H82 model

The monotherapy efficacy of chimeric antibody ADC molecules 31B6G8-ADC (abbreviated as 31B6G8 in FIG. 7 and Table 8), 22B12E5-ADC (abbreviated as 22B12E5 in FIG. 7 and Table 8), 33B10E8-ADC (abbreviated as 33B10E8 in FIG. 7 and Table 8), and Rova-ADC (abbreviated as PC(ROVA) in FIG. 7 and Table 8) was assessed in a tumor xenograft model. 1 × 10⁶ NCI-H82 human small cell lung cancer cells were inoculated into Nu/nu mice. When tumor volume reached 100-150 mm³, 31B6G8-ADC, 22B12E5-ADC, 33B10E8-ADC, and Rova-ADC, as well as a 0.9% sodium chloride injectable solution control (0.9% INJ NS), were intravenously injected once at 2 mg/kg. Tumor volume was calculated at the end of the experiment. Calculation formula: tumor volume (mm³) = 1/2 × (a × b²) (where a represents long diameter, and b represents short diameter). Compared to the solvent group, the groups could all inhibit tumor growth, and 22B12E5-ADC (P < 0.05) could significantly inhibit the volume of xenograft tumors. See FIG. 7 and Table 8 for details.

**Table 8. The tumor parameters of the groups on day 19 after administration of the DLL3-ADCs (chimeric antibodies)**

| Group | DAR | Dose (mg/kg) | Route of administration | Tumor volume (mm³) | | TGI% |
|---|---|---|---|---|---|---|
| | | | | D0 | D19 | |
| 0.9%INJ NS | -- | -- | i.v | 140.8± 56.9 | 2,315.4±1,026.9 | -- |
| 31B6G8 | 6.6 | 2 | | 144.3±37.0 | 1,143.8±497.6 | 54.0 |
| 22B12E5 | 7.6 | | | 144.2± 31.4 | 595.6± 172.1* | 79.2 |
| 33B10E8 | 6.4 | | | 146.9± 33.7 | 838.5± 650.3 | 68.2 |
| PC(ROVA) | 7.9 | | | 148.8+ 35.2 | 925.6+ 738.0 | 64.3 |

### 10.2. In vivo anti-tumor activity of ADC drugs in SHP77 model

The monotherapy efficacy of chimeric antibody ADC molecules 31B6G8-ADC (abbreviated as 31B6G8 in FIG. 8 and Table 9), 22B12ES5-ADC (abbreviated as 22B12E5 in FIG. 8 and Table 9), 33B10E8-ADC (abbreviated as 33B10E8 in FIG. 8 and Table 9), 44B8H2-ADC (abbreviated as 44B8H2 in FIG. 8 and Table 9), and Rova-ADC (abbreviated as PC(ROVA) in FIG. 8 and Table 9) was assessed in a tumor xenograft model. 1 × 10⁷ SHP77 cells were inoculated into NCG mice. When tumor volume reached 100-150 mm³, 31B6G8-ADC, 22B12E5-ADC, 33B10E8-ADC, 44B8H2-ADC, and Rova-ADC, as well as a 0.9% sodium chloride injectable solution control, were intravenously injected once at 1 mg/kg. The *in vivo* anti-tumor effects of the DLL3-ADCs were investigated. At the end of the experiment, compared to the solvent group, the 31B6G8-ADC group resulted in 63.8% tumor inhibition and a tumor volume of 726.9 ± 237.8, the 22B12E5-ADC group resulted in 76.4% tumor inhibition and a tumor volume of 519.3 ± 156.8, the 33B10E8-ADC group resulted in 79.5% tumor inhibition and a tumor volume of 466.7 ± 115.9, the 44B8H2-ADC group resulted in 57.3% tumor inhibition and a tumor volume of 834.7 ± 150.5, and the Rova-ADC (positive control) group resulted in 23.0% tumor inhibition and a tumor volume of 1408.7 ± 183.6. The groups (P < 0.05), except Rova-ADC (positive control), could significantly inhibit the volume of xenograft tumors. See FIG. 8 and Table 9 for details.

**Table 9. The tumor parameters of the groups on day 19 after administration of the DLL3-ADCs (chimeric antibodies)**

| Group | DAR | Dose (mg/kg) | Route of administration | Tumor volume (mm³) | | TGI% |
|---|---|---|---|---|---|---|
| | | | | D0 | D18 | |
| 0.9%INJ NS | -- | -- | | 125.3± 19.7 | 1,793.0± 346.2 | -- |
| PC(ROVA) | 7.9 | 1mpk(D0); | | 124.2± 24.0 | 1,408.7± 183.6 | 23.0 |
| 31B6G8 | 6.6 | | i.v | 123.9± 22.7 | 726.9± 237.8*** | 63.8 |
| 22B12E5 | 7.6 | | | 125.1± 21.1 | 519.3± 156.8*** | 76.4 |
| 33B10E8 | 6.4 | | | 125.0± 19.5 | 466.7± 115.9*** | 79.5 |
| 44B8H2 | 7.2 | | | 123.1± 28.1 | 834.7± 150.5*** | 57.3 |

### Example 11: Acquisition of DLL3 Biepitopic Antibodies

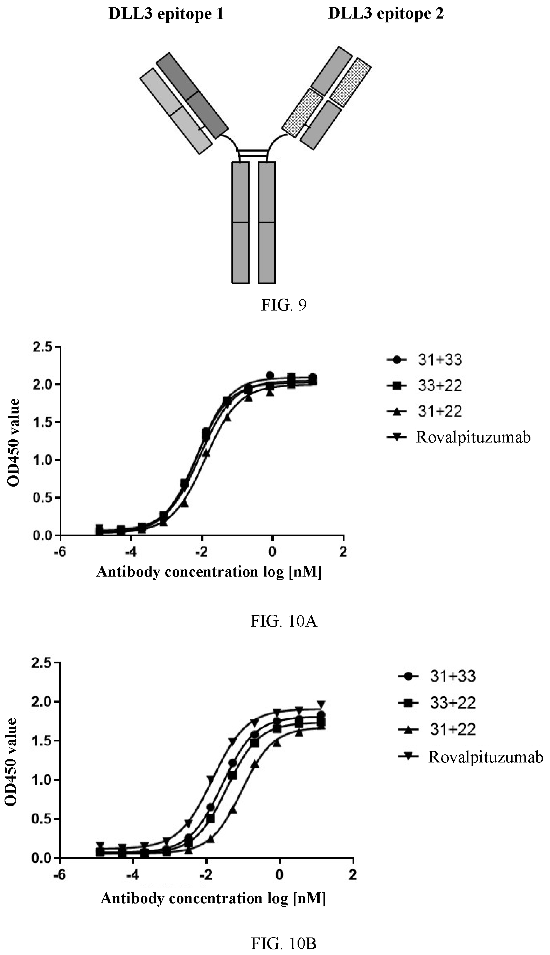

According to epitope analysis, 22B12E5, 31B6G8, and 33B10E8 bind to different epitopes. Their variable regions were used to construct biepitopic molecules in a symmetric 1:1 structural format. For the antibody heavy chain moieties, knobs-into-holes technology was adopted to produce two heavy chains with different sequences, and for the light chain moieties, Crossmab technology was used to produce two different light chains. hIgG1/Kappa was used as the constant region, and amino acid mutations L234A/L235A/P329A/P331S (numbered according to the EU index of Kabat) were introduced into the Fc sequences. Finally, three biepitopic antibody molecules were produced through pairwise combinations: 33B10E8+22B12E5 (33+22), 31B6G8+22B12E5 (31+22), and 33B10E8+31B6G8 (33+31). Their antibody structure is shown in FIG. 9, and their sequences are shown in Table 10.

**Table 10. The sequences of biparatopic antibodies**

| | |
|---|---|
| 33+31-HC1 (heavy chain 1 amino acid sequence, SEQ ID NO.41) | EVQLQQSGPELVKPGASVKMSCKASGYTFTDYYMKWVKQSHGKSLEWI GAFNLNNGDTFYNQKFKGKATLTVDKSSSTAYMQLNSLTSEDSAVYYCA RDVYGYGDYWGQGTSVTVSS(SEQ ID NO.31) ASTKGPSVFPLAPSSKSTSGGTAALGCLVEDYFPEPVTVSWNSGALTSGVH TFPAVLQSSGLYSLSSVVTVPSSSLGTQTYICNVNHKPSNTKVDEKVEPKS CDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKE YKCKVSNKALAASIEKTISKAKGQPREPQVCTLPPSRDELTKNQVSLSCAV KGFYPSDIAVEWESNGQPENNYKTTPPVLDSDGSFFLVSKLTVDKSRWQQ GNVFSCSVMHEALHNHYTQKSLSLSPGK(SEQ ID NO.42 ) |
| 33+31-HC2 (heavy chain 2 amino acid sequence, SEQ ID NO.43) | QIQLVQSGPELKKPGETVKISCKASGYTFTNYGMKWVKQAPGKGLKWM GWINTYTGEPTYADDFKGRFAFSLETSASTAYLQINNLKNEDTATYFCARF SDYGGFDYWGQGTTLTVSS(SEQ ID NO.23) ASVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC(SEQ ID NO.44) DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALAASIEKTISKAKGQPREPQVYTLPPCRDELTKNQVSLWCL VKGFYPSDIAVEWESNGQPENNYKTTPPVLDSDGSFFLYSKLTVDKSRWQ QGNVFSCSVMHEALHNHYTQKSLSLSPGK(SEQ ID NO.45) |
| 33+31-LC1 (light chain 1 amino acid sequence, SEQ ID NO.46) | DIVLTQSPASLAVSLGQRATISCRASKSVSTSGYSYMHWYQQKPGQPPKLL IYLASNLDSGVPARFSGSGSGTDFTLNIHPVEEEDAATYYCQHSRELPYTF GGGTKLEIK(SEQ ID NO.32) RTVAAPSVFIFPPSDRKLKSGTASVVCLLNNFYPREAKVQWKVDNALQSG NSQESVTEQDSKDSTYSLSSTLTLSKADYEKHKVYACEVTHQGLSSPVTK SFNRGEC(SEQ ID NO.47) |
| 33+31-LC2 (light chain 2 amino acid sequence, SEQ ID NO.48) | NIVMTQTPKFLLVSAGDRVTITCKASQSVSNDVAWYQQKPGQSPKLLIYY ASNRYTGVPDRFTGSGYGTDFTFTISTVQAEDLAVYFCQQDYSSPFTFGSG TKLEIK(SEQ ID NO.24) SSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEP KSC(SEQ ID NO.49) |
| 33+22-HC1 (heavy chain 1 amino acid sequence, SEQ ID NO.41) | EVQLQQSGPELVKPGASVKMSCKASGYTFTDYYMKWVKQSHGKSLEWI GAFNLNNGDTFYNQKFKGKATLTVDKSSSTAYMQLNSLTSEDSAVYYCA RDVYGYGDYWGQGTSVTVSS(SEQ ID NO.31) ASTKGPSVFPLAPSSKSTSGGTAALGCLVEDYFPEPVTVSWNSGALTSGVH TFPAVLQSSGLYSLSSVVTVPSSSLGTQTYICNVNHKPSNTKVDEKVEPKS CDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKE YKCKVSNKALAASIEKTISKAKGQPREPQVCTLPPSRDELTKNQVSLSCAV KGFYPSDIAVEWESNGQPENNYKTTPPVLDSDGSFFLVSKLTVDKSRWQQ GNVFSCSVMHEALHNHYTQKSLSLSPGK(SEQ ID NO.42) |
| 33+22-HC2 (heavy chain 2 amino acid sequence, SEQ ID NO.50) | EIQLQQSGPGLVNPGTSVKVSCKASGYSFTDYNMYWVKQSHGRSLEWIG YIDPYNGVTGFNQKFKGKATLTVDKSSSTAFMHLNSLTSEDSAVYYCSRG QGGYYIDYAMDYWGQGTSVTVSS(SEQ ID NO.7) ASVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC(SEQ ID NO.44) DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALAASIEKTISKAKGQPREPQVYTLPPCRDELTKNQVSLWCL VKGFYPSDIAVEWESNGQPENNYKTTPPVLDSDGSFFLYSKLTVDKSRWQ QGNVFSCSVMHEALHNHYTQKSLSLSPGK(SEQ ID NO.45) |
| 33+22-LC1 (light chain 1 amino acid sequence, SEQ ID NO.46) | DIVLTQSPASLAVSLGQRATISCRASKSVSTSGYSYMHWYQQKPGQPPKLL IYLASNLDSGVPARFSGSGSGTDFTLNIHPVEEEDAATYYCQHSRELPYTF GGGTKLEIK(SEQ ID NO.32) RTVAAPSVFIFPPSDRKLKSGTASVVCLLNNFYPREAKVQWKVDNALQSG NSQESVTEQDSKDSTYSLSSTLTLSKADYEKHKVYACEVTHQGLSSPVTK SFNRGEC(SEQ ID NO.47) |
| 33+22-LC2 (light chain 2 amino acid sequence, SEQ ID NO.51) | DIQMTQSPSSLSASLGERVSLTCRASQDIGDTLAWLQQEPDGTIKRLIYATS NLDSGVPKRFSGSRSGSDFSLTISSLESEDFVHYYCLQYAISPYTFGGGTKL EIK(SEQ ID NO.8) SSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEP KSC(SEQ ID NO.49) |
| 31+22-HC1 (heavy chain 1 amino acid sequence, SEQ ID NO.52) | QIQLVQSGPELKKPGETVKISCKASGYTFTNYGMKWVKQAPGKGLKWM GWINTYTGEPTYADDFKGRFAFSLETSASTAYLQINNLKNEDTATYFCARF SDYGGFDYWGQGTTLTVSS(SEQ ID NO.23) ASTKGPSVFPLAPSSKSTSGGTAALGCLVEDYFPEPVTVSWNSGALTSGVH TFPAVLQSSGLYSLSSVVTVPSSSLGTQTYICNVNHKPSNTKVDEKVEPKS CDKTHTCPPCPAPEAAGGPSVFLFPPKPKDTLMISRTPEVTCVVVDVSHED PEVKFNWYVDGVEVHNAKTKPREEQYNSTYRVVSVLTVLHQDWLNGKE YKCKVSNKALAASIEKTISKAKGQPREPQVCTLPPSRDELTKNQVSLSCAV KGFYPSDIAVEWESNGQPENNYKTTPPVLDSDGSFFLVSKLTVDKSRWQQ GNVFSCSVMHEALHNHYTQKSLSLSPGK(SEQ ID NO.42) |
| 31+22-HC2 (heavy chain 2 amino acid sequence, SEQ ID NO.50) | EIQLQQSGPGLVNPGTSVKVSCKASGYSFTDYNMYWVKQSHGRSLEWIG YIDPYNGVTGFNQKFKGKATLTVDKSSSTAFMHLNSLTSEDSAVYYCSRG QGGYYIDYAMDYWGQGTSVTVSS(SEQ ID NO.7) ASVAAPSVFIFPPSDEQLKSGTASVVCLLNNFYPREAKVQWKVDNALQSG NSQESVTEQDSKDSTYSLSSTLTLSKADYEKHKVYACEVTHQGLSSPVTK SFNRGEC(SEQ ID NO.44) DKTHTCPPCPAPEAAGGPSVFLFPPKPKDTLMISRTPEVTCVVVDVSHEDP EVKFNWYVDGVEVHNAKTKPREEQYNSTYRVVSVLTVLHQDWLNGKE YKCKVSNKALAASIEKTISKAKGQPREPQVYTLPPCRDELTKNQVSLWCL VKGFYPSDIAVEWESNGQPENNYKTTPPVLDSDGSFFLYSKLTVDKSRWQ QGNVFSCSVMHEALHNHYTQKSLSLSPGK(SEQ ID NO.45) |
| 31+22-LC1 (light chain 1 amino acid sequence, SEQ ID NO.53) | NIVMTQTPKFLLVSAGDRVTITCKASQSVSNDVAWYQQKPGQSPKLLIYY ASNRYTGVPDRFTGSGYGTDFTFTISTVQAEDLAVYFCQQDYSSPFTFGSG TKLEIK(SEQ ID NO.24) RTVAAPSVFIFPPSDRKLKSGTASVVCLLNNFYPREAKVQWKVDNALQSG NSQESVTEQDSKDSTYSLSSTLTLSKADYEKHKVYACEVTHQGLSSPVTK SFNRGEC(SEQ ID NO.47) |
| 31+22-LC2 (light chain 2 amino acid sequence, SEQ ID NO.51) | DIQMTQSPSSLSASLGERVSLTCRASQDIGDTLAWLQQEPDGTIKRLIYATS NLDSGVPKRFSGSRSGSDFSLTISSLESEDFVHYYCLQYAISPYTFGGGTKL EIK(SEQ ID NO.8) SSASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKKVEP KSC (SEQ ID NO.49) |

### Example 12: Determination of Affinities of DLL3 Biepitopic Antibodies

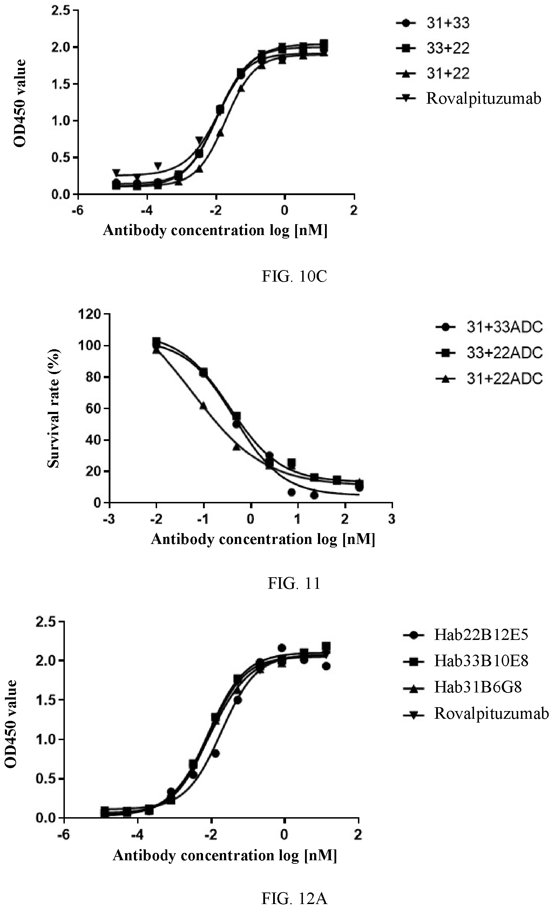

First, the affinities of biepitopic antibodies for recombinant human, cynomolgus monkey, and mouse DLL3 proteins were determined by ELISA. The DLL3 proteins were diluted to 5 µg/mL and used to coat microplates. After blocking, the antibodies (serially diluted 4-fold, 0-13.4 nM) were added. After incubation with a secondary antibody, color development was performed, and readings were taken. The affinities obtained after curves were fit to the results using GraphPad are shown in FIGs. 10A-10C and Table 11.

**Table 11. EC₅₀ values for the affinities of biparatopic antibodies as determined by ELISA**

| Antibody name | Human EC50 (nM) | Mouse EC50 (nM) | Cynomolgus monkey EC50 (nM) |
|---|---|---|---|
| 33+31 | 0.007506 | 0.01032 | 0.02714 |
| 33+22 | 0.007027 | 0.01087 | 0.03657 |
| Rovalpituzumab | 0.008867 | 0.01337 | 0.01488 |

Next, we used Fortebio to determine the affinities of the biepitopic antibodies; the experimental method was the same as that in Example 2. The results are shown in Table 12. The above results show that all the biepitopic antibodies were comparable or superior in affinity to rovalpituzumab.

**Table 12. The affinities of biepitopic antibodies for human DLL3 protein as determined by Fortebio**

| Sample | KD (M) | ka (1/Ms) | kd (1/s) |
|---|---|---|---|
| 31+22 | 8.14E-11 | 1.43E+06 | 1.16E-04 |
| 33+31 | 1.33E-10 | 1.49E+06 | 1.98E-04 |
| 33+22 | < 1.0E-12 | 9.53E+05 | < 1.0E-07 |
| Rovalpituzumab | 1.33E-10 | 7.34E+05 | 9.78E-05 |

### Example 13: Binding of Biepitopic Antibodies to Cells Expressing Human DLL3

The abilities of biepitopic antibodies to bind to cell surface DLL3 were assessed using SHP77 cells. Cells (density: 2 × 10⁶ cells/mL) were seeded in a 96-well plate at 100 µL/well. The antibodies (137 nM) were added, and the plate was incubated at 4 °C for 1 h. A goat anti-human secondary antibody was added, and labeling was performed at 4 °C for 45 min in a dark place. The cells were collected and washed, and then the fluorescence intensity of the cells was measured with a flow cytometer. The experimental results are shown in Table 13. The results show that the abilities of the biepitopic antibodies to bind to cells highly expressing DLL3 were superior to that of the control molecule rovalpituzumab.

**Table 13. The binding of biepitopic antibodies to cell surface DLL3 protein**

| Antibody name | Reading (137 nM) |
|---|---|
| Rovalpituzumab | 28,373 |
| 31+22 | 34,752 |
| 33+31 | 43,130 |
| 33+22 | 44,134 |

### Example 14: Endocytic Activity of Biepitopic Antibodies

The endocytosis rates of DLL3 biepitopic antibodies were determined by FACS, and the abilities of the biepitopic antibodies to induce the endocytosis of cell surface DLL3 were assessed using SHP77 cells. Cells (density: 2 × 10⁶ cells/mL) were seeded in a 96-well plate at 100 µL/well, and the antibodies (34.2 nM) were added. The plate was incubated at 4 °C for 1 h. The cells were washed with FACS buffer and then incubated at 4 °C and 37 °C for 2 h. A goat anti-human secondary antibody was added, and labeling was performed at 4 °C for 45 min in a dark place. The cells were collected and washed, and then the fluorescence intensity of the cells was measured with a flow cytometer. Internalization ratios were calculated: [(reading at 4 °C - negative control reading) - (reading at 37 °C - negative control reading)] / (reading at 4 °C - negative control reading) × 100%.

The calculated 2-h endocytosis ratios of the antibodies are shown in Table 14. The results show that the biepitopic antibodies exhibited relatively good endocytic activity, and that 31+22 was superior in endocytic ability to 33+31, 33+22, and rovalpituzumab.

**Table 14. A 2-h endocytosis experiment of DLL3 biepitopic antibodies**

| Antibody name | Endocytosis rate of binding to SHP77 cells (%) |
|---|---|
| Rovalpituzumab | 54.20% |
| 31+22 | 70.34% |
| 33+31 | 53.80% |
| 33+22 | 64.26% |

### Example 15: Study of Cytotoxicity of DLL3 Biepitopic Antibody-Drug Conjugates

JSSW-001 (Shanghai Haoyuan Chemexpress Co., Ltd.) was conjugated to biepitopic antibodies by cysteine conjugation (the method was the same as that in Example 8) to obtain 33+31ADC, 31+22ADC, and 33+22ADC, which had a DAR of 8. SHP77 cells, which highly express DLL3, were selected as the cell strain for assessing *in vitro* activity; the method was the same as that in Example 9. Data were read with a microplate reader and then processed with the survival rate of the drug-free whole cell group being 100%, and EC₅₀ was calculated. The results are shown in FIG. 11 and Table 15. The results show that the biepitopic ADCs exhibited significant *in vitro* cell killing effects on SHP77 cells, which highly express DLL3, with EC₅₀ values reaching the pM level.

**Table 15. A killing experiment of DLL3 antibody-drug conjugates**

| Antibody-drug conjugate name | EC₅₀ value (pM) |
|---|---|
| 33+31ADC | 449.7 |
| 31+22ADC | 60.05 |
| 33+22ADC | 36.27 |

### Example 16: Humanization of DLL3 Antibodies

Homology Modeling was performed using Discovery Studio and Schrödinger Antibody Modeling. Through structural simulation and rational design, human framework regions that most closely resemble the murine antibody framework regions were obtained. The CDRs of the light and heavy chains were grafted onto the framework sequences of matched human light and heavy chain genes, respectively, to obtain humanized antibodies Hab31B6G8, Hab33B10E8, and Hab22B12E5. Then, 3D models were constructed through Discovery Studio and Schrödinger Antibody Modeling to analyze whether there was any framework position where the substitution of a mouse amino acid with a human amino acid affected binding and/or the CDR conformation. Back mutations were performed, and the resulting sequences are shown in Table 16.

**Table 16. Sequences with VH back mutations (for the CDRs, the Kabat numbering scheme was used)**

| | | |
|---|---|---|
| Hab22B12E5 -VH(SEQ ID NO.54) | Heavy chain CDR1: DYNMY | SEQ ID NO.1 |
| | Heavy chain CDR2: YIDPYNGVTGFNQKFKG | SEQ ID NO.2 |
| | Heavy chain CDR3: GQGGYYIDYAMDY | SEQ ID NO.3 |
| | QVQLVQSGAEVKKPGASVKVSCKASGYTFTDYNMYWVRQAPGQRLEW MGYIDPYNGVTGFNQKFKGRVTITRDTSASTAYMELSSLRSEDTAVYYCA RGQGGYYIDYAMDYWGQGTSVTVSS (SEQ ID NO.54) | |
| Hab22B12E5 -VL(SEQ ID NO.55) | Light chain CDR1: RASQDIGDTLA | SEQ ID NO.4 |
| | Light chain CDR2: ATSNLDS | SEQ ID NO.5 |
| | Light chain CDR3: LQYAISPYT | SEQ ID NO.6 |
| | DIQMTQSPSSLSASVGDRVTITCRASQDIGDTLAWFQQKPGKAPKRLIYAT SNLDSGVPSRFSGSRSGTDFTLTISSLQPEDFATYYCLQYAISPYTFGGGTK LEIK (SEQ ID NO.55) | |
| Hab31B6G8-VH(SEQ ID NO.56) | Heavy chain CDR1: NYGMK | SEQ ID NO.17 |
| | Heavy chain CDR2: WINTYTGEPTYADDFKG | SEQ ID NO.18 |
| | Heavy chain CDR3: FSDYGGFDY | SEQ ID NO.19 |
| | QVQLVQSGAEVKKPGASVKVSCKASGYTFTNYGMKWVRQAPGQGLEW MGWINTYTGEPTYADDFKGRVTMTRDTSISTAYMELSRLRSDDTAVYYC ARFSDYGGFDYWGQGTTLTVSS (SEQ ID NO.56) | |
| Hab31B6G8-VL(SEQ ID NO.57) | Light chain CDR1: KASQSVSNDVA | SEQ ID NO.20 |
| | Light chain CDR2: YASNRYT | SEQ ID NO.21 |
| | Light chain CDR3: QQDYSSPFT | SEQ ID NO.22 |
| | DIVMTQSPDSLAVSLGERATINCKASQSVSNDVAWYQQKPGQPPKLLIYY ASNRYTGVPDRFSGSGSGTDFTLTISSLQAEDVAVYYCQQDYSSPFTFGSG TKLEIK (SEQ ID NO.57) | |
| Hab33B10E8 -VH(SEQ ID NO.58) | Heavy chain CDR1: DYYMK | SEQ ID NO.25 |
| | Heavy chain CDR2: AFNLNNGDTFYNQKFKG | SEQ ID NO.26 |
| | Heavy chain CDR3: DVYGYGDY | SEQ ID NO.27 |
| | QVQLVQSGAEVKKPGASVKVSCKASGYTFTDYYMKWVRQAPGQRLEW MGAFNLNNGDTFYNQKFKGRVTITRDTSASTAYMELSSLRSEDTAVYYCA RDVYGYGDYWGQGTSVTVSS (SEQ ID NO.58) | |
| Hab33B10E8 -VL(SEQ ID NO.59) | Light chain CDR1: RASKSVSTSGYSYMH | SEQ ID NO.28 |
| | Light chain CDR2: LASNLDS | SEQ ID NO.29 |
| | Light chain CDR3: QHSRELPYT | SEQ ID NO.30 |
| | DIVMTQSPDSLAVSLGERATINCRASKSVSTSGYSYMHWYQQKPGQPPKL LIYLASNLDSGVPDRFSGSGSGTDFTLTISSLQAEDVAVYYCQHSRELPYTF GGGTKLEIK (SEQ ID NO.59) | |

### Example 17: Determination of Affinities of Humanized Anti-DLL3 Antibodies

The humanized antibody variable regions obtained in Example 16 and the human IgG1/Kappa constant region were used to construct intact antibodies, and the antibodies were expressed and purified. Amino acid mutations L234A/L235A/P329A/P331S (numbered according to the EU index of Kabat) were introduced into the Fc sequences. The affinities of the humanized antibodies for human DLL3 protein were determined by Fortebio; the method was the same as that in Example 2. According to the association curves, the affinities of the antibodies for the antigen were calculated by regression analysis using analysis software. The results are shown in Table 17.

**Table 17. The affinities of humanized antibodies for human DLL3 protein as determined by Fortebio**

| Sample | KD (M) | kon (1/Ms) | kdis (1/s) | Full R^2 |
|---|---|---|---|---|
| Hab31B6G8 | 6.15E-10 | 5.97E+05 | 3.67E-04 | 0.9977 |
| Hab33B10E8 | 6.10E-10 | 4.66E+05 | 2.84E-04 | 0.9988 |
| Hab22B12E5 | 1.13E-09 | 4.45E+05 | 5.01E-04 | 0.9983 |

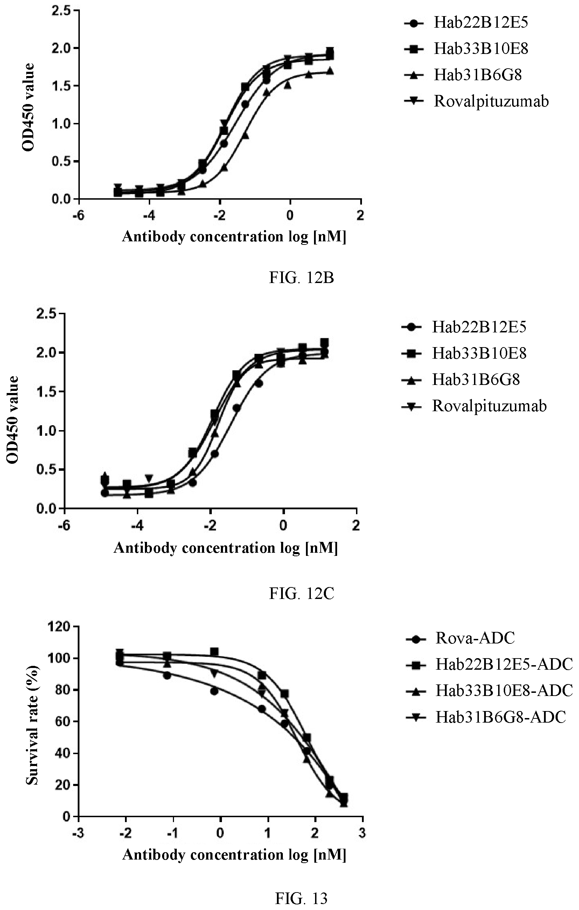

Next, the affinities of the humanized antibodies for recombinant human, cynomolgus monkey, and mouse DLL3 proteins were determined by ELISA. The affinities obtained after curves were fit to the results using GraphPad are shown in FIGs. 12A-12C and Table 18.

**Table 18. EC₅₀ values for the affinities of humanized antibodies as determined by ELISA**

| Antibody name | Human EC50 (nM) | Mouse EC50 (nM) | Cynomolgus monkey EC50 (nM) |
|---|---|---|---|
| Hab22B12E5 | 0.01877 | 0.03569 | 0.02832 |
| Hab33B10E8 | 0.008329 | 0.0116 | 0.01394 |
| Hab31B6G8 | 0.009 | 0.01622 | 0.05172 |
| Rovalpituzumab | 0.008867 | 0.01337 | 0.01488 |

The above results show that humanized antibodies Hab33B10E8 and Hab31B6G8 exhibited affinities substantially comparable to that of rovalpituzumab.

### Example 18: Binding of Humanized Antibodies to Cells Expressing Human DLL3

The abilities of humanized antibodies to bind to cell surface DLL3 were assessed using SHP77/NCI-H82 cells. Cells (density: 2 × 10⁶ cells/mL) were seeded in a 96-well plate at 100 µL/well. The antibodies (66.7 nM) were added, and the plate was incubated at 4 °C for 1 h. A goat anti-human secondary antibody was added, and labeling was performed at 4 °C for 45 min in a dark place. The cells were collected and washed, and then the fluorescence intensity of the cells was measured with a flow cytometer. The experimental results are shown in Table 19. The results show that the humanized antibodies could bind to the DLL3 protein expressed on the cell line. The affinity of antibody Hab33B10E8 for cell surface DLL3 was superior to that of the control molecule rovalpituzumab.

**Table 19. The binding of humanized antibodies to cell surface DLL3**

| Humanized antibody name | SHP77 Reading (66.7 nM) | NCI-H82 Reading (66.7 nM) |
|---|---|---|
| Hab22B12E5 | 21,605 | 22,839 |
| Hab31B6G8 | 23,445 | 23,134 |
| Hab33B10E8 | 41,350 | 35,480 |
| Rovalpituzumab | 26,848 | 29,781 |

### Example 19: Assessment of Endocytic Activity of Humanized Antibodies

The endocytosis rates of DLL3 humanized antibodies were determined by FACS, and the abilities of the humanized antibodies to induce the endocytosis of cell surface DLL3 were assessed using the human small cell lung cancer cell strains SHP77 and NCI-H82. Cells (density: 2 × 10⁶ cells/mL) were seeded in a 96-well plate at 100 µL/well, and the antibodies (final concentration: 34.2 nM) were added. The plate was incubated at 4 °C for 1 h. The cells were washed with FACS buffer and then incubated at 4 °C and 37 °C for 2 h. A goat anti-human secondary antibody was added, and labeling was performed at 4 °C for 45 min in a dark place. The cells were collected and washed, and then the fluorescence intensity of the cells was measured with a flow cytometer. Internalization ratios were calculated: [(reading at 4 °C - negative control reading) - (reading at 37 °C - negative control reading)] / (reading at 4 °C - negative control reading) × 100%.

The calculated 2-h endocytosis ratios of the antibodies are shown in Table 20. The results show that the endocytic activity of Hab33B10E8 on SHP77 cells was superior to that of rovalpituzumab, and that the endocytic activity of Hab33B10E8 and Hab22B12E5 on NCI-H82 cells was superior to that of rovalpituzumab.

**Table 20. A 2-h endocytosis experiment of humanized antibodies**

| Humanized antibody name | Endocytosis rate of binding to SHP77 cells (%) | Endocytosis rate of binding to NCI-H82 cells (%) |
|---|---|---|
| Hab33B10E8 | 81.68% | 83.36% |
| Hab31B6G8 | 62.45% | 60.55% |
| Hab22B12E5 | 72.87% | 72.14% |
| Rovalpituzumab | 73.20% | 63.20% |

### Example 20: Evaluation of In Vitro Cytotoxicity of Humanized ADC Samples

JSSW-001 (Shanghai Haoyuan Chemexpress Co., Ltd.) was conjugated to humanized antibodies Hab22B12E5, Hab31B6G8, and Hab33B10E8 by cysteine conjugation (the method was the same as that in Example 8) to obtain Hab22B12E5-ADC, Hab31B6G8-ADC, and Hab33B10E8-ADC, respectively. The small cell lung cancer cell strain SHP77, which highly expresses DLL3, was selected to assess *in vitro* activity, and the dose-response relationships of different antibody-drug conjugates in cell killing were observed. Cells (density: 2 × 10⁴ cells/mL) were seeded in a 96-well plate at 50 µL/well. The antibodies (serially diluted 3-fold, final concentrations: 0-400 nM) were added. After six days of co-incubation, Cell Titer Glo^{®} Luminescent Cell Viability Assay Reagent was added, and the plate was shaken at room temperature for 10 min for thorough mixing. Data were read with a microplate reader and then processed with the survival rate of the drug-free whole cell group being 100%, and IC₅₀ was calculated. The results are shown in FIG. 13 and Table 21. The results show that all the humanized ADCs exhibited significant *in vitro* cell killing effects on cells highly expressing DLL3, and that Hab33B10E8-ADC and Hab22B12E5-ADC exhibited relatively the strongest *in vitro* cytotoxic activity.

**Table 21. A killing experiment of DLL3 antibody-drug conjugates**

| Antibody-drug conjugate name | EC₅₀ value (nM) |
|---|---|
| Rova-ADC | NA |
| Hab22B12E5-ADC | 69.03 |
| Hab31B6G8-ADC | 853.4 |
| Hab33B10E8-ADC | 41.65 |

| | |
|---|---|
| Note: NA means that no EC₅₀ value was obtained. | |

### Example 21: Biological Activity of DLL3 Humanized Antibody-Drug Conjugates in Animals

### 21.1. Efficacy of humanized ADC samples against human small cell lung cancer NCI-H82 cell xenograft tumors

The monotherapy efficacy of Hab22B12E5-ADC, Hab33B10E8-ADC, and Rova-ADC (positive control) was assessed in a tumor xenograft model. 1 × 10⁶ NCI-H82 human small cell lung cancer cells were inoculated into NU/NU mice. On day 12 after the inoculation, Hab22B12E5-ADC, Hab33B10E8-ADC, and Rova-ADC (positive control), as well as a 0.9% sodium chloride injectable solution control, were intravenously injected once at 2 mg/kg. The *in vivo* anti-tumor effects of the DLL3-ADCs (humanized antibodies) were investigated.

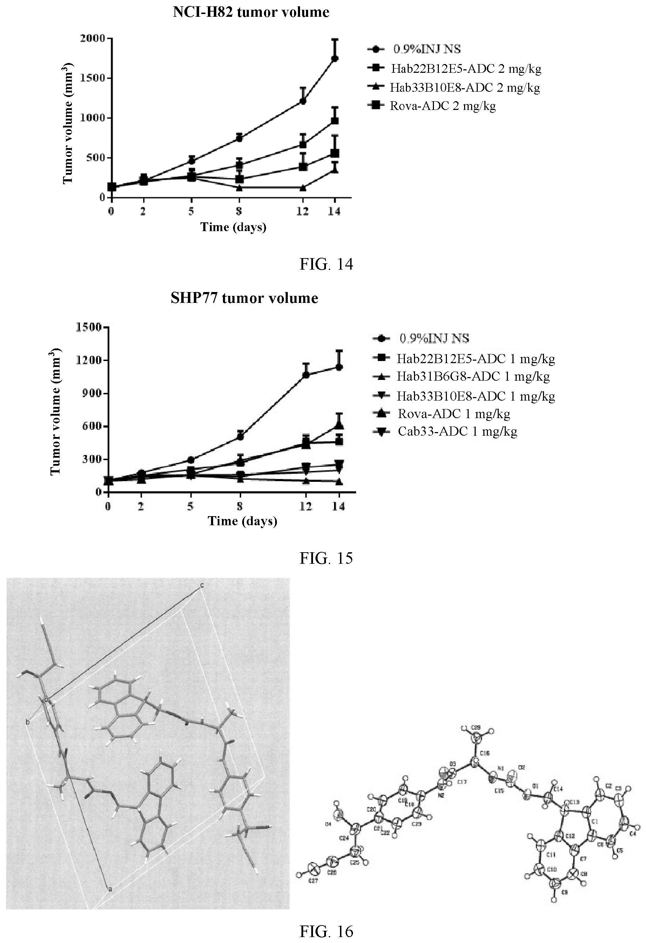

At the end of the experiment, compared to the solvent group, the Hab22B12E5-ADC group resulted in 48.3% tumor inhibition and a tumor volume of 966.0 ± 377.1, the Hab33B10E8-ADC group resulted in 86.3% tumor inhibition and a tumor volume of 351.1 ± 216.8, and the Rova-ADC (positive control) group resulted in 74.0% tumor inhibition and a tumor volume of 599.1 ± 495.6. Compared to the solvent group, the groups could all significantly inhibit the volume of xenograft tumors (P < 0.05), and the anti-tumor effect of Hab33B10E8-ADC was superior to that of the control molecule Rova-ADC. See FIG. 14 and Table 22 for details.

**Table 22. The tumor parameters of the groups on day 14 after administration of the DLL3-ADCs (humanized antibodies)**

| Group | DAR | Dose (mg/kg) | Route of administration | Tumor volume (mm³) | | TGI% |
|---|---|---|---|---|---|---|
| | | | | D0 | D14 | |
| 0.9%INJ NS | -- | -- | i.v | 130.4+ 41.6 | 1750+ 531.4 | -- |
| Hab22B12E5-ADC | 8 | 2mg/kg(D0) | | 128.7+ 39.2 | 966.0+ 377.1* | 48.3 |
| Hab33B10E8-ADC | | | | 128.6± 38.5 | 351.1+216.8*** | 86.3 |
| Rova-ADC (positive reference)^{#} | | | | 137.5+ 57.7 | 599.1+ 495.6** | 74.0 |

| | | | | | | |
|---|---|---|---|---|---|---|
| ^{#}Rova-ADC (positive reference) had a DAR of 7.9. | | | | | | |

### 21.2. Efficacy of humanized ADC samples against human small cell lung cancer SHP77 cell xenograft tumors

The monotherapy efficacy of Hab22B12E5-ADC, Hab31B6G8-ADC, Hab33B10E8-ADC, chimeric antibody 33B10E8-ADC (Cab33-ADC), and Rova-ADC (positive reference) was assessed in a tumor xenograft model. 1 × 10⁷ SHP77 human small cell lung cancer cells were inoculated into NCG mice. On day 6 after the inoculation, Hab22B12E5-ADC, Hab31B6G8-ADC, Hab33B10E8-ADC, Cab33-ADC, and Rova-ADC (positive control), as well as a 0.9% sodium chloride injectable solution control, were intravenously injected once at 1 mg/kg. The *in vivo* anti-tumor effects of the DLL3-ADCs (humanized antibodies) were investigated.

Compared to the solvent group, the Hab22B12E5-ADC group resulted in 65.9% tumor inhibition and a tumor volume of 459.1 ± 160.4, the Hab31B6G8-ADC group resulted in 100.6% tumor inhibition and a tumor volume of 101.8 ± 20.5, the Hab33B10E8-ADC group resulted in 91.0% tumor inhibition and a tumor volume of 201.7 ± 102.5, the Cab33-ADC (chimeric) group resulted in 85.7% tumor inhibition and a tumor volume of 255.1 ± 60.8, and the Rova-ADC (positive control) group inhibited tumor growth and resulted in 50.8% tumor inhibition and a tumor volume of 613.7 ± 252.1. Compared to the solvent group, the groups (P < 0.05) could all significantly inhibit the volume of xenograft tumors. All molecules were superior to the control Rova-ADC. See FIG. 15 and Table 23 for details.

**Table 23. The tumor parameters of the groups on day 14 after administration of the DLL3-ADCs (humanized antibodies)**

| Group | DAR | Dose (mg/kg) | Route of administration | Tumor volume (mm³) | | TGI% |
|---|---|---|---|---|---|---|
| | | | | D0 | D14 | |
| 0.9%INJ NS | -- | -- | | 109.1± 24.3 | 1139.5± 362.1 | -- |
| Hab22B12E5-ADC | 8 | 1mg/kg(D0) | | 107.7± 19.1 | 459.1± 160.4** | 65.9 |
| Hab31B6G8-ADC | | | | 107.8± 18.5 | 101.8±20.5*** | 100.6 |
| Hab33B10E8-ADC | | | i.v | 108.6± 17.8 | 201.7±102.5*** | 91.0 |
| Rova-ADC (positive reference)^{#} | | | | 106.3± 13.9 | 613.7± 252.1* | 50.8 |
| Cab33-ADC (chimeric)^{##} | | | | 107.6± 18.1 | 255.1± 60.8*** | 85.7 |

| | | | | | | |
|---|---|---|---|---|---|---|
| ^{#}Rova-ADC (positive reference) had a DAR of 7.9. ^{##}Cab33-ADC had a DAR of 6.4. | | | | | | |

### Example 22: Preparation of Antibody-Drug Conjugate (ADC) Hab33B10E8-045

### 22.1. Preparation of compound 045

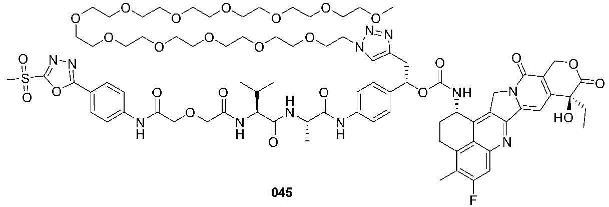

### 1. Preparation of intermediate compound 18

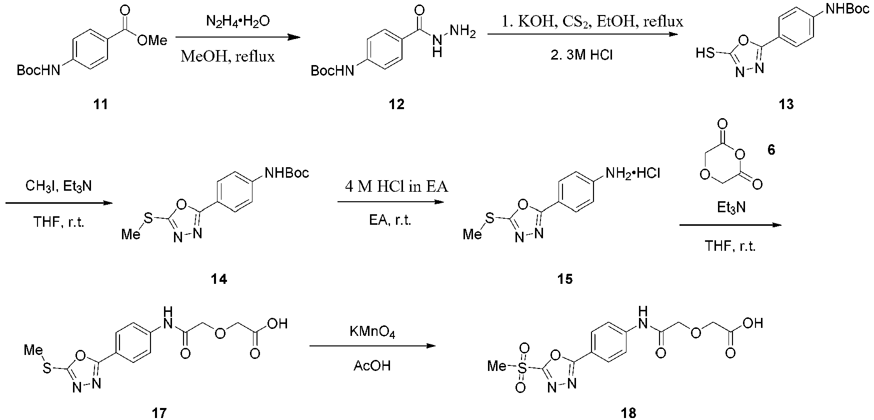

Compound 11 (47.5 g, 189 mmol) and methanol (250 mL) were added to a reaction flask, and 80% hydrazine hydrate (35.4 g, 567 mmol) was slowly added at room temperature. The mixture was heated to 70 °C and refluxed for 6 h. After cooling, a white crystal precipitated. The mixture was filtered under vacuum, and the remaining solid was washed with methanol (20 mL × 3) to give **12** (47.47 g, yield: 100%) as a white solid. MS (ESI): (M+H)⁺, calculated 252.1, found 252.2.

Compound **12** (47.4 g, 189 mmol), potassium hydroxide (12.7 g, 227 mmol), and ethanol (400 mL) were added to a reaction flask, and the mixture was stirred for dissolution at room temperature. Carbon disulfide (17 g, 283 mmol) was slowly added, and then the mixture was heated to 100 °C and refluxed for 5 h. The solvent was removed by concentration under reduced pressure, and then water (50 mL) was added. The pH of the mixture was adjusted to 6 with dilute hydrochloric acid, and extraction was performed with dichloromethane (100 mL × 3). The organic phases were combined, washed with saturated brine, dried over anhydrous sodium sulfate, and filtered, and then the filtrate was concentrated under reduced pressure. The residue was purified by silica gel chromatography to give compound **13** (49.9 g, yield: 90%). MS (ESI): (M+H)⁺, calculated 294.1, found 294.3.

Compound **13** (5.86 g, 20 mmol), triethylamine (2.42 g, 24 mmol), and tetrahydrofuran (36 mL) were added to a reaction flask, and iodomethane (3.12 g, 22 mmol) was added to the reaction solution described above. After 1.5 h of stirring at 25 °C, the reaction was complete. The solvent was removed by concentration under reduced pressure, and the residue was purified by silica gel chromatography to give compound **14** (4.05 g, yield: 66%). MS (ESI): (M+H)⁺, calculated 308.1, found 308.2.

Compound **14** (3.3 g, 10.6 mmol) and ethyl acetate (20 mL) were added to a reaction flask, and a solution of hydrogen chloride in ethyl acetate (2.7 mL, 4 M) was added dropwise at 25 °C. The mixture was left to react for 6 h, and the solvent was removed by concentration under reduced pressure to give crude **15**, which was directly used in the next step.

Crude **15**, diglycolic anhydride **6** (1.35 g, 11.7 mmol), triethylamine (2.14 g, 21.2 mmol), and tetrahydrofuran (30 mL) were added to a reaction flask. The mixture was left to react at 25 °C for 1.5 h, and the solvent was removed by concentration under reduced pressure. Diethyl ether was added to the residue. After suction filtration, washing was performed with water and diethyl ether to give compound **17** (3.4 g, yield: 99%). MS (ESI): (M+H)⁺, calculated 324.1, found 324.2.

Compound **17** (3.4 g, 10.5 mmol) and glacial acetic acid (20 mL) were added to a reaction flask. After dissolution, potassium permanganate (2.48 g, 15.7 mmol) was added at 0 °C. The mixture was warmed to 25 °C and left to react for 1 h. A saturated sodium sulfite solution was added until the solution became colorless, and extraction was performed with ethyl acetate (100 mL × 3). The organic phases were combined, washed with saturated brine, dried over anhydrous sodium sulfate, and filtered, and then the filtrate was concentrated under reduced pressure. The residue was purified by silica gel chromatography to give compound **18** (2.4 g, yield: 65%). MS (ESI): (M+H)⁺, calculated 356.0, found 356.0.

### 2. Preparation of compound 045

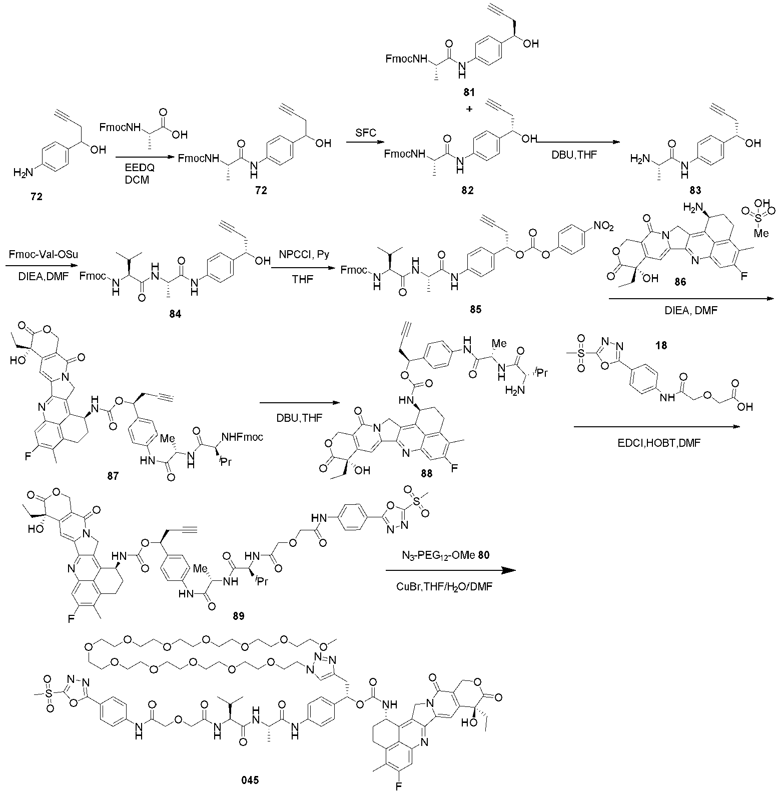

1-(4-Aminophenyl)-3-butyn-1-ol (4997 mg, 31 mmol), Fmoc-Ala-OH (9651 mg, 31 mmol), EEDQ (11,498 mg, 46.5 mmol), and extra-dry DCM (90 mL) were added to a reaction flask at 0 °C. After the mixture was left to react for 3 h, the solvent was removed by concentration under reduced pressure, and the residue was triturated with 500 mL of MTBE to give compound 72 (9007 mg, yield: 64%, *dr =* 1:1). MS (ESI): (M+H)⁺, calculated 455.2, found 455.2.

Chiral resolution of intermediate 72 (retention times: 13.55 min and 16.29 min): Compound 72 (50 g) was resolved by SFC to give 81 (19 g, retention time: 13.55 min) and 82 (20 g, retention time: 16.29 min). SFC resolution method: column model: DAICEL CHIRALCEL OD (250 mm-50 mm, 10 µm); mobile phases: A: CO₂, B: CO₂-ACN/i-PrOH (0.1% NH₃H₂O); isocratic elution: B in A for 50%; flow rate: 200 mL/min; detector: PDA; column temperature: 25 °C; back pressure: 100 Bar.

HPLC method: instrument information: Thermo liquid chromatograph (ADC-U3000-01); chromatography column: CHIRALPAK^{®}ID (4.6 × 150 mm, 5 µm); column temperature: 25 °C; sample tray temperature: 25 °C; mobile phases: A: 10 mM NH₄FA; B: ACN; flow rate: 0.8 mL/min; detection wavelength: 254 nm; sample injection volume: 2 µL.

### Gradient conditions:

| Time (min) | A% | B% |
|---|---|---|
| 0 | 40.0 | 60.0 |
| 30.000 | 40.0 | 60.0 |

X-RAY confirmed that compound **82** had the structure and configuration shown below. Its single-crystal diffraction pattern is shown in FIG. 16. 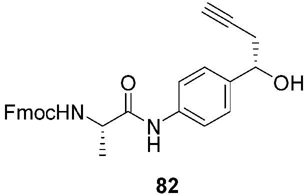 **82** (3632 mg, 8 mmol) and 90 mL of THF (commercially available) were added to a reaction flask at 0 °C. While the mixture was stirred, DBU (1215 mg, 8 mmol) was slowly added. The mixture was left to react for half an hour and then warmed to room temperature. After TLC monitoring showed the disappearance of the starting materials, the solvent was removed by concentration under reduced pressure, and the residue was purified by silica gel chromatography (DCM/MeOH 95:5) to give compound **83** (1707 mg, yield: 92%). MS (ESI): (M+H)⁺, calculated 233.1, found 233.2.

Compound **83** (1624 mg, 7 mmol), Fmoc-Val-OSu (362 mg, 8.4 mmol), and N,N-dimethylformamide (150 mL) were added to a reaction flask. While the mixture was stirred, DIEA (1158 µL, 7 mmol) was slowly added dropwise. The mixture was left to react at 25 °C for 12 h, and the solvent was removed by concentration under reduced pressure. The residue was triturated with 50 mL of EA and 50 mL of PE, and a white solid precipitated; this procedure was repeated three times to give compound **84**. MS (ESI): (M+H)⁺, calculated 554.3, found 554.4.

Compound **84** (1661 mg, 3 mmol), 4-nitrophenyl chloroformate (1206 mg, 6 mmol), and THF (150 mL) were added to a reaction flask. While the mixture was stirred, Py (474 µL, 6 mmol) was added dropwise. The mixture was left to react at 65 °C for 1 h, and the solvent was removed by concentration under reduced pressure. The residue was purified by silica gel chromatography (DCM/MeOH 98:2) to give compound **85** (1831 mg, yield: 85%). MS (ESI): (M+H)⁺, calculated 719.8, found 719.9.

Compound **85** (1436 mg, 2 mmol), exatecan mesylate **86** (1168.2 mg, 2.2 mmol), and N,N-dimethylformamide (100 mL) were added to a reaction flask. While the mixture was stirred, DIEA (695 µL, 4 mmol) was slowly added dropwise. The mixture was left to react at 25 °C for 20 h, and the solvent was removed by concentration under reduced pressure. The residue was purified by silica gel chromatography (DCM/MeOH 95:5) to give compound **87** (1664 mg, yield: 82%). MS (ESI): (M+H)⁺, calculated 1015.4, found 1015.1.

**87** (1522 mg, 1.5 mmol) and 40 mL of THF (commercially available) were added to a reaction flask at 0 °C. While the mixture was stirred, DBU (228 mg, 1.5 mmol) was slowly added. The mixture was left to react for half an hour, warmed to room temperature, and left to react for another 40 min until TLC showed the disappearance of the starting materials. The reaction solution was concentrated under vacuum to remove THF, and 15 mL of DCM and 300 mL of PE were added, resulting in the production of a large amount of yellowish-green solid. The solid was collected by filtration with a Büchner funnel to give **88** (1177 mg, yield: 99%). MS (ESI): (M+H)⁺, calculated 793.3, found 793.6.

Compound **88** (1031 mg, 1.3 mmol), **18** (554 mg, 1.56 mmol), EDCI (498 mg, 2.6 mmol), HOBT (263 mg, 1.95 mmol), and N,N-dimethylformamide (20 mL) were added to a reaction flask. The mixture was left to react at 25 °C for 1 h, and the solvent was removed by concentration under reduced pressure. The residue was purified on a C18 column (60% ACN/0.05% formic acid in H₂O), and the corresponding fraction was lyophilized to give compound **89** (1322 mg, yield: 90%). MS (ESI): (M+H)⁺, calculated 1130.4, found 1130.7.

Compound **89** (903 mg, 0.8 mmol), compound **80** (702 mg, 1.2 mmol), tris(3-hydroxypropyltriazolylmethyl)amine (34.7 mg, 0.08 mmol), and cuprous bromide (11.4 mg, 0.08 mmol) were added to a reaction flask. The mixture was purged three times with nitrogen, and a mixture of THF/DMF/H₂O (3.5 mL:0.6 mL:0.4 mL) was added. The resulting mixture was left to react at 25 °C for 0.5 h. The residue was purified on a preparative C18 column (62% ACN/0.05% formic acid in H₂O) to give compound **045** (1276 mg, yield: 93%). MS (ESI): (M+H)⁺, calculated 1715.7, found 1716.2. ¹H NMR (600 MHz, DMSO) δ 10.71 (s, 1H), 10.08 (s, 1H), 8.48 (d, *J* = 6.7 Hz, 1H), 8.17 (d, *J* = 8.6 Hz, 3H), 8.09 (d, *J* = 8.8 Hz, 1H), 8.03 (d, *J* = 8.7 Hz, 2H), 7.89 (s, 1H), 7.81 (d, *J* = 10.8 Hz, 1H), 7.66 (d, *J* = 8.4 Hz, 2H), 7.42-7.36 (m, 3H), 6.61 (s, 1H), 6.02 (t, *J* = 6.8 Hz, 1H), 5.55 (s, 2H), 5.38-5.26 (m, 3H), 4.55-4.47 (m, 3H), 4.46-4.41 (m, 1H), 4.36 (q, *J* = 15.6 Hz, 2H), 4.28 (s, 2H), 3.80 (s, 5H), 3.62-3.55 (m, 36H), 3.52-3.47 (m, 9H), 3.44-3.36 (m, 2H), 3.30-2.29 (m, 1H), 3.19-3.09 (m, 1H), 2.42 (s, 3H), 2.28-2.19 (m, 1H), 2.17 (s, 3H), 2.17-2.10 (m,1H), 2.04-1.89 (m, 2H), 1.42 (d, *J* = 7.0 Hz, 3H), 1.02 (d, *J* = 6.7 Hz, 3H), 0.98 (t, *J* = 7.2 Hz, 6H).

### 22.2. Preparation of antibody-drug conjugate (ADC) Hab33B10E8-045

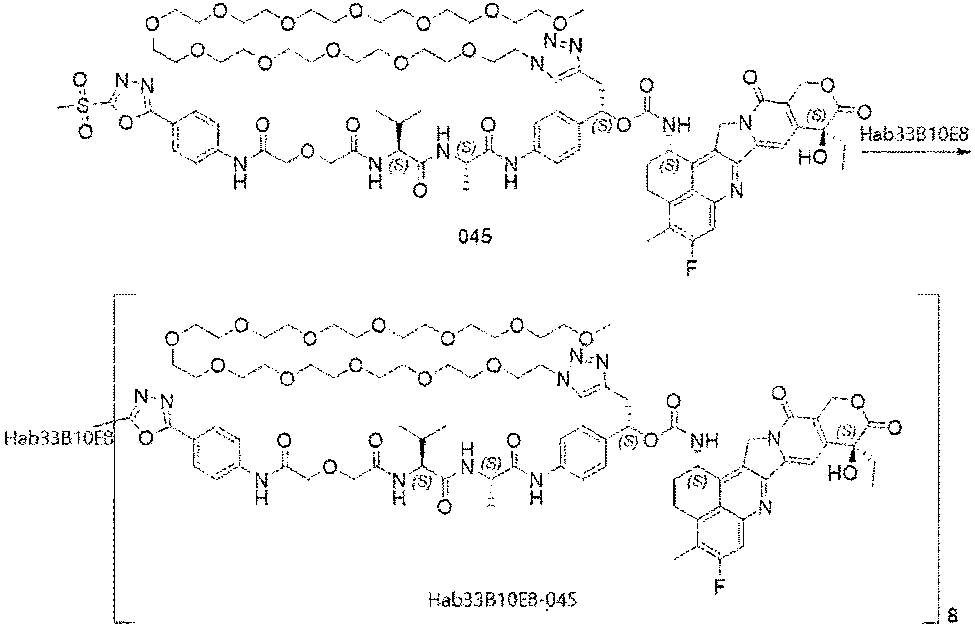

The concentration of antibody Hab33B10E8 was adjusted to 10 g/L using phosphate-buffered saline (pH 7.0). After buffer exchange, the antibody solution was placed into a centrifuge tube, and 10.0 equivalents of 5 mM TCEP (Adamas-beta, Ltd.) were added. The mixture was left to react at 37 °C for 1 h to reduce the interchain disulfide bonds of the antibody to sulfhydryl. Subsequently, a 20 mM solution of compound 045 in DMSO was added (12 equivalents of 045 per antibody). The mixture was left to react at 25 °C for 1 h. After conjugation, the solution was purified using an ultrafiltration membrane cassette (Cobetter, Ltd.) to remove the linker-toxin residue, and the conjugate was stored in PBS (pH 7.4). The average drug loading per antibody was determined to be about 8 by reversed-phase chromatography.

### Example 23: Determination of Binding Activity of Hab33B10E8-045 by Enzyme-Linked Immunosorbent Assay

The affinity of the antibody for recombinant human DLL3 protein was determined by ELISA. Human DLL3 protein (Kactus, Cat. No. DLL-HM103) was diluted to a final concentration of 0.1 µg/mL and added to a 96-well microplate, and the plate was incubated overnight at 4 °C. The next day, the supernatants were discarded. After blocking, the antibody (serially diluted 4-fold, 0-100 nM) was added. After incubation with a secondary antibody, color development was performed with a TMB chromogenic solution for 10 min. After 2 M HCl was added to stop the reactions, the absorbance was measured at 450 nm with a microplate reader, and EC₅₀ was calculated. The results are shown in Table 24. The affinity of Hab33B10E8-045 for human DLL3 protein was substantially comparable to the binding ability of Hab33B10E8 before conjugation.

**Table 24. Determination of the binding activity of Hab33B10E8-045 by enzyme-linked immunosorbent assay**

| No. | EC50 (nM) | Emax |
|---|---|---|
| Hab33B10E8 | 0.001873 | 2.5 |
| Hab33B10E8-045 | 0.006276 | 2.5 |

### Example 24: In Vitro Cytotoxicity of Hab33B10E8-045

The *in vitro* cytotoxicity mediated by Hab33B10E8-045 was evaluated using the DLL3-positive cell strains NCI-H82, SHP77, NCI-H526, and NCI-H209 and the DLL3-negative cell strains A431 and NCI-H69. Cells were harvested, cultured with Hab33B10E8-045 (serially diluted), and then incubated at 37 °C. The viability was determined using CTL Plus after 6 days. EnVision 2105 (PerkinElmer) readings were taken and analyzed to determine the IC₅₀ (half-maximal inhibitory concentration) value.

Experimental process: The density of the test cells was adjusted to 6.7 × 10⁴ cells/mL, and the cells were seeded into 96-well plates at 75 µL/well and cultured overnight in a 5% CO₂ incubator at 37 °C. Hab33B10E8-045 (serially diluted 5-fold, a total of 9 concentrations, 0-100 nM) was added to the cells at 75 µL/well. The blank controls were the culture media for the corresponding cells. Two replicate wells were set for each concentration. After the cells were cultured in a 5% CO₂ incubator at 37 °C for 6 days, 50 µL of CTL Plus (manufacturer: Beyotime, Cat. No. C0068XL) luminescent reagent was added, and the cells were incubated at room temperature for 10 min in a dark place. The chemiluminescence was measured with an EnVision 2105 microplate reader. Data analysis and collation: With the blank control as a zero-killing control, inhibition rates were calculated according to the following formula: inhibition rate (%) = (1 - test group / blank control group) × 100%. Data were processed and analyzed using GraphPad Prism, and IC₅₀ was calculated. The results are shown in Table 25. The results show that Hab33B10E8-045 exhibited good *in vitro* cytotoxicity against the DLL3-positive cell strains.

**Table 25. The in vitro cytotoxicity of Hab33B10E8-045**

| Cell line | IC₅₀ (nM) |
|---|---|
| NCI-H82 | 4.4 |
| SHP77 | 36.56 |
| NCI-H526 | 16.71 |
| NCI-H209 | 1.07 |
| NCI-69 | > 100 |
| A431 | > 100 |

### Example 25: Pharmacodynamic Evaluation of Hab33B10E8-045 in Human Small Cell Lung Cancer SHP77 Xenograft Tumor Mouse Model

10⁷ cells of the DLL3-positive cell strain SHP77 were inoculated in the right forelimb axilla of each nu/nu nude mouse (7 mice per group). When the mean tumor volume of the mice reached about 100 mm³, the mice were randomly grouped and dosed once via intravenous injection. The day of grouping was defined as day 0, and dosing was started on day 0. After tumor cell inoculation, routine monitoring included assessing the effect of tumor growth and treatment on the normal behavior of the experimental animals, specifically the activity, food and water intake, increases or decreases in body weight (measured twice weekly), and abnormalities in the eyes, the coat, and other aspects. Calculation formula for tumor volume: tumor volume (mm³) = 1/2 × (a × b²) (where a represents long diameter, and b represents short diameter). The pharmacodynamic evaluation of the test drug is shown in Table 26.

**Table 26. A pharmacodynamic evaluation of the test drug in a lung cancer xenograft SHP-P77 mouse model**

| Group | Dose (mg/kg) | Mean tumor volume (day 0)^{a} | Mean tumor volume (day 22)^{a} | TGI(%)^{b} | *P* value vs. control group^{c} |
|---|---|---|---|---|---|
| Vehicle control (normal saline) | - | 104.1±15.0 | 2932.5±443.9 | - | - |
| Hab33B10E8-045 | 1 | 104.1±14.5 | 158.3±82.4 | 98.1 | < 0.0001 |

| | | | | | |
|---|---|---|---|---|---|
| Note: a. Data are presented as "mean ± standard error"; b. TGI% = [1 - (Ti - T0) / (Ci - C0)] × 100, where T0 and C0 are the mean tumor volumes of the treatment group and the vehicle control group on the day of grouping (day 0), respectively, and Ti and Ci are the mean tumor volumes of the treatment group and vehicle control group on day 22, respectively; c. vs. the tumor volume of the vehicle control group. | | | | | |

Compared to the vehicle control group, the ADC of the present disclosure (1 mg/kg) significantly inhibited tumor growth in the SHP-77 model, with a tumor growth inhibition (TGI) rate of 98.1%; there was no animal death or significant decrease in animal body weight in the treatment group, and no significant drug-related toxic reaction was observed, indicating that the ADC of the present disclosure was well tolerated by mice during the treatment.

### Example 26: Pharmacodynamic Evaluation of Hab33B10E8-045 in Human Small Cell Lung Cancer NCI-H526 and NCI-H69 Xenograft Tumor Mouse Models

10⁷ cells of the DLL3-positive cell strain NCI-H526 were inoculated in the right forelimb axilla of each nu/nu nude mouse (3 mice per group). When the mean tumor volume of the mice reached about 160 mm³ (1-2 groups), the mice were randomly grouped and dosed once via intravenous injection. The day of grouping was defined as day 0, and dosing was started on day 0. After tumor cell inoculation, routine monitoring included assessing the effect of tumor growth and treatment on the normal behavior of the experimental animals, specifically the activity, food and water intake, increases or decreases in body weight (measured twice weekly), and abnormalities in the eyes, the coat, and other aspects. Calculation formula for tumor volume: tumor volume (mm³) = 1/2 × (a × b²) (where a represents long diameter, and b represents short diameter). The pharmacodynamic evaluation of the test drug is shown in Table 27.

**Table 27. A pharmacodynamic evaluation of the test drug in a human small cell lung cancer xenograft NCI-H526 mouse model**

| Group | Dose (mg/kg) | Mean tumor volume (day 0)^{a} | Mean tumor volume (day 21)^{a} | TGI(%)^{b} |
|---|---|---|---|---|
| Vehicle control (normal saline) | - | 160.4±40.5 | 970.0±589.8 | - |
| Hab33B10E8-045 | 3 | 162.7±21.1 | 57.3±49.7 | 113.0 |

10⁷ cells of the DLL3-positive cell strain NCI-H69 were inoculated in the right forelimb axilla of each nu/nu nude mouse (3 mice per group). When the mean tumor volume of the mice reached about 140 mm³ (1-2 groups), the mice were randomly grouped and dosed once via intravenous injection. The day of grouping was defined as day 0, and dosing was started on day 0. After tumor cell inoculation, routine monitoring included assessing the effect of tumor growth and treatment on the normal behavior of the experimental animals, specifically the activity, food and water intake, increases or decreases in body weight (measured twice weekly), and abnormalities in the eyes, the coat, and other aspects. Calculation formula for tumor volume: tumor volume (mm³) = 1/2 × (a × b²) (where a represents long diameter, and b represents short diameter). The pharmacodynamic evaluation of the test drug is shown in Table 28.

**Table 28. A pharmacodynamic evaluation of the test drug in a human small cell lung cancer xenograft NCI-H69 mouse model**

| Group | Dose (mg/kg) | Mean tumor volume (day 0)^{a} | Mean tumor volume (day 14)^{a} | TGI(%)^{b} |
|---|---|---|---|---|
| Vehicle control (normal saline) | - | 137.1±59.1 | 908.4±54.3 | - |
| Hab33B10E8-045 | 3 | 142.7±53.2 | 323.7±244.6 | 76.5 |

Compared to the vehicle control group, the ADC of the present disclosure (3 mg/kg) significantly inhibited tumor growth in both the NCI-H526 model and the NCI-H69 model, with tumor growth inhibition (TGI) rates of 113.0% and 76.5%, respectively; there was no animal death or significant decrease in animal body weight in the treatment group, and no significant drug-related toxic reaction was observed, indicating that the ADC of the present disclosure was well tolerated by mice during the treatment.

### Example 27: Assessment of Toxicokinetics and Tolerability of ADC in Cynomolgus Monkeys

Hab33B10E8-045 (5 mg/mL, injected over 30 min) was intravenously administered to two female cynomolgus monkeys at dosage levels of 10 mg/kg, 30 mg/kg, and 30 mg/kg at intervals of 2 weeks (3 doses in total). Parameters assessed during the study included general observations, body weight, food intake, body temperature, electrocardiogram (lead II), clinical pathology (hematology, blood biochemistry, and coagulation), and macroscopic and microscopic examinations of a large amount of tissue. Toxicokinetic samples were collected at 0 h, 24 h, 72 h, 120 h, 336 h, and 504 h after completion of each dose. The TK samples were analyzed using the MesoScale Discovery (MSD) electrochemiluminescence platform (total monoclonal antibody and total ADC) and LC-MS/MS (free exatecan). The experiment showed that Hab33B10E8-045 was well tolerated, with an MTD of 30 mg/kg. There may be a higher MTD. In the dose investigation, after injection of Hab33B10E8-045 at 10 mg/kg, 30 mg/kg, and 30 mg/kg, no abnormalities were observed during general observations of the cynomolgus monkeys, and no toxicological abnormalities were observed in body weight, body temperature, coagulation, and urinalysis. All changes were reversible before each dose. Hab33B10E8-045 was well tolerated by the cynomolgus monkeys and exhibited a stable pharmacokinetic profile in them.

**Table 29. The toxicokinetics and tolerability of Hab33B10E8-045 in cynomolgus monkeys**

| Test ADC | Clinical observations | Hematology | Blood biochemistry |
|---|---|---|---|
| Hab33B10E8-045 | No abnormalities | WBC; Lymph; LYM; RET%↓; | CK↓; |

## Claims

1. An anti-DLL3 antibody or an antigen-binding fragment thereof, comprising a heavy chain variable region, wherein the heavy chain variable region comprises an HCDR1, an HCDR2, and an HCDR3, wherein:
(1) the sequence of the HCDR1 is the sequence set forth in SEQ ID NO.25 or a sequence having at least 80%, 85%, 90%, 95%, 98%, or 99% identity to the sequence set forth in SEQ ID NO.25, the sequence of the HCDR2 is the sequence set forth in SEQ ID NO.26 or a sequence having at least 80%, 85%, 90%, 95%, 98%, or 99% identity to the sequence set forth in SEQ ID NO.26, and the sequence of the HCDR3 is the sequence set forth in SEQ ID NO.27 or a sequence having at least 80%, 85%, 90%, 95%, 98%, or 99% identity to the sequence set forth in SEQ ID NO.27;
(2) the sequence of the HCDR1 is the sequence set forth in SEQ ID NO.1 or a sequence having at least 80%, 85%, 90%, 95%, 98%, or 99% identity to the sequence set forth in SEQ ID NO.1, the sequence of the HCDR2 is the sequence set forth in SEQ ID NO.2 or a sequence having at least 80%, 85%, 90%, 95%, 98%, or 99% identity to the sequence set forth in SEQ ID NO.2, and the sequence of the HCDR3 is the sequence set forth in SEQ ID NO.3 or a sequence having at least 80%, 85%, 90%, 95%, 98%, or 99% identity to the sequence set forth in SEQ ID NO.3;
(3) the sequence of the HCDR1 is the sequence set forth in SEQ ID NO.9 or a sequence having at least 80%, 85%, 90%, 95%, 98%, or 99% identity to the sequence set forth in SEQ ID NO.9, the sequence of the HCDR2 is the sequence set forth in SEQ ID NO.10 or a sequence having at least 80%, 85%, 90%, 95%, 98%, or 99% identity to the sequence set forth in SEQ ID NO.10, and the sequence of the HCDR3 is the sequence set forth in SEQ ID NO.11 or a sequence having at least 80%, 85%, 90%, 95%, 98%, or 99% identity to the sequence set forth in SEQ ID NO.11;
(4) the sequence of the HCDR1 is the sequence set forth in SEQ ID NO.17 or a sequence having at least 80%, 85%, 90%, 95%, 98%, or 99% identity to the sequence set forth in SEQ ID NO.17, the sequence of the HCDR2 is the sequence set forth in SEQ ID NO.18 or a sequence having at least 80%, 85%, 90%, 95%, 98%, or 99% identity to the sequence set forth in SEQ ID NO.18, and the sequence of the HCDR3 is the sequence set forth in SEQ ID NO.19 or a sequence having at least 80%, 85%, 90%, 95%, 98%, or 99% identity to the sequence set forth in SEQ ID NO.19; or
(5) the sequence of the HCDR1 is the sequence set forth in SEQ ID NO.33 or a sequence having at least 80%, 85%, 90%, 95%, 98%, or 99% identity to the sequence set forth in SEQ ID NO.33, the sequence of the HCDR2 is the sequence set forth in SEQ ID NO.34 or a sequence having at least 80%, 85%, 90%, 95%, 98%, or 99% identity to the sequence set forth in SEQ ID NO.34, and the sequence of the HCDR3 is the sequence set forth in SEQ ID NO.35 or a sequence having at least 80%, 85%, 90%, 95%, 98%, or 99% identity to the sequence set forth in SEQ ID NO.35.

2. An anti-DLL3 antibody or an antigen-binding fragment thereof, comprising a light chain variable region, wherein the light chain variable region comprises an LCDR1, an LCDR2, and an LCDR3, wherein:
(1) the sequence of the LCDR1 is the sequence set forth in SEQ ID NO.28 or a sequence having at least 80%, 85%, 90%, 95%, 98%, or 99% identity to the sequence set forth in SEQ ID NO.28, the sequence of the LCDR2 is the sequence set forth in SEQ ID NO.29 or a sequence having at least 80%, 85%, 90%, 95%, 98%, or 99% identity to the sequence set forth in SEQ ID NO.29, and the sequence of the LCDR3 is the sequence set forth in SEQ ID NO.30 or a sequence having at least 80%, 85%, 90%, 95%, 98%, or 99% identity to the sequence set forth in SEQ ID NO.30;
(2) the sequence of the LCDR1 is the sequence set forth in SEQ ID NO.4 or a sequence having at least 80%, 85%, 90%, 95%, 98%, or 99% identity to the sequence set forth in SEQ ID NO.4, the sequence of the LCDR2 is the sequence set forth in SEQ ID NO.5 or a sequence having at least 80%, 85%, 90%, 95%, 98%, or 99% identity to the sequence set forth in SEQ ID NO.5, and the sequence of the LCDR3 is the sequence set forth in SEQ ID NO.6 or a sequence having at least 80%, 85%, 90%, 95%, 98%, or 99% identity to the sequence set forth in SEQ ID NO.6;
(3) the sequence of the LCDR1 is the sequence set forth in SEQ ID NO.12 or a sequence having at least 80%, 85%, 90%, 95%, 98%, or 99% identity to the sequence set forth in SEQ ID NO.12, the sequence of the LCDR2 is the sequence set forth in SEQ ID NO.13 or a sequence having at least 80%, 85%, 90%, 95%, 98%, or 99% identity to the sequence set forth in SEQ ID NO.13, and the sequence of the LCDR3 is the sequence set forth in SEQ ID NO.14 or a sequence having at least 80%, 85%, 90%, 95%, 98%, or 99% identity to the sequence set forth in SEQ ID NO.14;
(4) the sequence of the LCDR1 is the sequence set forth in SEQ ID NO.20 or a sequence having at least 80%, 85%, 90%, 95%, 98%, or 99% identity to the sequence set forth in SEQ ID NO.20, the sequence of the LCDR2 is the sequence set forth in SEQ ID NO.21 or a sequence having at least 80%, 85%, 90%, 95%, 98%, or 99% identity to the sequence set forth in SEQ ID NO.21, and the sequence of the LCDR3 is the sequence set forth in SEQ ID NO.22 or a sequence having at least 80%, 85%, 90%, 95%, 98%, or 99% identity to the sequence set forth in SEQ ID NO.22; or
(5) the sequence of the LCDR1 is the sequence set forth in SEQ ID NO.36 or a sequence having at least 80%, 85%, 90%, 95%, 98%, or 99% identity to the sequence set forth in SEQ ID NO.36, the sequence of the LCDR2 is the sequence set forth in SEQ ID NO.37 or a sequence having at least 80%, 85%, 90%, 95%, 98%, or 99% identity to the sequence set forth in SEQ ID NO.37, and the sequence of the LCDR3 is the sequence set forth in SEQ ID NO.38 or a sequence having at least 80%, 85%, 90%, 95%, 98%, or 99% identity to the sequence set forth in SEQ ID NO.38.

3. An anti-DLL3 antibody or an antigen-binding fragment thereof, comprising a heavy chain variable region and a light chain variable region, wherein the heavy chain variable region comprises an HCDR1, an HCDR2, and an HCDR3, and the light chain variable region comprises an LCDR1, an LCDR2, and an LCDR3, wherein:
(1) the sequence of the HCDR1 is the sequence set forth in SEQ ID NO.25 or a sequence having at least 80%, 85%, 90%, 95%, 98%, or 99% identity to the sequence set forth in SEQ ID NO.25, the sequence of the HCDR2 is the sequence set forth in SEQ ID NO.26 or a sequence having at least 80%, 85%, 90%, 95%, 98%, or 99% identity to the sequence set forth in SEQ ID NO.26, the sequence of the HCDR3 is the sequence set forth in SEQ ID NO.27 or a sequence having at least 80%, 85%, 90%, 95%, 98%, or 99% identity to the sequence set forth in SEQ ID NO.27, the sequence of the LCDR1 is the sequence set forth in SEQ ID NO.28 or a sequence having at least 80%, 85%, 90%, 95%, 98%, or 99% identity to the sequence set forth in SEQ ID NO.28, the sequence of the LCDR2 is the sequence set forth in SEQ ID NO.29 or a sequence having at least 80%, 85%, 90%, 95%, 98%, or 99% identity to the sequence set forth in SEQ ID NO.29, and the sequence of the LCDR3 is the sequence set forth in SEQ ID NO.30 or a sequence having at least 80%, 85%, 90%, 95%, 98%, or 99% identity to the sequence set forth in SEQ ID NO.30;
(2) the sequence of the HCDR1 is the sequence set forth in SEQ ID NO.1 or a sequence having at least 80%, 85%, 90%, 95%, 98%, or 99% identity to the sequence set forth in SEQ ID NO.1, the sequence of the HCDR2 is the sequence set forth in SEQ ID NO.2 or a sequence having at least 80%, 85%, 90%, 95%, 98%, or 99% identity to the sequence set forth in SEQ ID NO.2, the sequence of the HCDR3 is the sequence set forth in SEQ ID NO.3 or a sequence having at least 80%, 85%, 90%, 95%, 98%, or 99% identity to the sequence set forth in SEQ ID NO.3, the sequence of the LCDR1 is the sequence set forth in SEQ ID NO.4 or a sequence having at least 80%, 85%, 90%, 95%, 98%, or 99% identity to the sequence set forth in SEQ ID NO.4, the sequence of the LCDR2 is the sequence set forth in SEQ ID NO.5 or a sequence having at least 80%, 85%, 90%, 95%, 98%, or 99% identity to the sequence set forth in SEQ ID NO.5, and the sequence of the LCDR3 is the sequence set forth in SEQ ID NO.6 or a sequence having at least 80%, 85%, 90%, 95%, 98%, or 99% identity to the sequence set forth in SEQ ID NO.6;
(3) the sequence of the HCDR1 is the sequence set forth in SEQ ID NO.9 or a sequence having at least 80%, 85%, 90%, 95%, 98%, or 99% identity to the sequence set forth in SEQ ID NO.9, the sequence of the HCDR2 is the sequence set forth in SEQ ID NO.10 or a sequence having at least 80%, 85%, 90%, 95%, 98%, or 99% identity to the sequence set forth in SEQ ID NO.10, the sequence of the HCDR3 is the sequence set forth in SEQ ID NO.11 or a sequence having at least 80%, 85%, 90%, 95%, 98%, or 99% identity to the sequence set forth in SEQ ID NO.11, the sequence of the LCDR1 is the sequence set forth in SEQ ID NO.12 or a sequence having at least 80%, 85%, 90%, 95%, 98%, or 99% identity to the sequence set forth in SEQ ID NO.12, the sequence of the LCDR2 is the sequence set forth in SEQ ID NO.13 or a sequence having at least 80%, 85%, 90%, 95%, 98%, or 99% identity to the sequence set forth in SEQ ID NO.13, and the sequence of the LCDR3 is the sequence set forth in SEQ ID NO.14 or a sequence having at least 80%, 85%, 90%, 95%, 98%, or 99% identity to the sequence set forth in SEQ ID NO.14;
(4) the sequence of the HCDR1 is the sequence set forth in SEQ ID NO.17 or a sequence having at least 80%, 85%, 90%, 95%, 98%, or 99% identity to the sequence set forth in SEQ ID NO.17, the sequence of the HCDR2 is the sequence set forth in SEQ ID NO.18 or a sequence having at least 80%, 85%, 90%, 95%, 98%, or 99% identity to the sequence set forth in SEQ ID NO.18, the sequence of the HCDR3 is the sequence set forth in SEQ ID NO.19 or a sequence having at least 80%, 85%, 90%, 95%, 98%, or 99% identity to the sequence set forth in SEQ ID NO.19, the sequence of the LCDR1 is the sequence set forth in SEQ ID NO.20 or a sequence having at least 80%, 85%, 90%, 95%, 98%, or 99% identity to the sequence set forth in SEQ ID NO.20, the sequence of the LCDR2 is the sequence set forth in SEQ ID NO.21 or a sequence having at least 80%, 85%, 90%, 95%, 98%, or 99% identity to the sequence set forth in SEQ ID NO.21, and the sequence of the LCDR3 is the sequence set forth in SEQ ID NO.22 or a sequence having at least 80%, 85%, 90%, 95%, 98%, or 99% identity to the sequence set forth in SEQ ID NO.22; or
(5) the sequence of the HCDR1 is the sequence set forth in SEQ ID NO.33 or a sequence having at least 80%, 85%, 90%, 95%, 98%, or 99% identity to the sequence set forth in SEQ ID NO.33, the sequence of the HCDR2 is the sequence set forth in SEQ ID NO.34 or a sequence having at least 80%, 85%, 90%, 95%, 98%, or 99% identity to the sequence set forth in SEQ ID NO.34, the sequence of the HCDR3 is the sequence set forth in SEQ ID NO.35 or a sequence having at least 80%, 85%, 90%, 95%, 98%, or 99% identity to the sequence set forth in SEQ ID NO.35, the sequence of the LCDR1 is the sequence set forth in SEQ ID NO.36 or a sequence having at least 80%, 85%, 90%, 95%, 98%, or 99% identity to the sequence set forth in SEQ ID NO.36, the sequence of the LCDR2 is the sequence set forth in SEQ ID NO.37 or a sequence having at least 80%, 85%, 90%, 95%, 98%, or 99% identity to the sequence set forth in SEQ ID NO.37, and the sequence of the LCDR3 is the sequence set forth in SEQ ID NO.38 or a sequence having at least 80%, 85%, 90%, 95%, 98%, or 99% identity to the sequence set forth in SEQ ID NO.38.

4. The anti-DLL3 antibody or the antigen-binding fragment thereof according to any one of claims 1-3, wherein the anti-DLL3 antibody or the antigen-binding fragment thereof is a murine, chimeric, humanized, or fully human antibody or antigen-binding fragment; preferably, the anti-DLL3 antibody or the antigen-binding fragment thereof is a humanized antibody or antigen-binding fragment.

5. The anti-DLL3 antibody or the antigen-binding fragment thereof according to any one of claims 1-4, wherein the anti-DLL3 antibody or the antigen-binding fragment thereof comprises a heavy chain variable region, wherein:
(1) the sequence of the heavy chain variable region is the sequence set forth in SEQ ID NO.31 or a sequence having at least 80%, 85%, 90%, 95%, 98%, or 99% identity to the sequence set forth in SEQ ID NO.31, or the sequence of the heavy chain variable region is the sequence set forth in SEQ ID NO.58 or a sequence having at least 80%, 85%, 90%, 95%, 98%, or 99% identity to the sequence set forth in SEQ ID NO.58;
(2) the sequence of the heavy chain variable region is the sequence set forth in SEQ ID NO.7 or a sequence having at least 80%, 85%, 90%, 95%, 98%, or 99% identity to the sequence set forth in SEQ ID NO.7, or the sequence of the heavy chain variable region is the sequence set forth in SEQ ID NO.54 or a sequence having at least 80%, 85%, 90%, 95%, 98%, or 99% identity to the sequence set forth in SEQ ID NO.54;
(3) the sequence of the heavy chain variable region is the sequence set forth in SEQ ID NO.15 or a sequence having at least 80%, 85%, 90%, 95%, 98%, or 99% identity to the sequence set forth in SEQ ID NO.15;
(4) the sequence of the heavy chain variable region is the sequence set forth in SEQ ID NO.23 or a sequence having at least 80%, 85%, 90%, 95%, 98%, or 99% identity to the sequence set forth in SEQ ID NO.23, or the sequence of the heavy chain variable region is the sequence set forth in SEQ ID NO.56 or a sequence having at least 80%, 85%, 90%, 95%, 98%, or 99% identity to the sequence set forth in SEQ ID NO.56; or
(5) the sequence of the heavy chain variable region is the sequence set forth in SEQ ID NO.39 or a sequence having at least 80%, 85%, 90%, 95%, 98%, or 99% identity to the sequence set forth in SEQ ID NO.39.

6. The anti-DLL3 antibody or the antigen-binding fragment thereof according to any one of claims 1-4, wherein the anti-DLL3 antibody or the antigen-binding fragment thereof comprises a light chain variable region, wherein:
(1) the sequence of the light chain variable region is the sequence set forth in SEQ ID NO.32 or a sequence having at least 80%, 85%, 90%, 95%, 98%, or 99% identity to the sequence set forth in SEQ ID NO.32, or the sequence of the light chain variable region is the sequence set forth in SEQ ID NO.59 or a sequence having at least 80%, 85%, 90%, 95%, 98%, or 99% identity to the sequence set forth in SEQ ID NO.59;
(2) the sequence of the light chain variable region is the sequence set forth in SEQ ID NO.8 or a sequence having at least 80%, 85%, 90%, 95%, 98%, or 99% identity to the sequence set forth in SEQ ID NO.8, or the sequence of the light chain variable region is the sequence set forth in SEQ ID NO.55 or a sequence having at least 80%, 85%, 90%, 95%, 98%, or 99% identity to the sequence set forth in SEQ ID NO.55;
(3) the sequence of the light chain variable region is the sequence set forth in SEQ ID NO.16 or a sequence having at least 80%, 85%, 90%, 95%, 98%, or 99% identity to the sequence set forth in SEQ ID NO.16;
(4) the sequence of the light chain variable region is the sequence set forth in SEQ ID NO.24 or a sequence having at least 80%, 85%, 90%, 95%, 98%, or 99% identity to the sequence set forth in SEQ ID NO.24, or the sequence of the light chain variable region is the sequence set forth in SEQ ID NO.57 or a sequence having at least 80%, 85%, 90%, 95%, 98%, or 99% identity to the sequence set forth in SEQ ID NO.57;
(5) the sequence of the light chain variable region is the sequence set forth in SEQ ID NO.40 or a sequence having at least 80%, 85%, 90%, 95%, 98%, or 99% identity to the sequence set forth in SEQ ID NO.40.

7. The anti-DLL3 antibody or the antigen-binding fragment thereof according to any one of claims 1-4, wherein the anti-DLL3 antibody or the antigen-binding fragment thereof comprises a heavy chain variable region and a light chain variable region, wherein:
(1) the sequence of the heavy chain variable region is the sequence set forth in SEQ ID NO.31 or a sequence having at least 80%, 85%, 90%, 95%, 98%, or 99% identity to the sequence set forth in SEQ ID NO.31, and the sequence of the light chain variable region is the sequence set forth in SEQ ID NO.32 or a sequence having at least 80%, 85%, 90%, 95%, 98%, or 99% identity to the sequence set forth in SEQ ID NO.32; or the sequence of the heavy chain variable region is the sequence set forth in SEQ ID NO.58 or a sequence having at least 80%, 85%, 90%, 95%, 98%, or 99% identity to the sequence set forth in SEQ ID NO.58, and the sequence of the light chain variable region is the sequence set forth in SEQ ID NO.59 or a sequence having at least 80%, 85%, 90%, 95%, 98%, or 99% identity to the sequence set forth in SEQ ID NO.59;
(2) the sequence of the heavy chain variable region is the sequence set forth in SEQ ID NO.7 or a sequence having at least 80%, 85%, 90%, 95%, 98%, or 99% identity to the sequence set forth in SEQ ID NO.7, and the sequence of the light chain variable region is the sequence set forth in SEQ ID NO.8 or a sequence having at least 80%, 85%, 90%, 95%, 98%, or 99% identity to the sequence set forth in SEQ ID NO.8; or the sequence of the heavy chain variable region is the sequence set forth in SEQ ID NO.54 or a sequence having at least 80%, 85%, 90%, 95%, 98%, or 99% identity to the sequence set forth in SEQ ID NO.54, and the sequence of the light chain variable region is the sequence set forth in SEQ ID NO.55 or a sequence having at least 80%, 85%, 90%, 95%, 98%, or 99% identity to the sequence set forth in SEQ ID NO.55;
(3) the sequence of the heavy chain variable region is the sequence set forth in SEQ ID NO.15 or a sequence having at least 80%, 85%, 90%, 95%, 98%, or 99% identity to the sequence set forth in SEQ ID NO.15, and the sequence of the light chain variable region is the sequence set forth in SEQ ID NO.16 or a sequence having at least 80%, 85%, 90%, 95%, 98%, or 99% identity to the sequence set forth in SEQ ID NO.16;
(4) the sequence of the heavy chain variable region is the sequence set forth in SEQ ID NO.23 or a sequence having at least 80%, 85%, 90%, 95%, 98%, or 99% identity to the sequence set forth in SEQ ID NO.23, and the sequence of the light chain variable region is the sequence set forth in SEQ ID NO.24 or a sequence having at least 80%, 85%, 90%, 95%, 98%, or 99% identity to the sequence set forth in SEQ ID NO.24; or the sequence of the heavy chain variable region is the sequence set forth in SEQ ID NO.56 or a sequence having at least 80%, 85%, 90%, 95%, 98%, or 99% identity to the sequence set forth in SEQ ID NO.56, and the sequence of the light chain variable region is the sequence set forth in SEQ ID NO.57 or a sequence having at least 80%, 85%, 90%, 95%, 98%, or 99% identity to the sequence set forth in SEQ ID NO.57;
(5) the sequence of the heavy chain variable region is the sequence set forth in SEQ ID NO.39 or a sequence having at least 80%, 85%, 90%, 95%, 98%, or 99% identity to the sequence set forth in SEQ ID NO.39, and the sequence of the light chain variable region is the sequence set forth in SEQ ID NO.40 or a sequence having at least 80%, 85%, 90%, 95%, 98%, or 99% identity to the sequence set forth in SEQ ID NO.40.

8. The anti-DLL3 antibody or the antigen-binding fragment thereof according to any one of claims 1-7, wherein the antigen-binding fragment is an Fv, VHH, scFv, Fab, Fab', or (Fab')₂.

9. The anti-DLL3 antibody or the antigen-binding fragment thereof according to any one of claims 1-7, wherein the anti-DLL3 antibody is a full-length antibody.

10. The anti-DLL3 antibody or the antigen-binding fragment thereof according to claim 9, wherein the anti-DLL3 antibody is an IgG antibody; preferably, the anti-DLL3 antibody is an IgG1 antibody.

11. The anti-DLL3 antibody or the antigen-binding fragment thereof according to claim 10, wherein an Fc region of the anti-DLL3 antibody comprises an amino acid mutation that reduces ADCC, CDC, and/or ADCP effects.

12. The anti-DLL3 antibody or the antigen-binding fragment thereof according to claim 11, wherein the amino acid mutation is one or more amino acid mutations selected from the group consisting of the following mutations: L234A, L235A, P329A, and P331S.

13. A nucleic acid molecule encoding the anti-DLL3 antibody or the antigen-binding fragment thereof according to any one of claims 1-12.

14. An expression vector, comprising the nucleic acid molecule according to claim 13.

15. A host cell, comprising the expression vector according to claim 14.

16. A bispecific antibody, comprising a first antigen-binding site and a second antigen-binding site, wherein one of the antigen-binding sites comprises the anti-DLL3 antibody or the antigen-binding fragment thereof according to any one of claims 1-12.

17. A bispecific antibody, comprising a first antigen-binding site and a second antigen-binding site, wherein one of the antigen-binding sites comprises the anti-DLL3 antibody or the antigen-binding fragment thereof according to any one of claims 1-12, and the other antigen-binding site comprises another anti-DLL3 antibody or an antigen-binding fragment thereof according to any one of claims 1-12.

18. The bispecific antibody according to claim 17, wherein the first antigen-binding site and the second antigen-binding site bind to different epitopes of the same antigen.

19. The bispecific antibody according to claim 18, wherein:
(1) the first antigen-binding site comprises a heavy chain variable region and a light chain variable region, wherein the heavy chain variable region comprises an HCDR1 whose sequence is the sequence set forth in SEQ ID NO.25 or a sequence having 85%, 90%, 95%, 98%, or 99% identity to the sequence set forth in SEQ ID NO.25, an HCDR2 whose sequence is the sequence set forth in SEQ ID NO.26 or a sequence having 85%, 90%, 95%, 98%, or 99% identity to the sequence set forth in SEQ ID NO.26, and an HCDR3 whose sequence is the sequence set forth in SEQ ID NO.27 or a sequence having 85%, 90%, 95%, 98%, or 99% identity to the sequence set forth in SEQ ID NO.27, and the light chain variable region comprises an LCDR1 whose sequence is the sequence set forth in SEQ ID NO.28 or a sequence having 85%, 90%, 95%, 98%, or 99% identity to the sequence set forth in SEQ ID NO.28, an LCDR2 whose sequence is the sequence set forth in SEQ ID NO.29 or a sequence having 85%, 90%, 95%, 98%, or 99% identity to the sequence set forth in SEQ ID NO.29, and an LCDR3 whose sequence is the sequence set forth in SEQ ID NO.30 or a sequence having 85%, 90%, 95%, 98%, or 99% identity to the sequence set forth in SEQ ID NO.30; the second antigen-binding site comprises a heavy chain variable region and a light chain variable region, wherein the heavy chain variable region comprises an HCDR1 whose sequence is the sequence set forth in SEQ ID NO.17 or a sequence having 85%, 90%, 95%, 98%, or 99% identity to the sequence set forth in SEQ ID NO.17, an HCDR2 whose sequence is the sequence set forth in SEQ ID NO.18 or a sequence having 85%, 90%, 95%, 98%, or 99% identity to the sequence set forth in SEQ ID NO.18, and an HCDR3 whose sequence is the sequence set forth in SEQ ID NO.19 or a sequence having 85%, 90%, 95%, 98%, or 99% identity to the sequence set forth in SEQ ID NO.19, and the light chain variable region comprises an LCDR1 whose sequence is the sequence set forth in SEQ ID NO.20 or a sequence having 85%, 90%, 95%, 98%, or 99% identity to the sequence set forth in SEQ ID NO.20, an LCDR2 whose sequence is the sequence set forth in SEQ ID NO.21 or a sequence having 85%, 90%, 95%, 98%, or 99% identity to the sequence set forth in SEQ ID NO.21, and an LCDR3 whose sequence is the sequence set forth in SEQ ID NO.22 or a sequence having 85%, 90%, 95%, 98%, or 99% identity to the sequence set forth in SEQ ID NO.22;
(2) the first antigen-binding site comprises a heavy chain variable region and a light chain variable region, wherein the heavy chain variable region comprises an HCDR1 whose sequence is the sequence set forth in SEQ ID NO.25 or a sequence having 85%, 90%, 95%, 98%, or 99% identity to the sequence set forth in SEQ ID NO.25, an HCDR2 whose sequence is the sequence set forth in SEQ ID NO.26 or a sequence having 85%, 90%, 95%, 98%, or 99% identity to the sequence set forth in SEQ ID NO.26, and an HCDR3 whose sequence is the sequence set forth in SEQ ID NO.27 or a sequence having 85%, 90%, 95%, 98%, or 99% identity to the sequence set forth in SEQ ID NO.27, and the light chain variable region comprises an LCDR1 whose sequence is the sequence set forth in SEQ ID NO.28 or a sequence having 85%, 90%, 95%, 98%, or 99% identity to the sequence set forth in SEQ ID NO.28, an LCDR2 whose sequence is the sequence set forth in SEQ ID NO.29 or a sequence having 85%, 90%, 95%, 98%, or 99% identity to the sequence set forth in SEQ ID NO.29, and an LCDR3 whose sequence is the sequence set forth in SEQ ID NO.30 or a sequence having 85%, 90%, 95%, 98%, or 99% identity to the sequence set forth in SEQ ID NO.30; the second antigen-binding site comprises a heavy chain variable region and a light chain variable region, wherein the heavy chain variable region comprises an HCDR1 whose sequence is the sequence set forth in SEQ ID NO.1 or a sequence having 85%, 90%, 95%, 98%, or 99% identity to the sequence set forth in SEQ ID NO.1, an HCDR2 whose sequence is the sequence set forth in SEQ ID NO.2 or a sequence having 85%, 90%, 95%, 98%, or 99% identity to the sequence set forth in SEQ ID NO.2, and an HCDR3 whose sequence is the sequence set forth in SEQ ID NO.3 or a sequence having 85%, 90%, 95%, 98%, or 99% identity to the sequence set forth in SEQ ID NO.3, and the light chain variable region comprises an LCDR1 whose sequence is the sequence set forth in SEQ ID NO.4 or a sequence having 85%, 90%, 95%, 98%, or 99% identity to the sequence set forth in SEQ ID NO.4, an LCDR2 whose sequence is the sequence set forth in SEQ ID NO.5 or a sequence having 85%, 90%, 95%, 98%, or 99% identity to the sequence set forth in SEQ ID NO.5, and an LCDR3 whose sequence is the sequence set forth in SEQ ID NO.6 or a sequence having 85%, 90%, 95%, 98%, or 99% identity to the sequence set forth in SEQ ID NO.6; or
(3) the first antigen-binding site comprises a heavy chain variable region and a light chain variable region, wherein the heavy chain variable region comprises an HCDR1 whose sequence is the sequence set forth in SEQ ID NO.17 or a sequence having 85%, 90%, 95%, 98%, or 99% identity to the sequence set forth in SEQ ID NO.17, an HCDR2 whose sequence is the sequence set forth in SEQ ID NO.18 or a sequence having 85%, 90%, 95%, 98%, or 99% identity to the sequence set forth in SEQ ID NO.18, and an HCDR3 whose sequence is the sequence set forth in SEQ ID NO.19 or a sequence having 85%, 90%, 95%, 98%, or 99% identity to the sequence set forth in SEQ ID NO.19, and the light chain variable region comprises an LCDR1 whose sequence is the sequence set forth in SEQ ID NO.20 or a sequence having 85%, 90%, 95%, 98%, or 99% identity to the sequence set forth in SEQ ID NO.20, an LCDR2 whose sequence is the sequence set forth in SEQ ID NO.21 or a sequence having 85%, 90%, 95%, 98%, or 99% identity to the sequence set forth in SEQ ID NO.21, and an LCDR3 whose sequence is the sequence set forth in SEQ ID NO.22 or a sequence having 85%, 90%, 95%, 98%, or 99% identity to the sequence set forth in SEQ ID NO.22; the second antigen-binding site comprises a heavy chain variable region and a light chain variable region, wherein the heavy chain variable region comprises an HCDR1 whose sequence is the sequence set forth in SEQ ID NO.1 or a sequence having 85%, 90%, 95%, 98%, or 99% identity to the sequence set forth in SEQ ID NO.1, an HCDR2 whose sequence is the sequence set forth in SEQ ID NO.2 or a sequence having 85%, 90%, 95%, 98%, or 99% identity to the sequence set forth in SEQ ID NO.2, and an HCDR3 whose sequence is the sequence set forth in SEQ ID NO.3 or a sequence having 85%, 90%, 95%, 98%, or 99% identity to the sequence set forth in SEQ ID NO.3, and the light chain variable region comprises an LCDR1 whose sequence is the sequence set forth in SEQ ID NO.4 or a sequence having 85%, 90%, 95%, 98%, or 99% identity to the sequence set forth in SEQ ID NO.4, an LCDR2 whose sequence is the sequence set forth in SEQ ID NO.5 or a sequence having 85%, 90%, 95%, 98%, or 99% identity to the sequence set forth in SEQ ID NO.5, and an LCDR3 whose sequence is the sequence set forth in SEQ ID NO.6 or a sequence having 85%, 90%, 95%, 98%, or 99% identity to the sequence set forth in SEQ ID NO.6.

20. The bispecific antibody according to claim 19, wherein the bispecific antibody is an IgG-like bispecific antibody, wherein a left arm is the first antigen-binding site, and a right arm is the second antigen-binding site.

21. The bispecific antibody according to claim 20, wherein:
(1) the first antigen-binding site comprises a heavy chain variable region and a light chain variable region, wherein the sequence of the heavy chain variable region is the sequence set forth in SEQ ID NO.31 or a sequence having 85%, 90%, 95%, 98%, or 99% identity to the sequence set forth in SEQ ID NO.31, and the sequence of the light chain variable region is the sequence set forth in SEQ ID NO.32 or a sequence having 85%, 90%, 95%, 98%, or 99% identity to the sequence set forth in SEQ ID NO.32; the second antigen-binding site comprises a heavy chain variable region and a light chain variable region, wherein the sequence of the heavy chain variable region is the sequence set forth in SEQ ID NO.23 or a sequence having 85%, 90%, 95%, 98%, or 99% identity to the sequence set forth in SEQ ID NO.23, and the sequence of the light chain variable region is the sequence set forth in SEQ ID NO.24 or a sequence having 85%, 90%, 95%, 98%, or 99% identity to the sequence set forth in SEQ ID NO.24;
(2) the first antigen-binding site comprises a heavy chain variable region and a light chain variable region, wherein the sequence of the heavy chain variable region is the sequence set forth in SEQ ID NO.31 or a sequence having 85%, 90%, 95%, 98%, or 99% identity to the sequence set forth in SEQ ID NO.31, and the sequence of the light chain variable region is the sequence set forth in SEQ ID NO.32 or a sequence having 85%, 90%, 95%, 98%, or 99% identity to the sequence set forth in SEQ ID NO.32; the second antigen-binding site comprises a heavy chain variable region and a light chain variable region, wherein the sequence of the heavy chain variable region is the sequence set forth in SEQ ID NO.7 or a sequence having 85%, 90%, 95%, 98%, or 99% identity to the sequence set forth in SEQ ID NO.7, and the sequence of the light chain variable region is the sequence set forth in SEQ ID NO.8 or a sequence having 85%, 90%, 95%, 98%, or 99% identity to the sequence set forth in SEQ ID NO.8;
(3) the first antigen-binding site comprises a heavy chain variable region and a light chain variable region, wherein the sequence of the heavy chain variable region is the sequence set forth in SEQ ID NO.23 or a sequence having 85%, 90%, 95%, 98%, or 99% identity to the sequence set forth in SEQ ID NO.23, and the sequence of the light chain variable region is the sequence set forth in SEQ ID NO.24 or a sequence having 85%, 90%, 95%, 98%, or 99% identity to the sequence set forth in SEQ ID NO.24; the second antigen-binding site comprises a heavy chain variable region and a light chain variable region, wherein the sequence of the heavy chain variable region is the sequence set forth in SEQ ID NO.7 or a sequence having 85%, 90%, 95%, 98%, or 99% identity to the sequence set forth in SEQ ID NO.7, and the sequence of the light chain variable region is the sequence set forth in SEQ ID NO.8 or a sequence having 85%, 90%, 95%, 98%, or 99% identity to the sequence set forth in SEQ ID NO.8.

22. The bispecific antibody according to any one of claims 20-21, wherein the bispecific antibody is an IgG1 antibody; preferably, an Fc region of the bispecific antibody comprises an amino acid mutation that reduces ADCC, CDC, and/or ADCP effects, and/or comprises knob- and/or hole-forming amino acid mutations that facilitate purification of the bispecific antibody; preferably, the bispecific antibody carries a CH1-CL chain exchange between a first heavy chain and a first light chain or between a second heavy chain and a second light chain; preferably, the Fc region of the bispecific antibody comprises, in a CH3 region, knob- and/or hole-forming amino acid mutations that facilitate purification of the bispecific antibody; further preferably, the hole-forming mutations are Y349C, T366S, L368A, and Y407V, and the knob-forming mutations are S354C and T366W.

23. The bispecific antibody according to claim 22, wherein:
(1) the bispecific antibody comprises 2 heavy chains and 2 light chains, wherein the amino acid sequence of a heavy chain 1 is set forth in SEQ ID NO.41, the amino acid sequence of a heavy chain 2 is set forth in SEQ ID NO.43, the amino acid sequence of a light chain 1 is set forth in SEQ ID NO.46, and the amino acid sequence of a light chain 2 is set forth in SEQ ID NO.48;
(2) the bispecific antibody comprises 2 heavy chains and 2 light chains, wherein the amino acid sequence of a heavy chain 1 is set forth in SEQ ID NO.41, the amino acid sequence of a heavy chain 2 is set forth in SEQ ID NO.50, the amino acid sequence of a light chain 1 is set forth in SEQ ID NO.46, and the amino acid sequence of a light chain 2 is set forth in SEQ ID NO.51;
(3) the bispecific antibody comprises 2 heavy chains and 2 light chains, wherein the amino acid sequence of a heavy chain 1 is set forth in SEQ ID NO.52, the amino acid sequence of a heavy chain 2 is set forth in SEQ ID NO.50, the amino acid sequence of a light chain 1 is set forth in SEQ ID NO.53, and the amino acid sequence of a light chain 2 is set forth in SEQ ID NO.51.

24. A nucleic acid molecule encoding the bispecific antibody according to any one of claims 16-23.

25. An expression vector, comprising the nucleic acid molecule according to claim 24.

26. A host cell, comprising the expression vector according to claim 25.

27. An antibody-drug conjugate, comprising the anti-DLL3 antibody or the antigen-binding fragment thereof according to any one of claims 1-12 or the bispecific antibody according to any one of claims 16-23.

28. The antibody-drug conjugate according to claim 27, wherein the antibody-drug conjugate is obtained by conjugating a therapeutic agent to the anti-DLL3 antibody or the antigen-binding fragment thereof according to any one of claims 1-12 or the bispecific antibody according to any one of claims 16-23.

29. The antibody-drug conjugate according to claim 28, wherein the therapeutic agent is selected from the group consisting of a cytotoxic drug, an immunopotentiator, and a radioisotope; preferably, the therapeutic agent is a cytotoxic drug selected from a tubulin inhibitor, a DNA topoisomerase inhibitor, a DNA-damaging agent, an antimetabolite, and an anti-tumor antibiotic; more preferably, the therapeutic agent is a DNA topoisomerase inhibitor selected from DXD, SN38, irinotecan, irinotecan hydrochloride, camptothecin, 9-aminocamptothecin, 9-nitrocamptothecin, 10-hydroxycamptothecin, 9-chloro-10-hydroxycamptothecin, 22-hydroxyacuminatine, topotecan, lurtotecan, belotecan, exatecan, homosilatecan, 6,8-dibromo-2-methyl-3-[2-(D-xylopyranosylamino)phenyl]-4(3H)-quinazolinone, 2-cyano-3-(3,4-dihydroxyphenyl)-N-(phenylmethyl)-(2E)-2-propenamide, 2-cyano-3-(3,4-dihydroxyphenyl)-N-(3-hydroxyphenylpropyl)-(E)-2-propenamide, 12-β-D-glucopyranosyl-12,13-dihydro-2,10-dihydroxy-6-[[2-hydroxy-1-(hydroxymethyl)ethyl]amino]-5H-indolo[2,3-a]pyrrolo[3,4-c]carbazole-5,7(6H)-dione, N-[2-(dimethylamino)ethyl]-4-acridinecarboxamide dihydrochloride, and N-[2-(dimethylamino)ethyl]-4-acridinecarboxamide.

30. The antibody-drug conjugate according to claim 29, wherein the antibody or the antigen-binding fragment thereof is conjugated to the therapeutic agent by a linker, and the linker may be a cleavable linker or a non-cleavable linker.

31. The antibody-drug conjugate according to claim 27, having a structure represented by formula (I):
A-(L-D)_{d} (I)
wherein A is the anti-DLL3 antibody or the antigen-binding fragment thereof according to any one of claims 1-12 or the bispecific antibody according to any one of claims 16-23;
L is a linker moiety with one end linked to A and one end linked to a therapeutic agent D;
d represents an average molar ratio of the therapeutic agent to A (also known as DAR, i.e., drug-to-antibody ratio), and is selected from an integer and a decimal from 1 to 12 (e.g., 1, 2, 3, 4, 5, 6, 7, 8, 9, 10, 11, or 12); when d is a decimal, d refers to an average number of linker-therapeutic agent (L-D) compounds conjugated to each A.

32. The antibody-drug conjugate according to claim 29, wherein A is selected from antibody 22B12E5, antibody 25G10A9, antibody 31B6G8, antibody 33B10E8, antibody 44B8H2, Hab22B12E5, Hab31B6G8, Hab33B10E8, 33+31, 33+22, and 31+22, wherein:
antibody 22B12E5 comprises a heavy chain variable region whose sequence is SEQ ID NO.7 and a light chain variable region whose sequence is SEQ ID NO.8;
antibody 25G10A9 comprises a heavy chain variable region whose sequence is SEQ ID NO.15 and a light chain variable region whose sequence is SEQ ID NO.16;
antibody 31B6G8 comprises a heavy chain variable region whose sequence is SEQ ID NO.23 and a light chain variable region whose sequence is SEQ ID NO.24;
antibody 33B10E8 comprises a heavy chain variable region whose sequence is SEQ ID NO.31 and a light chain variable region whose sequence is SEQ ID NO.32;
antibody 44B8H2 comprises a heavy chain variable region whose sequence is SEQ ID NO.39 and a light chain variable region whose sequence is SEQ ID NO.40;
antibody Hab22B12E5 comprises a heavy chain variable region whose sequence is SEQ ID NO.54 and a light chain variable region whose sequence is SEQ ID NO.55;
antibody Hab31B6G8 comprises a heavy chain variable region whose sequence is SEQ ID NO.56 and a light chain variable region whose sequence is SEQ ID NO.57;
antibody Hab33B10E8 comprises a heavy chain variable region whose sequence is SEQ ID NO.58 and a light chain variable region whose sequence is SEQ ID NO.59;
antibody 33+31 comprises a heavy chain 1 of SEQ ID NO.41, a heavy chain 2 of SEQ ID NO.43, a light chain 1 of SEQ ID NO.46, and a light chain 2 of SEQ ID NO.48;
antibody 33+22 comprises a heavy chain 1 of SEQ ID NO.41, a heavy chain 2 of SEQ ID NO.50, a light chain 1 of SEQ ID NO.46, and a light chain 2 of SEQ ID NO.51;
antibody 31+22 comprises a heavy chain 1 of SEQ ID NO.52, a heavy chain 2 of SEQ ID NO.50, a light chain 1 of SEQ ID NO.53, and a light chain 2 of SEQ ID NO.51.

33. The antibody-drug conjugate according to any one of claims 31-32, wherein -L- is selected from -L₁-L₂-L₃-L₄-, wherein L₁ is a covalent linking unit covalently linked to A, L₂ is an extension unit, L₃ is selected from a peptide residue consisting of 2-8 amino acids, and L₄ is a bond or a self-cleaving fragment.

34. The antibody-drug conjugate according to claim 33, wherein:
L₁ is selected from 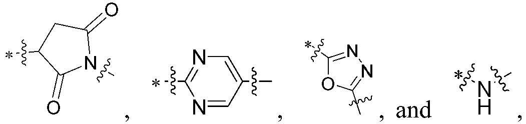 wherein * indicates linking to A;
L₂ is selected from -L₂ₐ-, -L₂ₐ-C(O)-, and -L₂ₐ-NH-C(O)-L_{2b}-C(O)-, wherein L₂ₐ and L_{2b} are each independently selected from -C₁-C₆ alkylene-, -C₁-C₃ alkylene-C₃-C₆ cycloalkylene-, -ethynylene-C₁-C₆ alkylene-, -(CH₂CH₂O)ₙ-, -(CH₂CH₂O)ₙC₁₋₃ alkylene-, -C₁-C₃ alkylene(CH₂CH₂O)ₙ-C₁-C₃ alkylene-, -C₁-C₃ alkylene-O-C₁-C₃ alkylene, phenyl, and -phenyl-C₁-C₃ alkylene-, wherein n is selected from an integer of 1-12 (preferably 3-8, e.g., 1, 2, 3, 4, 5, 6, 7, 8, 9, 10, 11, or 12);
L₃ is selected from a peptide residue consisting of 2-8 amino acids, and the amino acids are selected from phenylalanine, valine, alanine, serine, glutamine, arginine, lysine, asparagine, glutamic acid, proline, citrulline, aspartic acid, and glycine;
L₄ is selected from: a bond, -NCH₂-, and 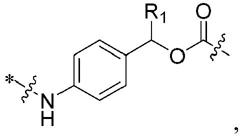 wherein R₁ is selected from hydrogen and 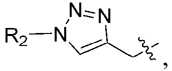
wherein R₂ is selected from: 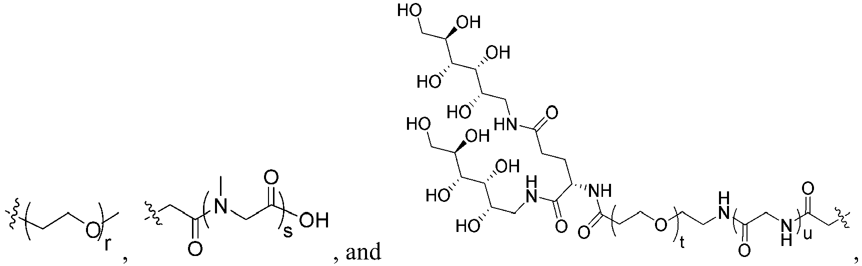
wherein r, s, t, and u are each independently selected from an integer of 1-50 (e.g., 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

35. The antibody-drug conjugate according to any one of claims 33-34, wherein:
L₂ is selected from: 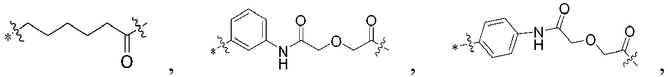 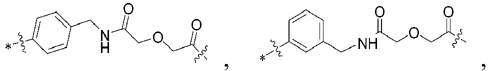 (CH₂CH₂O)ₙCH₂C(O)-, -(CH₂CH₂O)ₙCH₂CH₂-, 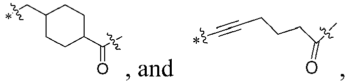 wherein the * position is linked to L₁, and n is as defined in claim 34.

36. The antibody-drug conjugate according to any one of claims 33-35, wherein:
L₃ is selected from: 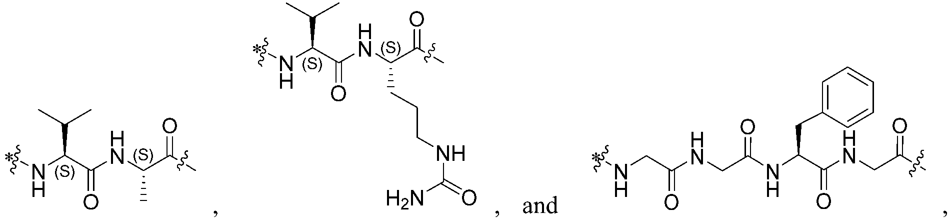 wherein the * position is linked to L₂.

37. The antibody-drug conjugate according to any one of claims 33-36, wherein:
L₄ is selected from:
-NCH₂-, 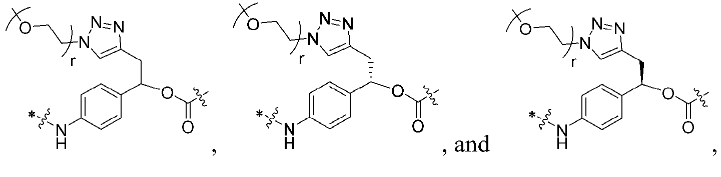
wherein r is selected from an integer of 1-50 (e.g., , , 3, , 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50).

38. The antibody-drug conjugate according to claim 34, wherein L is selected from: 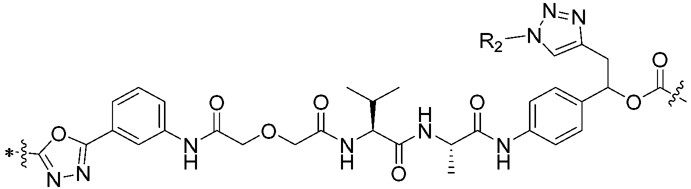 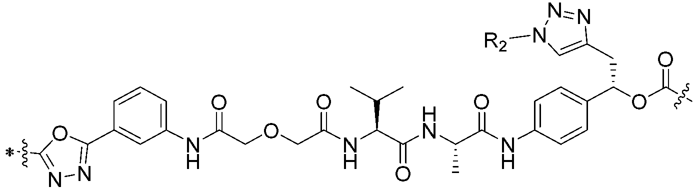 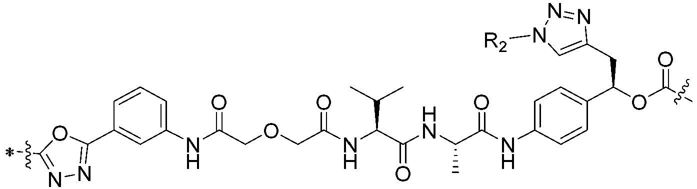 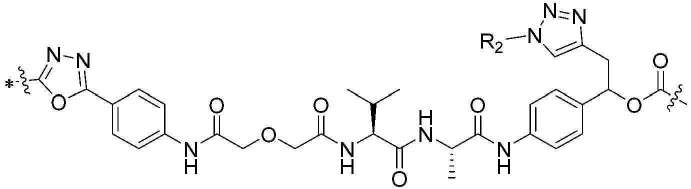 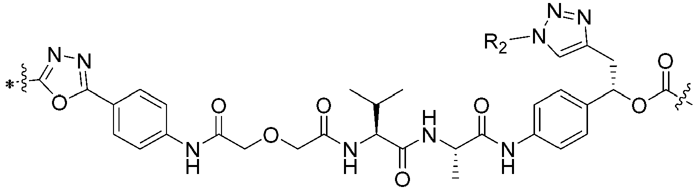 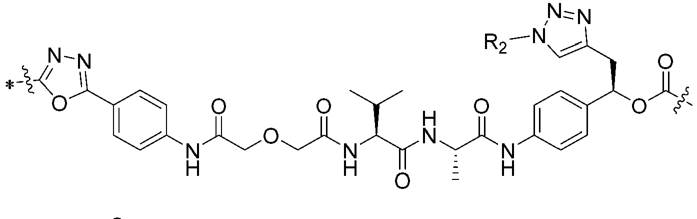 and 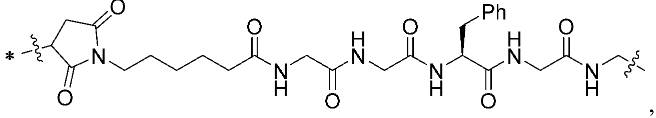 wherein * indicates linking to A.

39. The antibody-drug conjugate according to any one of claims 33-38, wherein D is selected from: 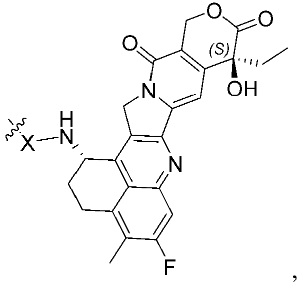 wherein X is selected from a bond and 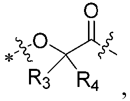 wherein R₃ and R₄ are each independently selected from H, C₁₋₃ alkyl (preferably methyl, ethyl, n-propyl, or isopropyl), and 3-6 membered cycloalkyl (preferably cyclopropyl, cyclobutyl, cyclopentyl, or cyclohexyl), or R₃ and R₄, together with the carbon atom to which they are attached, form 3-6 membered cycloalkyl (preferably cyclopropyl, cyclobutyl, cyclopentyl, or cyclohexyl), wherein the * position is linked to L₄.

40. The antibody-drug conjugate according to any one of claims 33-39, wherein D is selected from:

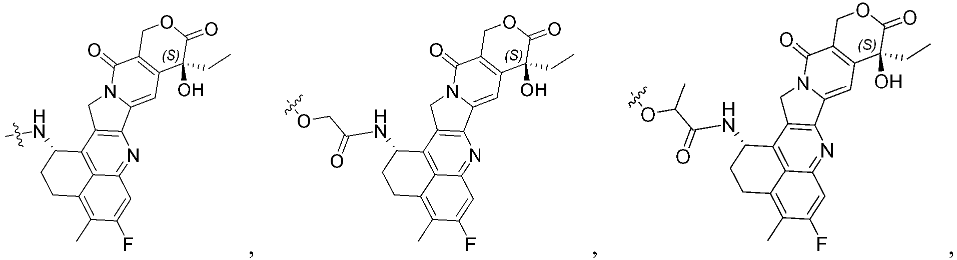

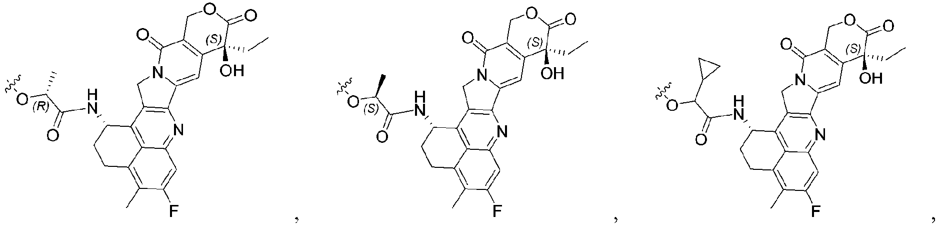

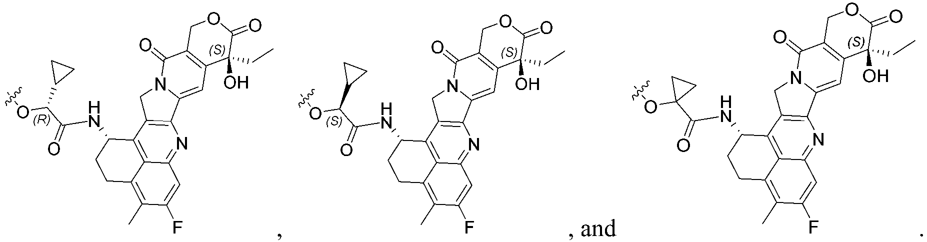

41. The antibody-drug conjugate according to any one of claims 27-40, being selected from the following structure: 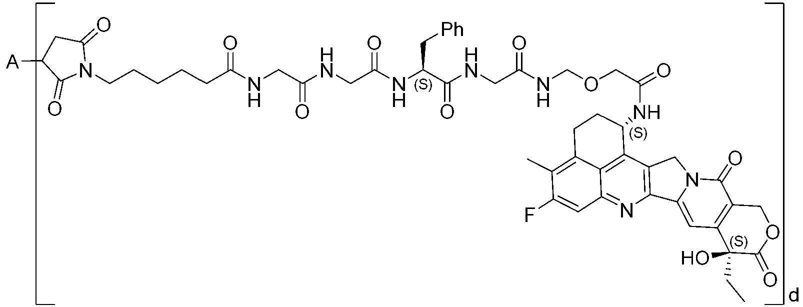 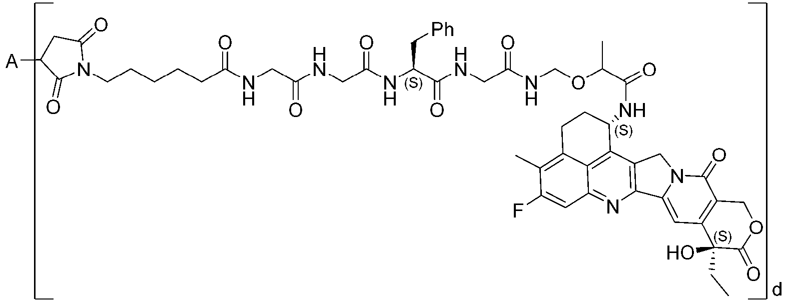 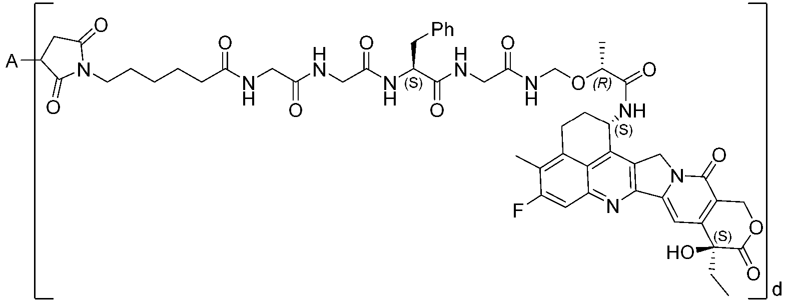 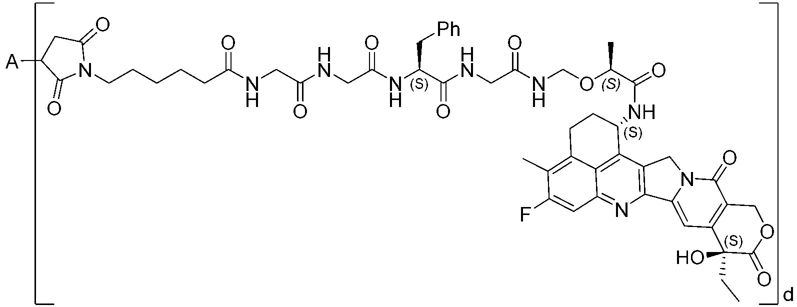 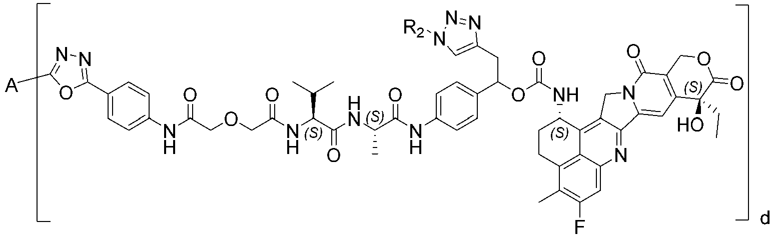 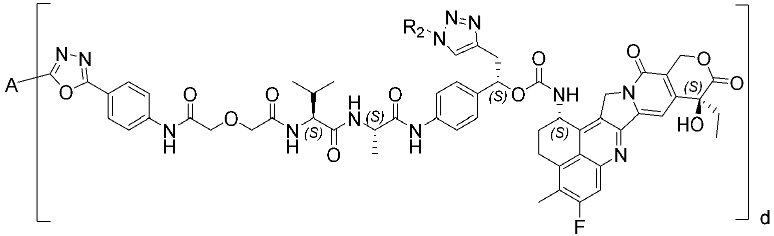 and

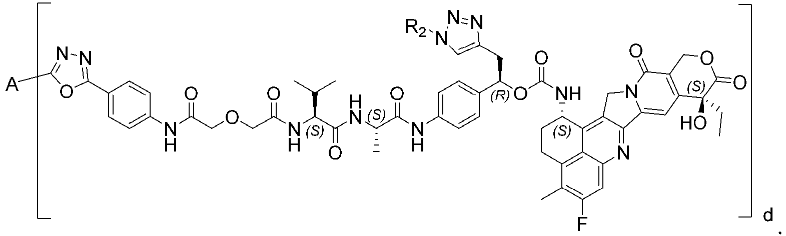

42. A fusion protein, comprising the anti-DLL3 antibody or the antigen-binding fragment thereof according to any one of claims 1-12.

43. A chimeric antigen receptor polypeptide (CAR), comprising the anti-DLL3 antibody or the antigen-binding fragment thereof according to any one of claims 1-12 or the bispecific antibody according to any one of claims 16-23.

44. A genetically modified cell, wherein the cell expresses the chimeric antigen receptor (CAR) polypeptide according to claim 43, and the cell is selected from autologous or allogeneic T cells, B cells, NK cells, macrophages, monocytes, dendritic cells, neutrophils, basophils, eosinophils, mast cells, NK-T cells, MAIT cells, hematopoietic stem cells, embryonic stem cells, induced pluripotent stem cells, and erythrocytes.

45. A pharmaceutical composition, comprising the anti-DLL3 antibody or the antigen-binding fragment thereof according to any one of claims 1-12, the bispecific antibody according to any one of claims 16-23, the antibody-drug conjugate according to any one of claims 27-41, the fusion protein according to claim 42, the chimeric antigen receptor polypeptide according to claim 43 or a nucleic acid molecule expressing same, or the genetically modified cell according to claim 44.

46. Use of the anti-DLL3 antibody or the antigen-binding fragment thereof according to any one of claims 1-12, the bispecific antibody according to any one of claims 16-23, the antibody-drug conjugate according to any one of claims 27-41, the fusion protein according to claim 42, the chimeric antigen receptor polypeptide according to claim 43 or a nucleic acid molecule expressing same, the genetically modified cell according to claim 44, or the pharmaceutical composition according to claim 45 in the preparation of a medicament for treating a tumor in an individual, wherein preferably, the tumor is a tumor expressing DLL3 (DLL3+) on the surface of tumor cells; more preferably, the tumor is a solid tumor or a hematologic tumor associated with high DLL3 expression; further preferably, the tumor is lung cancer; more preferably, the tumor is small cell lung cancer.
